(19) Europäisches Patentamt
European Patent Office
Office européen des brevets

(11) EP 4 763 844 A1

(12) EUROPEAN PATENT APPLICATION
published in accordance with Art. 153(4) EPC

(43) Date of publication:
24.06.2026 Bulletin 2026/26

(21) Application number: 24853848.0

(22) Date of filing: 15.08.2024

(51) International Patent Classification (IPC):
*C07D 471/04* *(2006.01)* *C07D 401/04* *(2006.01)*
*C07D 403/04* *(2006.01)* *A61K 31/495* *(2006.01)*
*A61P 35/00* *(2006.01)*

(52) Cooperative Patent Classification (CPC):
**A61K 31/495; A61P 35/00; C07D 401/04; C07D 403/04; C07D 471/04**

(86) International application number:
**PCT/CN2024/112283**

(87) International publication number:
**WO 2025/036442 (20.02.2025 Gazette 2025/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.08.2023 CN 202311028557**

(71) Applicant: **Genfleet Therapeutics (Shanghai) Inc.**
**Shanghai 201203 (CN)**

(72) Inventors:
- **ZHAO, Jinzhu**
  **Shanghai 201203 (CN)**
- **CAO, Yudong**
  **Shanghai 201203 (CN)**
- **XIONG, Lun**
  **Shanghai 201203 (CN)**
- **WEI, Xiong**
  **Shanghai 201203 (CN)**
- **LIU, Xiangchao**
  **Shanghai 201203 (CN)**
- **MENG, Mengting**
  **Shanghai 201203 (CN)**
- **ZHOU, Fusheng**
  **Shanghai 201203 (CN)**
- **LI, Jingrong**
  **Shanghai 201203 (CN)**
- **LAN, Jiong**
  **Shanghai 201203 (CN)**
- **LU, Qiang**
  **Shanghai 201203 (CN)**

(74) Representative: **Elzaburu S.L.P.**
**Edificio Torre de Cristal**
**Paseo de la Castellana 259 C, planta 28**
**28046 Madrid (ES)**

# (54) METHOD FOR PREPARING PYRAZINONAPHTHYRIDINE DIONE COMPOUND, AND INTERMEDIATE THEREOF

(57) The present invention belongs to the technical field of medicine. In particular, provided in the present invention are a method for preparing a pyrazinonaphthyridine dione compound, and an intermediate thereof. The method uses simple, cheap and readily available starting raw materials, has a low reaction cost and simple and convenient operations, and is suitable for industrial application.

EP 4 763 844 A1

## Description

The present application claims priority to a prior application with the patent application No. 2023110285571, entitled "METHOD FOR PREPARING PYRAZINONAPHTHYRIDINE DIONE COMPOUND AND INTERMEDIATE THEREOF" and filed with China National Intellectual Property Administration on August 15, 2023, which is incorporated herein by reference in its entirety.

## TECHNICAL FIELD

The present disclosure belongs to the technical field of pharmaceuticals, and particularly relates to a method for preparing a pyrazinonaphthyridine dione compound and an intermediate thereof.

## BACKGROUND

Lung cancer is the cancer with the highest incidence rate in the world, with nearly 80% of cases being non-small cell lung cancer (NSCLC). RAS gene mutations are identified in about 32% of lung cancers, and the mutation in any one of the three major subtypes of the RAS gene (HRAS, NRAS, or KRAS) may lead to the development of tumors in humans. It has been reported that the highest mutation frequency among RAS genes was observed in the KRAS gene, and KRAS mutation was detected in 25-30% of tumors. The most common KRAS mutations are found at residues G12 and G13 in the P-loop and residue Q61. The G12C mutation is a frequent mutation in the KRAS gene (a glycine-12 mutation to cysteine). This mutation has been found in about 13% of cancers, about 43% of lung cancers, and almost 100% of MYH-related polyposis (familial colon cancer syndrome). In order to improve the inhibitory activity against KRAS mutations and reduce the inhibitory activity against wild-type KRAS, the development of novel selective inhibitors against KRAS mutants with higher activity, better selectivity, and lower toxicity has great significance.

PCT patent application WO2021083167A1 reports a series of inhibitors for selectively inhibiting KRAS mutations, and reports a representative compound: (4aR,8R)-3-acryloyl-11-chloro-10-(2-fluoro-6-hydroxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (compound of formula I) and a preparation method therefor, bringing a breakthrough progress to the research of KRAS inhibitors. Therefore, there is a need in the art to optimize the synthetic route for the compound of formula I.

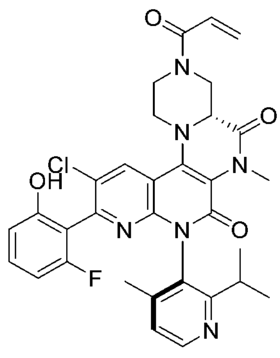

I

## SUMMARY

In a first aspect of the present disclosure, provided is a method for preparing a compound D9, which comprises the following steps:

step D: subjecting a compound D6 to a ring-closing reaction in the presence of a base and then to a resolution reaction with a chiral resolving reagent to give a compound D8;

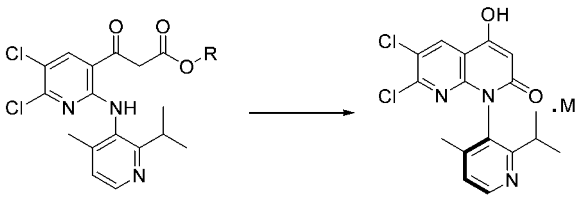

D6 D8 ;

and

step E: removing the chiral resolving reagent from the compound D8 to give the compound D9;

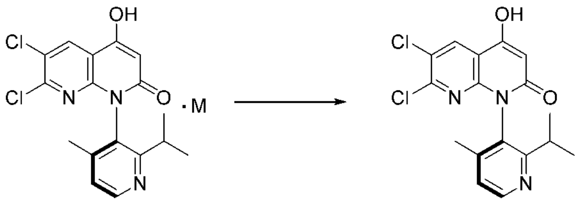

D8 D9 ;

wherein M is the chiral resolving reagent;

R is a chiral auxiliary group.

The reaction conditions in step D and step E are compatible with the preparation method described in the content of the first aspect below.

In the present disclosure, the selectivity of the target configuration is improved by introducing a chiral auxiliary group, and the compound D9 with a single R configuration is obtained by resolution.

In some embodiments, R is selected from

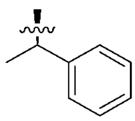 , 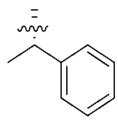 , 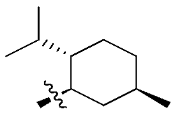 , 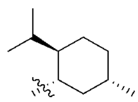 ,

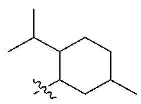 , 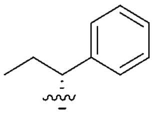 , 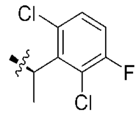 , 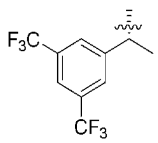 , 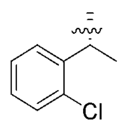 , 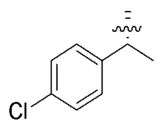 , 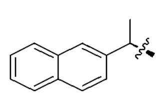 ,

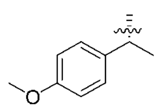 , and 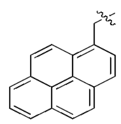 .

In some embodiments, the chiral resolving reagent M is selected from one or more of D-(+)-di-p-toluoyl tartaric acid, N-acetyl-L-phenylalanine, L-(-)-dibenzoyl tartaric acid, riboflavin, L-(-)-camphorsulfonic acid, glutamic acid, L-tartaric acid, D-(+)-di-p-anisoyl tartaric acid, D-glycine, D-mandelic acid, R-methoxy-trifluoromethylphenylacetic acid, L-aspartic acid, D-(+)-dibenzoyl tartaric acid, L-pyroglutamic acid, and (R)-(-)-1,1'-binaphthyl-2,2'-diyl hydrogenphosphate;

preferably, the chiral resolving reagent M is selected from one or more of D-(+)-di-p-toluoyl tartaric acid, L-(-)-camphorsulfonic acid, L-tartaric acid, (R)-(-)-1,1'-binaphthyl-2,2'-diyl hydrogenphosphate, and D-(+)-dibenzoyl tartaric acid;

more preferably, the chiral resolving reagent M is (R)-(-)-1,1'-binaphthyl-2,2'-diyl hydrogenphosphate.

In some embodiments, the compound D9 is used for preparing a compound of formula I:

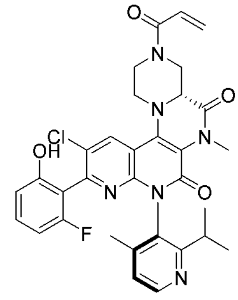

I ;

or for preparing any one or a combination of compounds selected from a compound D10, a compound D13, a compound D14, a compound D15, a compound D17, and a compound D18;

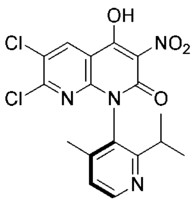

D10 ,

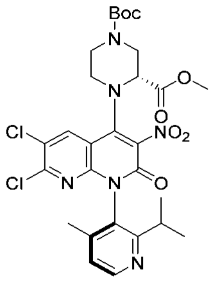

D13 ,

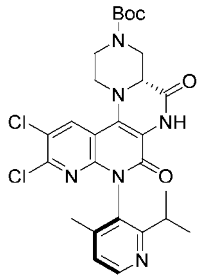

D14 ,

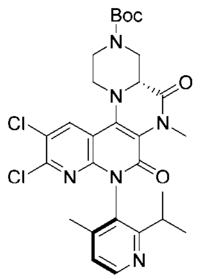

D15 ,

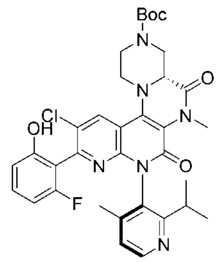

D17 ,

and

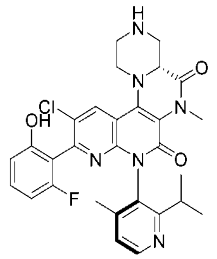

D18 .

The compound of formula I with R configuration is synthesized by taking the compound D9 as a starting material, which can effectively reduce the reaction cost and reduce the three wastes generated in the reaction process, compared with the compound with R configuration that is obtained by resolution after the racemate of the compound of formula I with R configuration and S configuration is prepared.

Preferably, the compound D10, the compound D13, the compound D14, the compound D15, the compound D17, and the compound D18, alone or in any combination thereof, are used for preparing the compound of formula I. In some embodiments, in the preparation method described above, a method for preparing the compound D10 comprises the following step:

step F: subjecting the compound D9 to a nitration reaction to give the compound D 10;

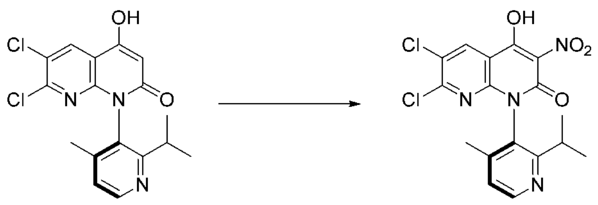

D9 D10 .

The reaction conditions in step F are compatible with the preparation method described in an eighth aspect below. In some embodiments, in the preparation method described above, a method for preparing the compound D13 comprises the following step:
step G: substituting the hydroxy group in the compound D10 to give the compound D13;

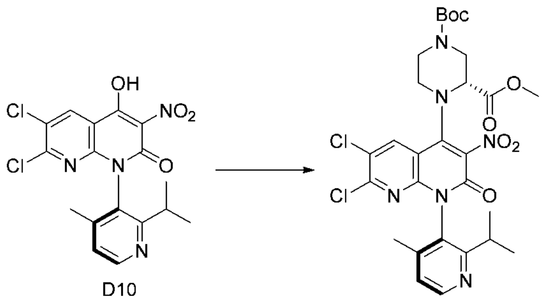
D10
D13
.

wherein preferably, a method for substituting the hydroxy group in the compound D10 comprises: subjecting the compound D10 to an esterification reaction with sulfonic anhydride or sulfonyl halide, and then subjecting to a substitution reaction with a compound D12 to give the compound D13;

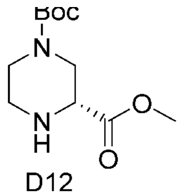
D12
.

The reaction conditions in step G are compatible with the preparation method described in a seventh aspect below.
In some embodiments, in the preparation method described above, a method for preparing the compound D14 comprises the following step:
step H: subjecting the compound D13 to a reaction in the presence of a reductant to give the compound D14;

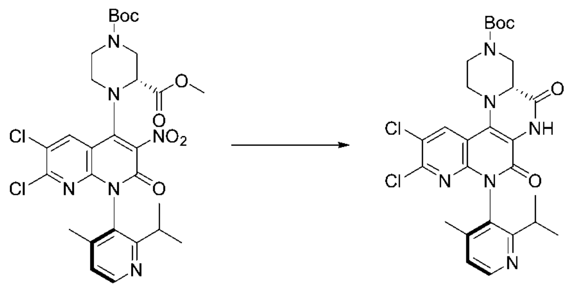
D13
D14
.

Preferably, the reductant is sodium dithionite.
Preferably, in the presence of the reductant, the compound D13 is subjected to reduction and cyclization reactions to give the compound D14.
The reaction conditions in step H are compatible with the preparation method described in a sixth aspect below. In some embodiments, in the preparation method described above, a method for preparing the compound D15 comprises the following step:
step I: subjecting the compound D14 to a reaction with a methylating reagent to give the compound D15;

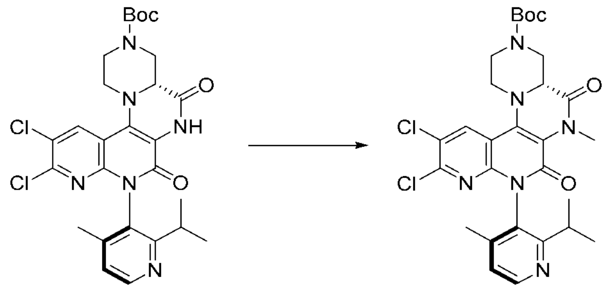
D14 D15

.

The reaction conditions in step I are compatible with the preparation method described in a fifth aspect below. In some embodiments, in the preparation method described above, a method for preparing the compound D17 comprises the following step:

step J: subjecting the compound D15 to a coupling reaction with a compound D16 to give the compound D17;

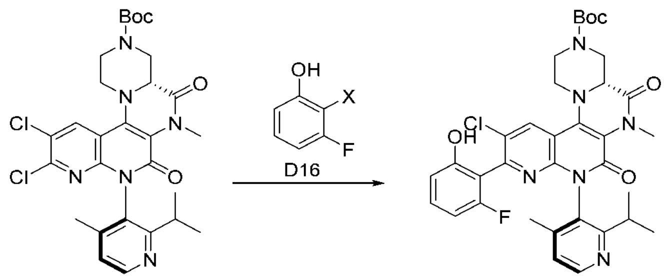
D15 D16 D17

;

wherein X is a group capable of being subjected to a coupling reaction with a chlorine substituent at an ortho position to the N atom on the naphthyridine ring.

The reaction conditions in step J are compatible with the preparation method described in a fourth aspect below. In some embodiments, in the preparation method described above, a method for preparing the compound D18 comprises the following step:

step K: removing the Boc group from the compound D17 to give the compound D18;

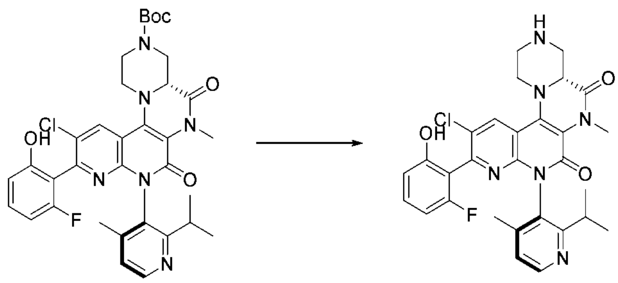
D17 D18

.

The reaction conditions in step K are compatible with the preparation method described in a third aspect below. In some embodiments, in the preparation method described above, a method for preparing the compound of formula I comprises the following step:

step L: subjecting the compound D18 to a reaction with an acylating agent to give the compound of formula I;

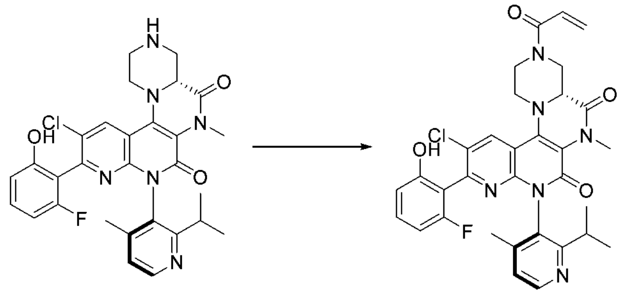

D18 I.

The reaction conditions in step L are compatible with the preparation method described in a second aspect below. In a second aspect, the present disclosure provides a method for preparing the compound of formula I, which comprises the following step:
step L: subjecting the compound D18 to a reaction with an acylating agent to give the compound of formula I;

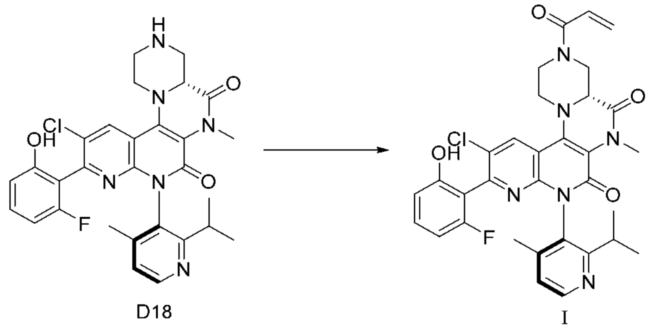

D18 I.

In one embodiment, the acylating agent is selected from acrylic anhydride and acryloyl chloride.

Preferably, the compound D18 is subjected to an acylation reaction with the acylating agent to give the compound of formula I.

In one embodiment, a feeding mass ratio of the acylating agent to the compound D18 is (0.1-1):1, e.g., (0.1-0.5):1 or 0.2:1.

In one embodiment, the acylating agent is added dropwise to the reaction system at a temperature of -5 °C to 15 °C.

In one embodiment, a solvent for the acylation reaction is one of dichloromethane, tetrahydrofuran, 2-methyltetrahydrofuran, and water, or a mixture thereof; preferably, the solvent is tetrahydrofuran or a mixed solvent of 2-methyltetrahydrofuran and water; the solvent is used in an amount of 3-10 times by weight relative to the weight of the compound D18.

In one embodiment, the acylation reaction is performed at a temperature of -5 °C to 30 °C, e.g., 0 °C to 10 °C; the acylation reaction is performed for a period of 0.5 hours to 10 hours.

In one embodiment, the preparation method may further comprise one or more of the following post-treatment steps: after the reaction is completed, adding water and dichloromethane for extraction, concentrating, adding butanone for concentration, adding methyl tert-butyl ether for crystallization and purification, and/or adding dichloromethane for slurrying, then filtering, and drying.

In a third aspect, the present disclosure provides a method for preparing the compound D18, which comprises:
step K: removing the Boc group from the compound D17 to give the compound D18;

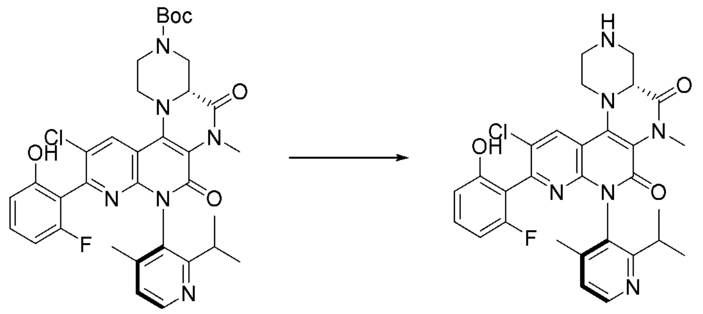

D17 D18

Preferably, the Boc group of the compound D17 is removed under an acidic condition to give the compound D18.

In one embodiment, the acidic condition is an organic solution of an acid or an aqueous solution of an acid; preferably, the acidic condition is any one of a hydrogen chloride-methanol solution, a hydrogen chloride-ethanol solution, a hydrogen chloride-isopropanol solution, a hydrogen chloride-ethyl acetate solution, hydrogen chloride-dioxane, a hydrogen chloride-water solution, or concentrated hydrochloric acid; more preferably, the acidic condition is a hydrogen chloride-dioxane solution or a hydrogen chloride-water solution.

In one embodiment, the organic solution of the acid or the aqueous solution of the acid may have a concentration of 1-10 mol/L, preferably a concentration of 4 mol/L.

In one embodiment, a feeding equivalent ratio (molar ratio) of the organic solution of the acid or the aqueous solution of the acid to the compound D17 is (5-10):1.

In one embodiment, the solvent for the reaction of removing the Boc group is one or more of an alcohol solvent, an ester solvent, an ether solvent, and water;

preferably, the solvent is one or more of methanol, ethanol, isopropanol, and water;
preferably, the solvent is used in an amount of 5-10 times by weight relative to the weight of the compound D17. In one embodiment, the reaction for removing the Boc group is performed at a temperature of -5 °C to 60 °C, e.g., -5 °C to 30 °C, room temperature, or 20 °C to 30 °C; the reaction for removing the Boc group is performed for a period of 0.5 hours to 20 hours or 0.5 hours to 10 hours, e.g., 0.5 hours to 1.5 hours or 1 hour.

In one embodiment, the preparation method may further comprise one or more of the following post-treatment steps: after the reaction is completed, concentrating the reaction solution, adding water and dichloromethane for extraction, performing liquid separation, adding N,N-dimethylformamide and methyl tert-butyl ether to the aqueous phase, adjusting the pH of the reaction solution with an aqueous sodium carbonate solution, and/or adding n-heptane and stirring, then filtering, and drying.

In a fourth aspect, the present disclosure provides a method for preparing the compound D17, which comprises: step J: subjecting the compound D15 to a coupling reaction with a compound D16 to give the compound D17;

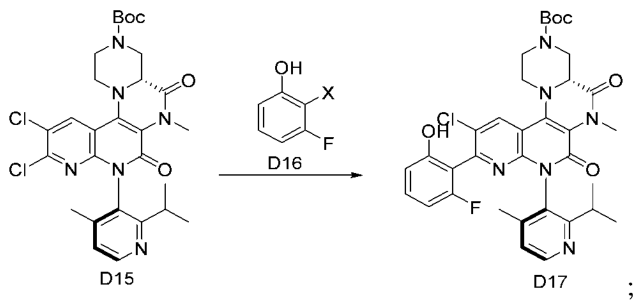


D15 D17 ;

wherein X is a group capable of being subjected to a coupling reaction with a chlorine substituent at an ortho position to the N atom on the naphthyridine ring, and is preferably a boronic acid group, a boronic acid ester group, or a potassium fluoroborate.

Preferably, the compound D15 and the compound D16 are subjected to a coupling reaction in the presence of a catalyst and a base to give the compound D17.

In one embodiment, a feeding equivalent ratio (molar ratio) of the compound D16 to the compound D15 is (1-3):1, e.g., 1.5:1 or 1.4:1.

In one embodiment, the base in the coupling reaction may be an organic base or an inorganic base, wherein the

organic base is one or more of triethylamine, N,N-diisopropylethylamine, n-butyllithium, lithium diisopropylamide, lithium bis(trimethylsilyl)amide, potassium acetate, sodium tert-butoxide, potassium tert-butoxide, and sodium n-butoxide, and the inorganic base is one or more of sodium hydride, potassium phosphate, dipotassium phosphate, sodium carbonate, potassium carbonate, cesium carbonate, sodium bicarbonate, potassium bicarbonate, sodium hydroxide, and lithium hydroxide.

In one embodiment, the base is dipotassium phosphate, which is beneficial for avoiding the racemization of the product.

In one embodiment, a feeding equivalent ratio (molar ratio) of the base to the compound D15 in the coupling reaction is (1-3):1, e.g., 1.2:1, 2.4:1, or 3.1:1.

In one embodiment, the catalyst in the coupling reaction is a palladium catalyst; preferably, the catalyst is one or more of tetrakis(triphenylphosphine)palladium, palladium dichloride, palladium acetate, bis(dibenzylideneacetone)palladium, chloro(2-dicyclohexylphosphino-2',4',6'-triisopropyl-1,1'-biphenyl)[2-(2'-amino-1,1'-biphenyl)]palladium (II), [1,1'-bis(diphenylphosphino)ferrocene]dichloropalladium(II), and tris(dibenzylideneacetone)dipalladium.

In one embodiment, a feeding equivalent ratio (molar ratio) of the catalyst to the compound D15 is (0.005-0.05):1, e.g., 0.025:1, 0.02:1 or 0.015:1.

In one embodiment, the solvent for the coupling reaction is one of tetrahydrofuran, dimethyltetrahydrofuran, 1,4-dioxane, N,N-dimethylformamide, N-methylpyrrolidone, dimethyl sulfoxide, and water, or a mixture thereof; preferably, the reaction solvent is a mixed solvent of 1,4-dioxane and water.

In one embodiment, the reaction solvent is used in an amount of 10-30 times by volume, or 20-30 times by weight or 25-30 times by weight relative to the weight of the compound D15, for example, 20 times by volume or 21 times by weight.

In one embodiment, the coupling reaction is performed at a temperature of 50 °C to 100 °C, e.g., 75 °C to 85 °C; the coupling reaction is performed for a period of 1 hour to 20 hours or 1 hour to 10 hours, e.g., 2 hours to 4 hours.

In one embodiment, the coupling reaction is performed under an inert gas or nitrogen atmosphere; the inert gas is selected from any one of helium and argon.

In one embodiment, the preparation method may further comprise one or more of the following post-treatment steps: after the reaction is completed, concentrating the reaction solution, adding 2-methyltetrahydrofuran and water for extraction, then concentrating, and/or adding ethanol and water and stirring, then filtering, and drying. In a fifth aspect, the present disclosure provides a method for preparing the compound D15, which comprises:

step I: subjecting the compound D14 to a reaction with a methylating reagent to give the compound D15;

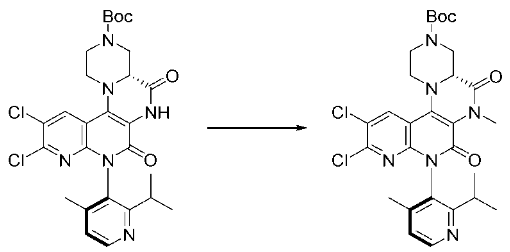

D14 D15 .

Preferably, the compound D14 is reacted with a methylating reagent in the presence of a base to give the compound D15.

In one embodiment, the methylating reagent is one or more of iodomethane, dimethyl sulfate, and dimethyl carbonate; a feeding equivalent ratio (molar ratio) of the methylating reagent to the compound D14 is (1-5):1; e.g., (1-2):1 or 1.5:1.

In one embodiment, the methylating reagent is a combination of dimethyl sulfate and dimethyl carbonate.

In one embodiment, the base is one or more of lithium bis(trimethylsilyl)amide, sodium bis(trimethylsilyl)amide, lithium diisopropylamide, n-butyllithium, sodium hydroxide, potassium hydroxide, lithium hydroxide, sodium carbonate, potassium carbonate, and cesium carbonate.

In one embodiment, a feeding equivalent ratio (molar ratio) of the base to the compound D14 is (1-5):1; e.g., (1-2):1.

In one embodiment, the solvent for the reaction is one or more of an ether solvent, a halogenated hydrocarbon solvent, an ester solvent, a nitrile solvent, an amide solvent, an alcohol solvent, and water; preferably, the solvent for the reaction is one or more of tetrahydrofuran, 2-methyltetrahydrofuran, dioxane, ethylene glycol dimethyl ether, methyl tert-butyl ether, dichloromethane, dichloroethane, methyl formate, ethyl formate, ethyl acetate, isopropyl acetate, acetonitrile, N,N-dimethylformamide, N,N-dimethylacetamide, N-methylpyrrolidone (NMP), dimethyl sulfoxide, methanol, ethanol, isopropanol, and water; more preferably, the solvent is one or more of N,N-dimethylformamide, N,N-dimethylacetamide,

dimethyl sulfoxide, and N-methylpyrrolidone.

In one embodiment, the reaction solvent is used in an amount of 5-15 times by volume or 5-15 times by weight relative to the weight of the compound D14, for example, 10 times by volume.

In one embodiment, the reaction is performed at a temperature of 0 °C to 40 °C; preferably, the reaction is performed at room temperature or 20 °C to 30 °C; the reaction is performed for a period of 8 hours to 30 hours or 16 hours to 20 hours.

In one embodiment, the temperature of the reaction is lower than 0 °C, e.g., -10 °C to 0 °C, to help improve the stability of the reaction.

In one embodiment, the preparation method may further comprise one or more of the following post-treatment steps: after the reaction is completed, adding water and stirring, filtering, and/or adding water and dichloromethane for extraction, and concentrating.

In one embodiment, the post-treatment steps further comprise crystallizing the separated compound D15, preferably by adding a crystallization solvent and then cooling for crystallization.

In one embodiment, the crystallization solvent is one or more of n-heptane, dichloromethane, and methyl tert-butyl ether.

In one embodiment, the cooling crystallization is performed at 20±5 °C.

In a sixth aspect, the present disclosure provides a method for preparing the compound D14, which comprises: step H: subjecting the compound D13 to a reaction in the presence of a reductant to give the compound D14;

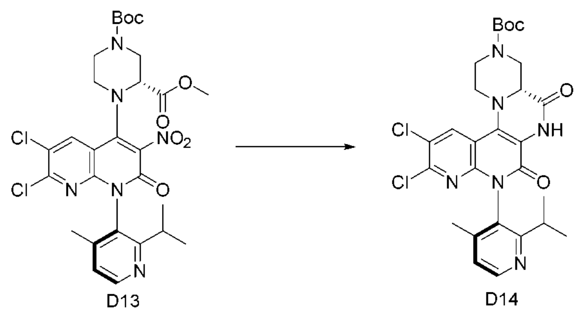

Preferably, the reductant is sodium dithionite.

Preferably, in the presence of the reductant, the compound D13 is subjected to reduction and cyclization reactions to give the compound D14.

In one embodiment, the method for preparing the compound D14 comprises subjecting the compound D13 to a reduction reaction in the presence of a reductant, followed by a cyclization reaction to give the compound D14. In one embodiment, the reductant is one or more of hydrogen, palladium on carbon, platinum on carbon, Raney nickel, zinc powder, iron powder, sodium hydrosulfite (sodium dithionite), sodium sulfide, and hydrazine hydrate; preferably, the reductant is sodium hydrosulfite, palladium on carbon, or platinum on carbon.

In one embodiment, a feeding mass ratio of the reductant to the compound D13 is (0.1-4):1, e.g., 3.4:1.

In one embodiment, the reductant is an aqueous sodium hydrosulfite solution, and the aqueous solution is added dropwise to the reaction system.

Optionally, a product of the reduction reaction may be directly subjected to a cyclization reaction without post-treatment.

In one embodiment, the reductant is hydrogen, and the reduction reaction is performed in the presence of a catalyst; the catalyst is preferably a noble metal catalyst, further preferably a platinum catalyst, and more preferably a platinum-on-carbon catalyst.

In one embodiment, after the metal catalyst is removed in the reduction reaction, the cyclization reaction is performed in the presence of an acid catalyst; the acid catalyst is preferably acetic acid.

In one embodiment, the reduction and cyclization reactions are performed under nitrogen or inert gas atmosphere; the inert gas is one of argon and helium.

In one embodiment, the solvent for the reduction and cyclization reactions is one or more of an alcohol solvent, an ester solvent, and an ether solvent; preferably, the solvent for the reduction and cyclization reactions is one or more of methanol, ethanol, isopropanol, methyl formate, ethyl formate, ethyl acetate, isopropyl acetate, tetrahydrofuran, and 1,4-dioxane.

In one embodiment, the solvent is used in an amount of 5-10 times by volume relative to the weight of the compound D13.

In one embodiment, the reduction reaction is performed at a temperature of 0 °C to 30 °C, e.g., 20 °C to 30 °C; the cyclization reaction is performed at a temperature of 20 °C to 100 °C or 50 °C to 100 °C, e.g., 75 °C to 85 °C; the reaction

progress may be detected by a conventional monitoring method in the art (e.g., TLC, HPLC, or NMR), and the end point of the reaction is generally considered the point at which the compound disappears or no longer reacts.

In one embodiment, the preparation method may further comprise one or more of the following post-treatment steps: after the cyclization reaction is completed, cooling, adding water and ethyl acetate for extraction, concentrating, and/or adding isopropyl acetate and stirring, then filtering, and drying.

In one embodiment, the cooling crystallization is performed at 0 °C to 10 °C.

In a seventh aspect, the present disclosure provides a method for preparing the compound D13, which comprises: step G: substituting the hydroxy group in the compound D10 to give the compound D13;

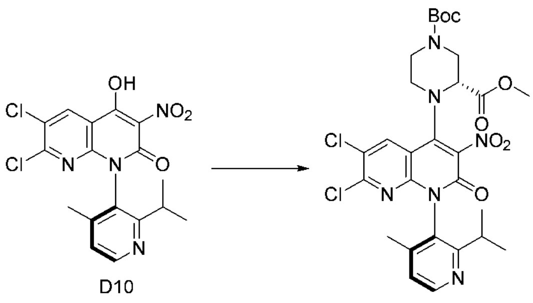

D10 D13 ;

wherein preferably, a method for substituting the hydroxy group in the compound D10 comprises: subjecting the compound D10 to an esterification reaction with sulfonic anhydride or sulfonyl halide, and then subjecting to a substitution reaction with a compound D12 to give the compound D13.

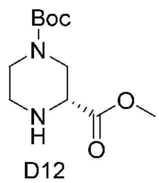

D12

Preferably, the compound D10 is subjected to an esterification reaction with sulfonic anhydride or sulfonyl halide in the presence of a base to give an intermediate compound D11, and then the intermediate compound D11 is subjected to a substitution reaction with the compound D12 to give the compound D13;

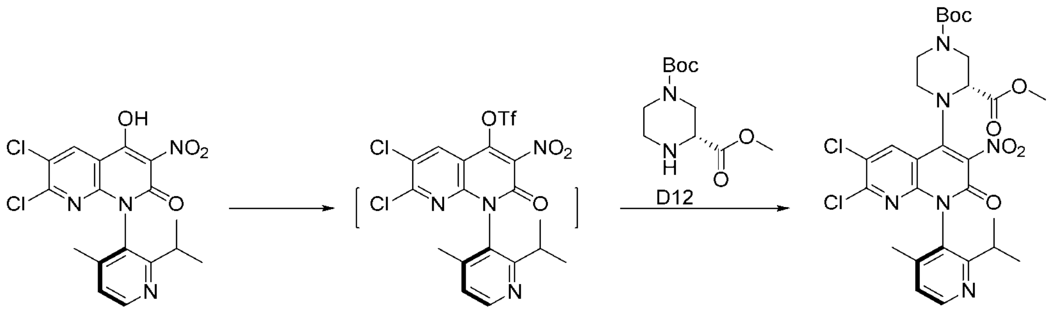

D10 D11 D13 .

The hydroxy group is substituted to prepare an activated sulfonate compound, and the sulfonate is used as a leaving group, such that the reaction is easier to perform, the side reactions are fewer, and the purity of the product is high.

In one embodiment, the sulfonic anhydride or sulfonyl halide in the esterification reaction is one or more of trifluoromethanesulfonic anhydride, trifluoroacetic anhydride, methanesulfonic anhydride, ethanesulfonic anhydride, benzenesulfonyl anhydride, p-toluenesulfonic anhydride, trifluoroacetyltrifluoromethanesulfonic anhydride, p-methylbenzenesulfonyl chloride, methanesulfonyl chloride, ethylsulfonyl chloride, benzenesulfonyl chloride, 4-methylbenzenesulfonyl chloride, and 4-nitrobenzenesulfonyl chloride.

In one embodiment, a feeding equivalent ratio (molar ratio) of the sulfonic anhydride or sulfonyl halide to the compound D10 is (1-3):1, e.g., 1.65:1, 1.5:1, or 1.4:1.

In one embodiment, the sulfonic anhydride or sulfonyl halide is added dropwise to the reaction system.

In one embodiment, the base in the esterification reaction is one or more of triethylamine, diisopropylamine, diisopropylethylamine, pyridine, N-methylmorpholine, sodium bicarbonate, sodium carbonate, potassium carbonate, sodium hydroxide, and potassium hydroxide.

In one embodiment, a feeding equivalent ratio (molar ratio) of the base to the compound D10 is (1-5):1, e.g., 3:1 or 4:1.

In one embodiment, the solvent for the esterification reaction is one or more of ethyl acetate, isopropyl acetate, dichloromethane, dichloroethane, tetrahydrofuran, dimethyltetrahydrofuran, acetonitrile, and N,N-dimethylformamide.

In one embodiment, the solvent is used in an amount of 5-15 times by volume or 5-15 times by weight relative to the weight of the compound D10, for example, 10 times by volume.

In one embodiment, the esterification reaction is performed at a temperature of -60 °C to 0 °C, e.g., -55 °C to - 45 °C, -30 °C to 0 °C, or -20 °C to -30 °C; the esterification reaction is preferably performed for a period of 10 minutes to 120 minutes, e.g., 20 minutes.

In one embodiment, the esterification reaction is performed at a temperature of below -30 °C, further below - 35 °C, -40 °C, or -45 °C, e.g., -55 °C to -45 °C or -45 °C to -35 °C, which is beneficial for controlling the generation of impurities or the content of impurities in the reaction.

In one embodiment, the reaction solution of the esterification reaction may be directly subjected to a substitution reaction without post-treatment.

In one embodiment, in the substitution reaction, a feeding equivalent ratio (molar ratio) of the compound D12 to the compound D10 is (1-2):1, e.g., 1.3:1.

In one embodiment, the compound D12 is added dropwise to the reaction system.

In one embodiment, the substitution reaction is performed at a temperature of -30 °C to 0 °C, e.g., -20 °C to 30 °C; the substitution reaction is preferably performed for a period of 10 minutes to 120 minutes, e.g., 30 minutes.

In one embodiment, the preparation method may further comprise one or more of the following post-treatment steps: after the substitution reaction is completed, adding water and stirring, performing layer separation and liquid separation, concentrating the organic phase, and/or adding ethyl acetate and n-heptane and stirring, then filtering, and drying.

In some embodiments, a solution of D13 in an organic solvent may also be prepared and directly used in the next step, wherein the organic solvent is preferably a solvent for the next step.

In an eighth aspect, the present disclosure provides a method for preparing the compound D10, which comprises: step F: subjecting the compound D9 to a nitration reaction to give the compound D10;

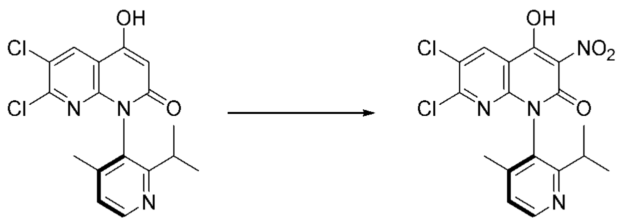

D9 D10

In one embodiment, a nitration reagent in the nitration reaction may be nitric acid, nitrate, a mixture of nitric acid and other acids or anhydrides, or a mixture of nitrate and other acids or anhydrides; the other acids or anhydrides are preferably selected from one or more of sulfuric acid, concentrated sulfuric acid, glacial acetic acid, and acetic anhydride. The nitric acid is selected from dilute nitric acid, concentrated nitric acid, and fuming nitric acid.

Preferably, the nitration reagent is one of a mixed system of concentrated nitric acid-concentrated sulfuric acid, concentrated nitric acid-glacial acetic acid, and concentrated nitric acid-acetic anhydride.

In one embodiment, the nitrate is selected from one or more of potassium nitrate, sodium nitrate, and sodium nitrite.

In one embodiment, a feeding equivalent ratio (molar ratio) of the nitration reagent to the compound D9 is (1-2):1, e.g., 1:1 or 2:1; the other acid or anhydride is used in an amount of 1-5 times by volume or 1-6 times by weight relative to the weight of the compound D9.

In one embodiment, the nitration reaction is performed at a temperature of 0 °C to 30 °C; preferably, the reaction is performed at room temperature, e.g., 20 °C to 30 °C.

In one embodiment, the nitration reaction is performed for a period of 0.5 hours to 2 hours or 0.5 hours to 1 hour. In one embodiment, the preparation method may further comprise a post-treatment, wherein the post-treatment may be one or more of the following steps: after the reaction is completed, adding water and stirring, then filtering, and drying, and/or adding methanol and stirring, then filtering, and drying.

In one embodiment, the preparation method may further comprise a post-treatment, wherein the post-treatment

may be one or more of the following steps: after the reaction is completed, adding water and stirring (and/or adding water for washing), cooling to 10-20 °C, and/or stirring and filtering, then washing a filter cake with water and/or an acetonitrile solution, and drying.

In the first aspect of the present disclosure, further provided is a method for preparing the compound D9, which comprises:

step D: subjecting a compound D6 to a ring-closing reaction in the presence of a base and then to a resolution reaction with a chiral resolving reagent M to give a compound D8;

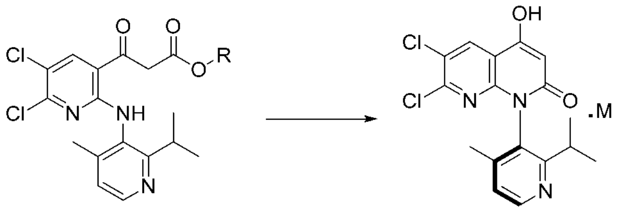

D6 D8

;

and

a step of removing the chiral resolving reagent M from the compound D8 to give the compound D9;

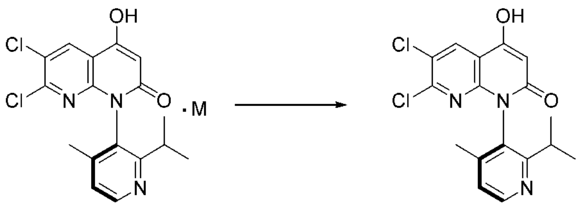

D8 D9

.

Preferably, the compound D6 is subjected to a ring-closing reaction in the presence of a base to give an intermediate compound D7; and the intermediate compound D7 is subjected to a resolution reaction with the chiral resolving reagent M to give the compound D8.

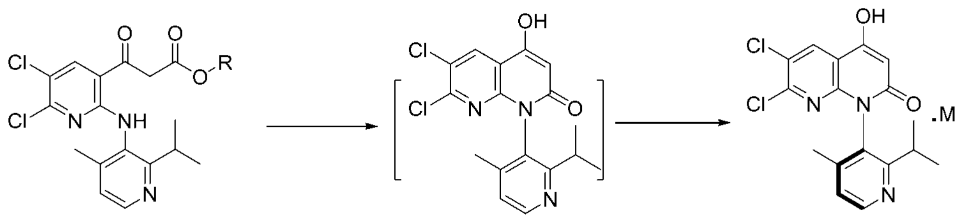

D6 D7 D8

.

In one embodiment, the base in the ring-closing reaction is one or more of potassium carbonate, sodium carbonate, cesium carbonate, sodium methoxide, sodium ethoxide, potassium tert-butoxide, triethylamine, N,N-diisopropylethylamine, and DBU (1,8-diazabicyclo[5.4.0]undec-7-ene); a feeding equivalent ratio (molar ratio) of the base to the compound D6 is (1-10):1, preferably (1-5):1, (1-3):1, or (1-2):1.

In one embodiment, the solvent for the ring-closing reaction is one or more of acetone, methanol, ethanol, isopropanol, tert-butanol, dichloromethane, 1,2-dichloroethane, tetrahydrofuran, and toluene, or optionally further contains water.

In one embodiment, the solvent is used in an amount of 5-10 times by volume relative to the weight of the compound D6.

In one embodiment, the ring-closing reaction is performed at a temperature of below 40 °C, further, 0 °C to 40 °C or 0 °C to 30 °C, e.g., 25 °C to 30 °C, 20 °C to 30 °C, or 30 °C to 40 °C.

In one embodiment, the reaction temperature is 10 °C to 50 °C, preferably room temperature.

In one embodiment, the ring-closing reaction is preferably performed for a period of 30 hours to 72 hours, e.g., 48 hours to 72 hours.

In one embodiment, the chiral resolving reagent M is selected from one or more of D-(+)-di-p-toluoyl tartaric acid, N-acetyl-L-phenylalanine, L-(-)-dibenzoyl tartaric acid, riboflavin, L-(-)-camphorsulfonic acid, glutamic acid, L-tartaric acid, D-(+)-di-p-anisoyl tartaric acid, D-glycine, D-mandelic acid, R-methoxy-trifluoromethylphenylacetic acid, L-aspartic acid, D-(+)-dibenzoyl tartaric acid, L-pyroglutamic acid, and (R)-(-)-1,1'-binaphthyl-2,2'-diyl hydrogenphosphate; preferably, the chiral resolving reagent M is selected from one or more of D-(+)-di-p-toluoyl tartaric acid, L-(-)-camphorsulfonic acid, L-tartaric acid, (R)-(-)-1,1'-binaphthyl-2,2'-diyl hydrogenphosphate, and D-(+)-dibenzoyl tartaric acid; more preferably, the chiral resolving reagent M is (R)-(-)-1,1'-binaphthyl-2,2'-diyl hydrogenphosphate.

In one embodiment, a feeding equivalent ratio (molar ratio) of the resolving reagent M to the compound D6 is (0.25-5):1 (0.25-2):1, e.g., 0.25:1, 0.5:1, 1:1, 1.5:1, or 2:1; preferably, the feeding equivalent ratio (molar ratio) is 1:1.

In one embodiment, a feeding mass ratio of the resolving reagent M to the compound D6 is (0.5-1):1, e.g., 0.67:1. In the present disclosure, the compound D8 may be present together with the resolving reagent in the form of a cocrystal, a salt, a corresponding solvate thereof, or the like.

In one embodiment, the solvent for the resolution reaction is one or more of an ether solvent, an alcohol solvent, a ketone solvent, a sulfoxide solvent, an ester solvent, and an amide solvent, or optionally further contains water; preferably, the solvent is selected from one or more of 1,4-dioxane, methyltetrahydrofuran, methanol, ethanol, isopropanol, acetone, methanone, ethanone, butanone, methyl isobutyl ketone, cyclohexanone, ethyl acetate, isopropyl acetate, and N,N-dimethylformamide, or optionally further contains water.

In one embodiment, the solvent for the resolution reaction is used in an amount of 5-20 times by volume relative to the weight of the compound D7.

In one embodiment, the temperature of the resolution reaction is -30 °C to 50 °C, preferably -25 °C to -15 °C or 0 °C to 5 °C or 0 °C to 50 °C; the reaction progress may be monitored by HPLC, with the complete reaction generally being the end point.

In one embodiment, the preparation method may further comprise the following post-treatment steps: after the resolution reaction is completed, filtering and drying.

In one embodiment, the compound D8 is subjected to an acid neutralization reaction to remove the chiral resolving reagent M to give the compound D9.

In one embodiment, the acid used in the acid neutralization reaction is one or more of p-toluenesulfonic acid, p-nitrobenzoic acid, hydrochloric acid, phosphoric acid, sulfuric acid, trifluoroacetic acid, and methanesulfonic acid.

In one embodiment, a feeding equivalent ratio (molar ratio) of the acid to the compound D8 is (1-5): 1.

In one embodiment, a feeding mass ratio of the acid to the compound D8 is (1-5): 1.

In one embodiment, the solvent for the acid neutralization reaction is one of an ether solvent and a nitrile solvent; preferably, the solvent is acetonitrile.

In one embodiment, the solvent for the acid neutralization reaction is used in an amount of 1-10 times by volume or 1-10 times by weight relative to the weight of the compound D8.

In one embodiment, the acid neutralization reaction is performed at a temperature of 0 °C to 30 °C, e.g., 20 °C to 25 °C; preferably, the reaction temperature is room temperature; the reaction progress may be detected by a conventional monitoring method in the art (e.g., TLC, HPLC, or NMR), and the end point of the reaction is generally considered the point at which the compound disappears or no longer reacts.

In one embodiment, after the reaction is completed, the method may further comprise one or more of the following post-treatment steps: after the reaction is completed, filtering, adding water and concentrating, adjusting pH (e.g., 4.5-5) with a sodium hydroxide solution, then filtering, and drying.

In one embodiment, the preparation method may further comprise the following post-treatment steps: after the resolution reaction is completed, crystallizing the separated compound D9 to give a product with good properties and easy drying. This reduces the problems of too much water or high viscosity in the product obtained from the crystallization system, resulting in difficulties in transfer or drying.

In one preferred embodiment, the crystallization is cooling crystallization by adding a crystallization solvent. In one embodiment, the crystallization solvent is n-heptane.

In one embodiment, the cooling crystallization is performed at 10 °C to 20 °C.

In a ninth aspect, the present disclosure provides a method for preparing the compound D6, which comprises: step C: subjecting a compound D4 to a condensation reaction with a compound D5 to give the compound D6;

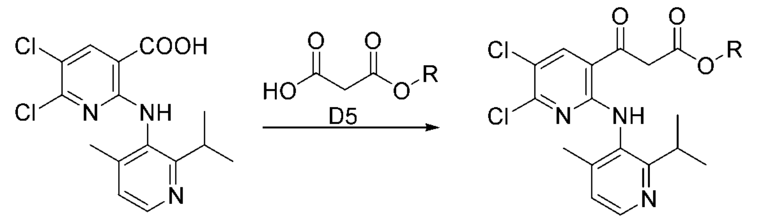


D4 D6

;

wherein R is selected from

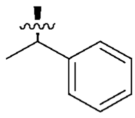, 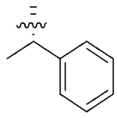, 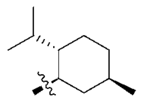, 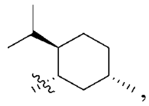, 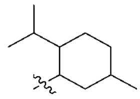, 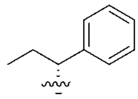,

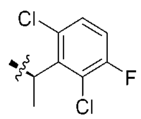, 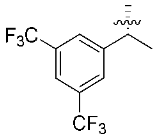, 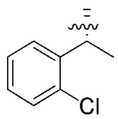, 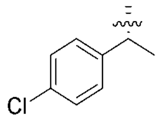, 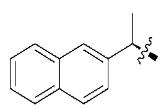, 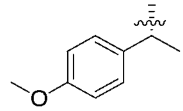, and 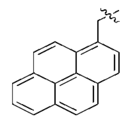.

In one embodiment, the method for preparing the compound D6 comprises subjecting the compound D4 to an acylation reaction with an acylating reagent, and then to a condensation reaction with the compound D5 in the presence of a base and a catalyst to give the compound D6.

In one embodiment, the acylating reagent in the acylation reaction is one or more of phosphorus oxychloride, thionyl chloride, oxalyl chloride, acetyl chloride, thionyl chloride, and pivaloyl chloride.

In one embodiment, a feeding equivalent ratio (molar ratio) of the acylating reagent to the compound D4 is (1-5):1.

In one embodiment, the acylation reaction is performed in the presence of a catalyst, which is a catalyst commonly used in the art, e.g., optionally N,N-dimethylformamide or thionyl chloride.

In one embodiment, the solvent for the acylation reaction is one or more of dichloromethane, dichloroethane, acetonitrile, tetrahydrofuran, 2-methyltetrahydrofuran, and toluene.

In one embodiment, the solvent is used in an amount of 5-10 times by volume or 5-10 times by weight relative to the weight of the compound D4.

In one embodiment, the acylation reaction is performed at a temperature of 40 °C to 100 °C, preferably 45 °C to 55 °C or 50 °C to 100 °C; the reaction progress may be monitored by HPLC, with the complete reaction generally being the end point.

In one embodiment, a product of the acylation reaction is added to a solvent, and the next step is performed directly; the solvent is one or more of tetrahydrofuran and 2-methyltetrahydrofuran.

In one embodiment, a feeding equivalent ratio (molar ratio) of the compound D5 to the compound D4 in the condensation reaction is (1-3): 1, e.g., 1:1, 1.5:1, 2:1, 2.5:1, or 3:1; preferably, the feeding equivalent ratio (molar ratio) is (1.5-2):1.

In one embodiment, the base in the condensation reaction is one or more of triethylamine, N,N-diisopropylethylamine, and DBU; the feeding equivalent ratio (molar ratio) of the base to the compound D4 is (1-10):1, e.g., 1:1, 2:1, 3:1, 4:1, or 5:1.

In one embodiment, the catalyst in the condensation reaction is one or more of magnesium chloride, magnesium bromide, and magnesium iodide.

In one embodiment, a feeding equivalent ratio (molar ratio) of the catalyst to the compound D4 is (1-3):1.

In one embodiment, the solvent for the condensation reaction is one or more of dichloromethane, dichloroethane, acetonitrile, tetrahydrofuran, 2-methyltetrahydrofuran, and toluene.

In one embodiment, the condensation reaction is performed at a temperature of 0 °C to 30 °C or 0 °C to 10 °C; the reaction progress may be monitored by HPLC, with the complete reaction generally being the end point.

In one embodiment, provided is a method for preparing the compound D5, which comprises the following step: subjecting a compound SM1 to a transesterification reaction with a chiral alcohol to give the compound D5;

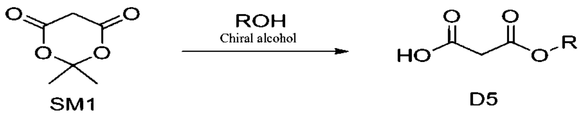


SM1 D5

.

In one embodiment, in the transesterification reaction, a feeding equivalent ratio (molar ratio) of the chiral alcohol to the compound SM1 is (0.9-3):1 or (1-3):1.

In one embodiment, the solvent for the transesterification reaction is one or more of an ether solvent, an aromatic hydrocarbon solvent, and an amide solvent; preferably, the solvent is one or more of ethylene glycol monomethyl ether, ethylene glycol dimethyl ether, methyl tert-butyl ether, benzene, toluene, xylene, and N,N-dimethylformamide.

In one embodiment, the transesterification reaction is performed at a temperature of 50 °C to 110 °C or 50 °C to 100 °C; the reaction progress may be monitored by HPLC, with the complete reaction generally being the end point.

In one embodiment, after the reaction is completed, the method may further comprise a post-treatment, wherein the post-treatment may be one or more of the following steps: after the reaction is completed, adding water, methyl tert-butyl ether, and potassium carbonate, and performing liquid separation, and/or adding methyl tert-butyl ether to the aqueous phase, adjusting the pH value of the reaction solution with hydrochloric acid, stirring, performing liquid separation, and concentrating the organic phase.

In some embodiments, the post-treatment further comprises crystallizing the separated compound D5; the solvent for the crystallization is preferably n-heptane, and the crystallization is preferably performed at a temperature of -15 °C to 0 °C.

In one embodiment, the compound D5 is selected from the following group:

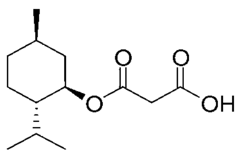
D5-1 ,
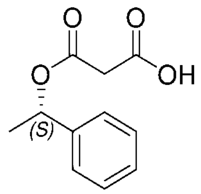
D5-2 , and
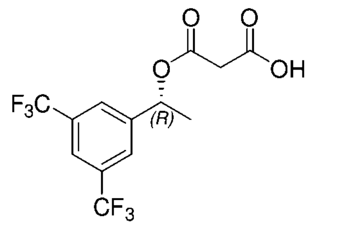
D5-3 .

In one embodiment, the compound D6 is selected from the following group:

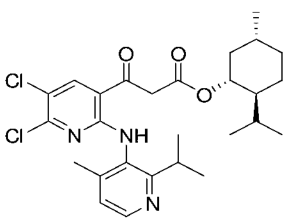
D6-1 ,
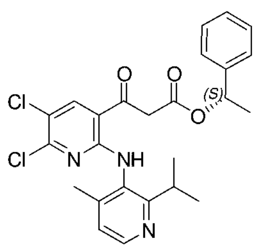
D6-2 , and
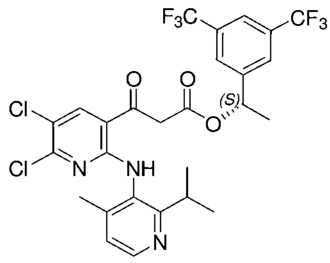
D6-3 .

In a tenth aspect, the present disclosure provides a method for preparing the compound D4, which comprises: step B: subjecting a compound D3 to a reaction with a chlorinating reagent to give the compound D4;

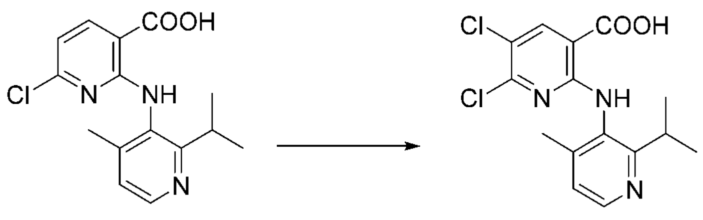

D3 D4 .

In one embodiment, the chlorinating reagent is selected from one or more of N-chlorosuccinimide, 1,3-dichloro-5,5-dimethylhydantoin, and chlorine gas.

In one embodiment, a feeding molar ratio of the chlorinating reagent to the compound D3 is (0.5-2): 1, e.g., 0.5:1, 1:1, 1.5:1, or 2:1.

Preferably, the feeding molar ratio of the chlorinating reagent to the compound D3 is (0.5-1):1.

In one embodiment, the solvent for the chlorination reaction is selected from one or more of a nitrile solvent, an amide solvent, and an ether solvent;
preferably, the solvent for the chlorination reaction is selected from one or more of acetonitrile, N,N-dimethylformamide, N,N-dimethylacetamide, tetrahydrofuran, 2-methyltetrahydrofuran, and tetrahydropyran, or optionally further contains water.

In one embodiment, the solvent is used in an amount of 5-15 times by volume relative to the weight of the compound D3.

In one embodiment, the chlorination reaction is performed at a temperature of 10 °C to 85 °C, e.g., 75 °C to 85 °C, room temperature, or -5 °C to 5 °C.

Preferably, the reaction temperature is room temperature. The reaction progress may be monitored by means of TLC, HPLC, or the like, with the complete reaction generally being the end point.

In one embodiment, the chlorination reaction may be performed under acid catalysis, preferably at room temperature, and more preferably at 20 °C to 30 °C or -5 °C to 5 °C.

In one embodiment, the acid is selected from one or more of methanesulfonic acid, hydrochloric acid (e.g., concentrated hydrochloric acid), trifluoromethanesulfonic acid, benzenesulfonic acid, p-toluenesulfonic acid, acetic acid, and trifluoroacetic acid.

Preferably, the acid is methanesulfonic acid or hydrochloric acid.

In one embodiment, in the chlorination reaction, the chlorinating reagent is 1,3-dichloro-5,5-dimethylhydantoin or N-chlorosuccinimide; a feeding molar ratio of the chlorination reagent to the compound D3 is (0.5-1): 1; the solvent for the chlorination reaction is acetonitrile or tetrahydrofuran; the reaction temperature is 10 °C to 80 °C; the reaction process may be monitored by means of TLC, HPLC, or the like, with the complete reaction generally being the end point.

In one embodiment, the reaction described above is performed under the catalysis of methanesulfonic acid or hydrochloric acid.

In some embodiments, the chlorination reaction may be performed under the catalysis of concentrated hydrochloric acid and in the presence of water.

Preferably, the hydrochloric acid is a hydrochloric acid solution with a concentration of 20%-35%. The acid catalyst can protonate 1,3-dichloro-5,5-dimethylhydantoin, enhancing its activity. It can induce and initiate the formation of a chloronium ion, facilitating the generation of a cation that attacks the aromatic ring, leading to an electrophilic chlorination reaction.

In one embodiment, the preparation method may further comprise the following post-treatment steps: after the reaction is completed, adding water and stirring, then filtering, and drying.

In an eleventh aspect, the present disclosure provides a method for preparing the compound D3, which comprises the following step:

step A: subjecting a compound D1 to a reaction with a compound D2 to give the compound D3;

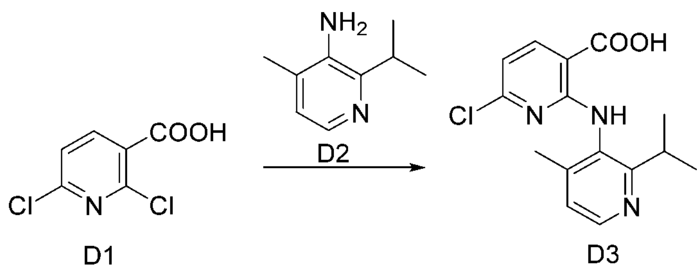

;

preferably, the compound D1 and the compound D2 are subjected to a substitution reaction to give the compound D3.

In one embodiment, the substitution reaction comprises the following steps: in a solvent, mixing the compound D1 with a base to give a stock solution 1, and mixing the compound D2 with a base to give a stock solution 2; and contacting the stock solution 1 with the stock solution 2 for a reaction to give the compound D3;
or mixing the compound D1 with a base in a solvent, and then adding the compound D2 for a reaction to give the compound D3.

In one embodiment, a feeding equivalent ratio (molar ratio) of the compound D1 to the compound D2 is (0.5-2):1,

e.g., 1:1.

In one embodiment, the base in the substitution reaction is selected from one or more of lithium bis(trimethylsilyl) amide, sodium bis(trimethylsilyl)amide, potassium bis(trimethylsilyl)amide, lithium diisopropylamide, isopropylmagnesium chloride, n-butyllithium, and tert-butyllithium; preferably, the base is lithium bis(trimethylsilyl)amide, sodium bis(trimethylsilyl)amide, or a combination thereof.

In one embodiment, a feeding equivalent ratio (molar ratio) of the base to the compound D1 in the stock solution 1 is (0.5-2):1, e.g., 0.5:1, 1:1, 1.5:1, or 2:1; preferably, a feeding equivalent ratio of the base to the compound D1 in the stock solution 1 is (1-2):1.

In one embodiment, a feeding equivalent ratio (molar ratio) of the base to the compound D2 in the stock solution 2 is (1-2):1, e.g., 1:1, 1.5:1, or 2:1; preferably, a feeding equivalent ratio (molar ratio) of the base to the compound D1 in the stock solution 2 is (1-2):1.

In one embodiment, in the method for preparing the aforementioned compound D3, a feeding equivalent ratio (molar ratio) of the base and the compound D1 and the compound D2 is (1.5-4):1:1, further (2.5-4):1:1, e.g., 3:1:1 or 3.42:1:1.

In the present disclosure, the base is added dropwise to the reaction system at a temperature of 0 °C to 30 °C, e.g., 0 °C to 5 °C or 0 °C to 20 °C.

In one embodiment, the solvent for the substitution reaction is an ether solvent; preferably, the solvent for the substitution reaction is selected from one or more of tetrahydrofuran, and 2-methyltetrahydrofuran.

In one embodiment, the solvent is used in an amount of 10-20 times by volume relative to the weight of the compound D1.

In one embodiment, the substitution reaction is performed at a temperature of 0 °C to 50 °C, e.g., 0 °C to 5 °C or 0 °C to 10 °C; the substitution reaction is performed for a period of 0.5 hours to 4 hours; the reaction progress may be monitored by HPLC, with the complete reaction generally being the end point.

In one embodiment, in the substitution reaction, the base in the stock solution 1 is lithium bis(trimethylsilyl)amide or sodium bis(trimethylsilyl)amide, and a feeding molar ratio of the base to the compound D1 is (1-2):1.

In one embodiment, in the substitution reaction, the base in the stock solution 2 is lithium bis(trimethylsilyl)amide, and a feeding molar ratio of the base to the compound D2 is (1-2):1.

In one embodiment, in the substitution reaction, the solvent for the substitution reaction is tetrahydrofuran; the reaction solvent is used in an amount of 10-15 times by volume relative to the weight of the compound D1 and the compound D2.

In one embodiment, in the substitution reaction, the reaction temperature is 0 °C to 10 °C or 10 °C to 20 °C.

In one embodiment, the substitution reaction comprises: mixing the compound D1 with a base in a solvent, and then adding the mixture for a reaction to give the compound D3, wherein the reaction temperature is 10 °C to 20 °C; the reaction is simple and convenient to operate, and energy consumption is low.

In one embodiment, the substitution reaction is performed for a period of 0.5 hours to 2 hours, and further 0.5 hours to 1 hour or 1 hour to 2 hours.

In one embodiment, the preparation method may further comprise one or more of the following post-treatment steps: after the reaction is completed, adding water to quench the reaction, concentrating, and/or adding a hydrochloric acid solution to adjust the pH (to adjust the pH to acidity, e.g., to adjust the pH to 2-4, which may further be the pH used in the corresponding steps in the examples of the present disclosure), then stirring, filtering, and drying.

In the present disclosure, on the basis of the general knowledge in the art, the aforementioned preferred conditions can be combined arbitrarily to obtain the preferred embodiments of the present disclosure.

The present disclosure provides a method for preparing the compound of formula I, which comprises the following steps:

step A: subjecting a compound D1 to a substitution reaction with a compound D2 to give a compound D3;

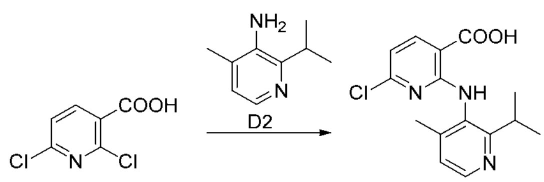


D1 D3 ;

step B: subjecting the compound D3 to a reaction with a chlorinating reagent to give a compound D4;

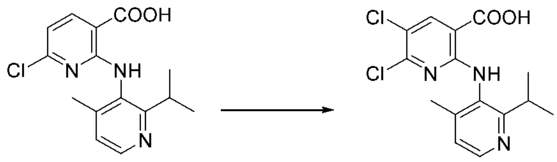

D3 D4 ;

step C: subjecting the compound D4 to a condensation reaction with a compound D5 to give the compound D6;

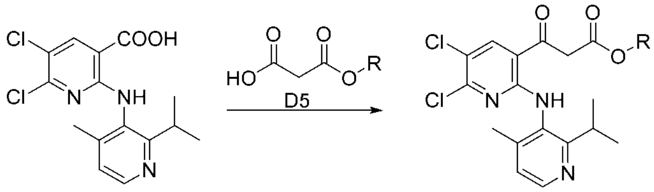


D4 D6 ;

wherein R is selected from

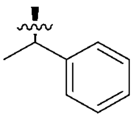 , 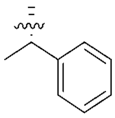 , 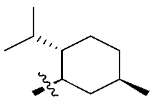 , 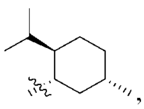 , 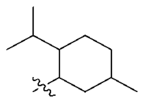 , 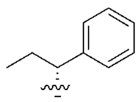 ,

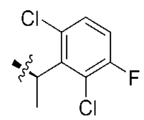 , 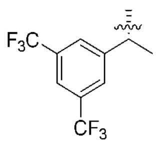 , 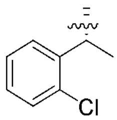 , 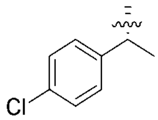 , 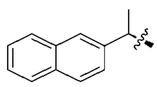 , 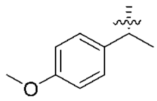 , and 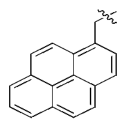 ;

step D: subjecting the compound D6 to a ring-closing reaction in the presence of a base and then to a resolution reaction with a chiral resolving reagent M to give the compound D8;

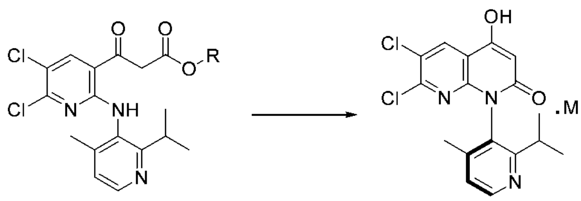

D6 D8 ;

and

step E: removing the chiral resolving reagent M from the compound D8 to give the compound D9;

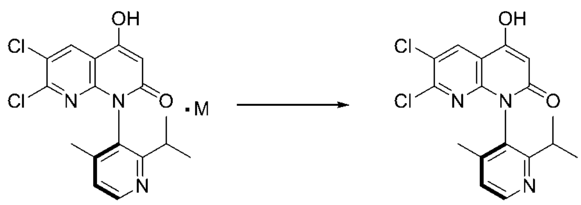

D8 D9 .

Further, the method for preparing the compound of formula I further comprises the following steps:

step F: subjecting the compound D9 to a nitration reaction to give the compound D10;

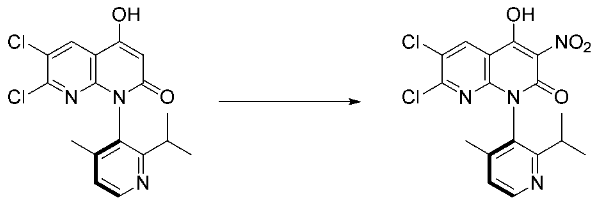
D9 D10 ;

step G: substituting the hydroxy group in the compound D10 to give the compound D13;

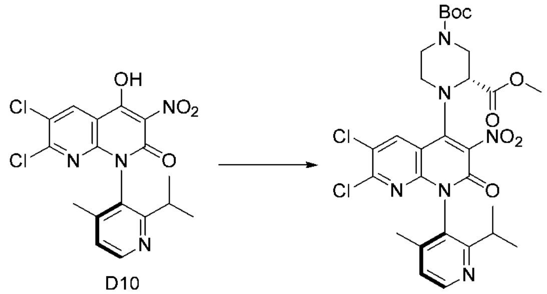
D10 D13 ;

step H: subjecting the compound D13 to a reaction in the presence of a reductant to give the compound D14;

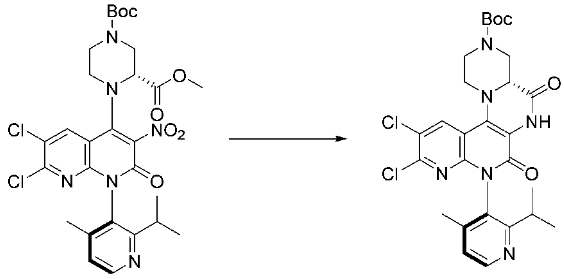
D13 D14 ;

step I: subjecting the compound D14 to a reaction with a methylating reagent to give the compound D15;

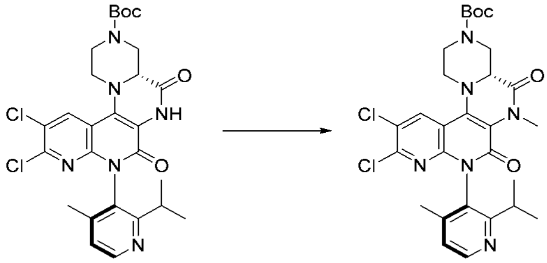
D14 D15 ;

step J: subjecting the compound D15 to a coupling reaction with a compound D16 to give the compound D17;

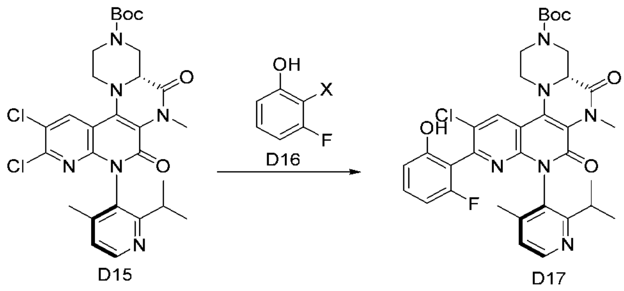

;

wherein X is a group capable of being subjected to a coupling reaction with a chlorine substituent at an ortho position to the N atom on the naphthyridine ring;

step K: removing the Boc group from the compound D17 to give the compound D18;

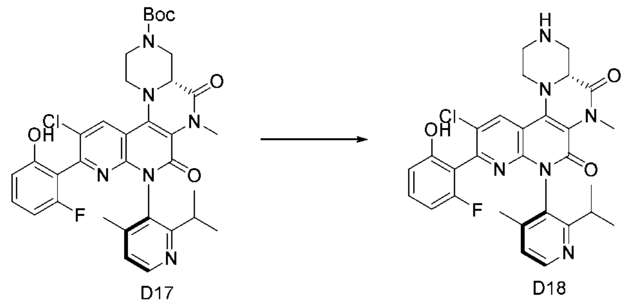

;

and

step L: subjecting the compound D18 to a reaction with an acylating agent to give the compound of formula I;

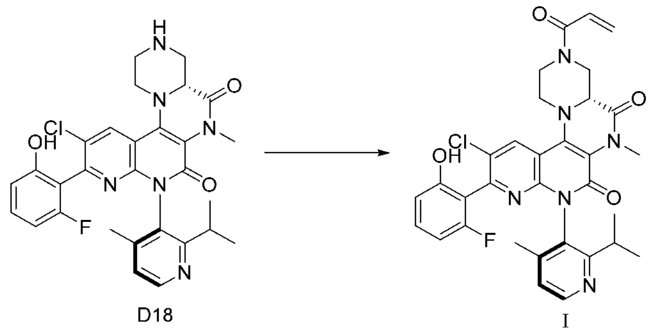

;

wherein the acylating agent is selected from acrylic anhydride and acryloyl chloride.

The reaction conditions in each reaction step are compatible with the preparation method described above. In one embodiment, the method for preparing the compound of formula I comprises the following steps:

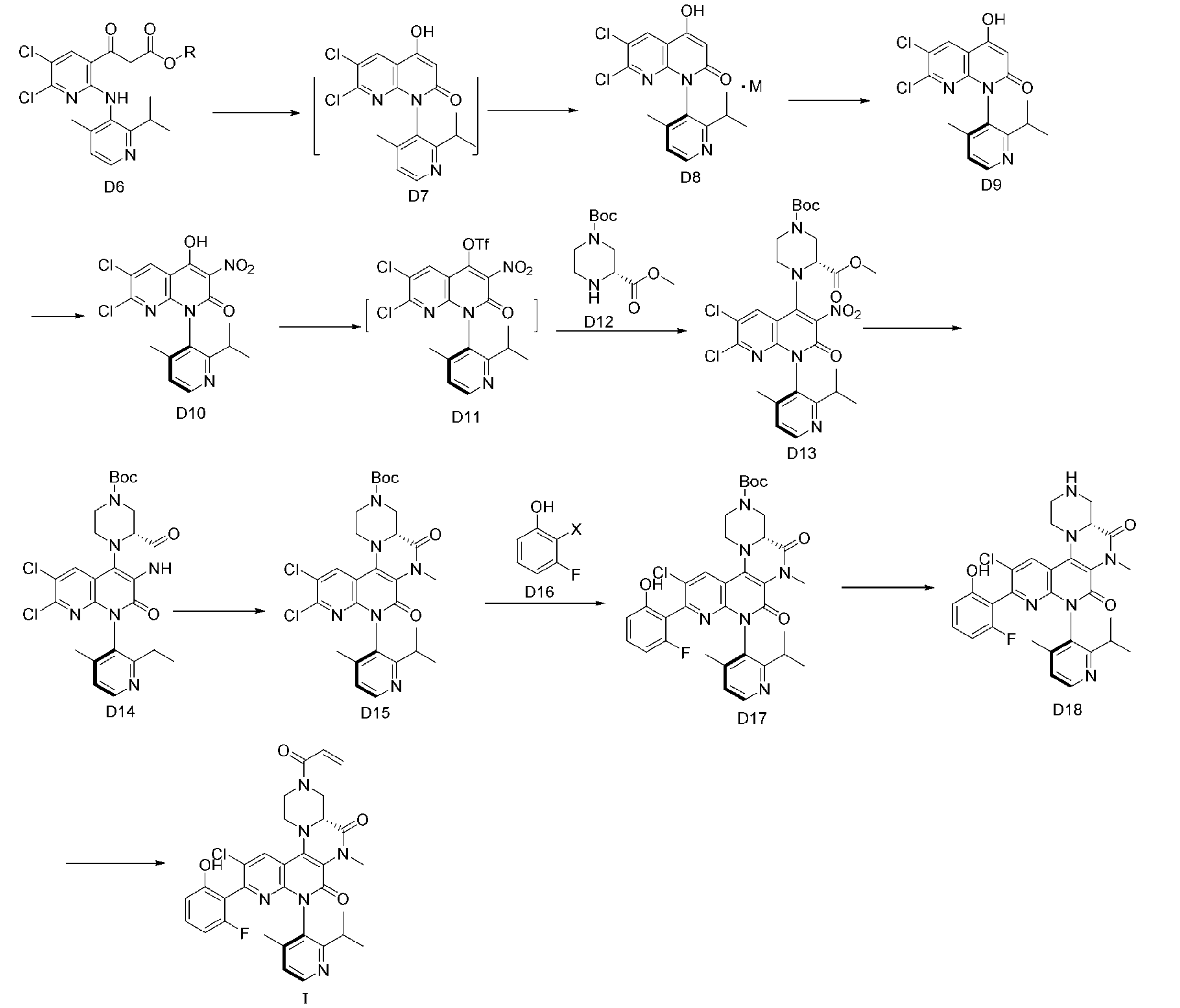

D6 D7 D8 D9

D10 D11 D12 D13

D14 D15 D16 D17 D18

I

wherein R and X are defined as above, and the reaction conditions in each reaction step are compatible with the preparation method described above.

In one embodiment, the method for preparing the compound of formula I comprises the following steps:

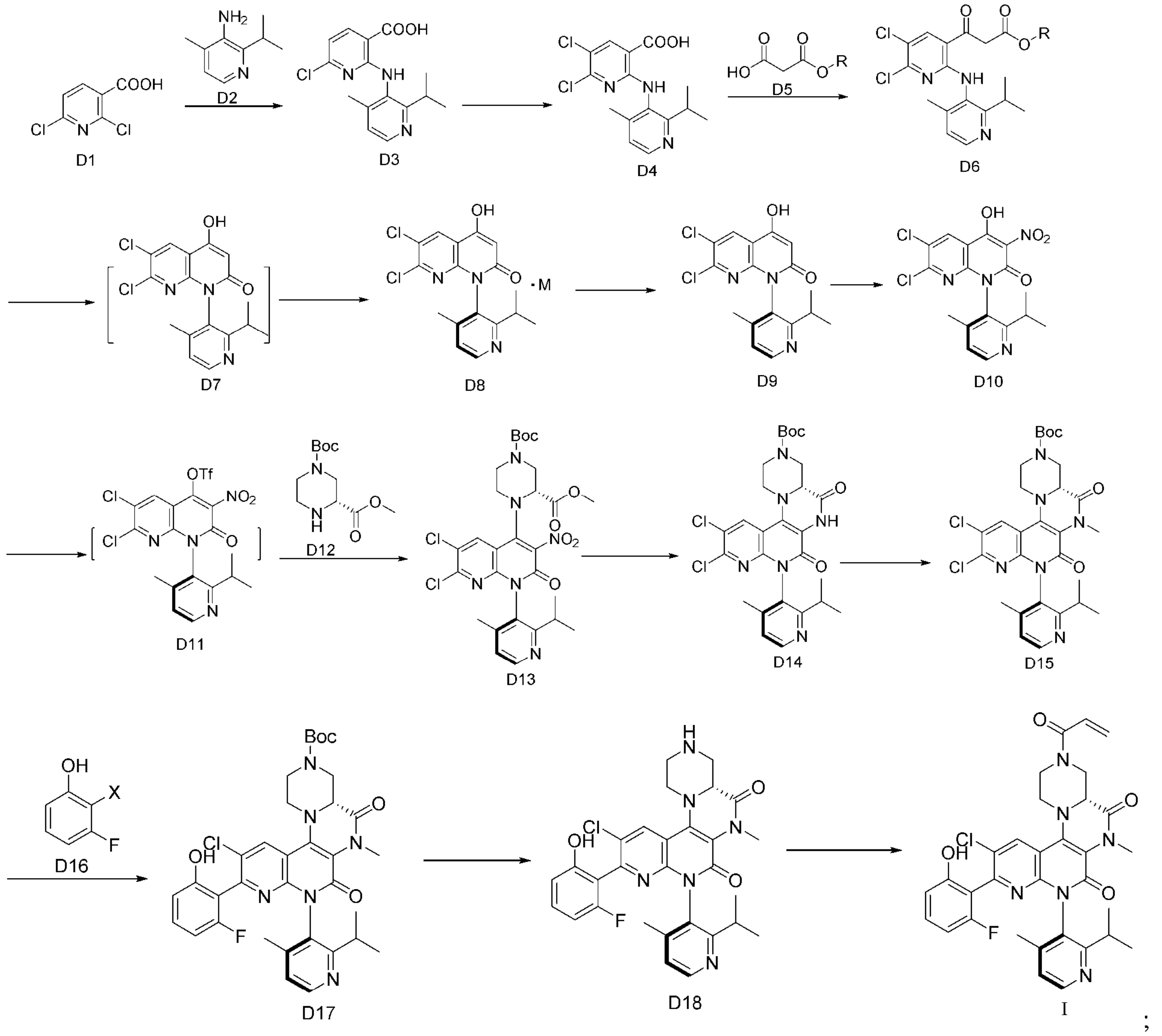


;

wherein R and X are defined as above, and the reaction conditions in each reaction step are compatible with the preparation method described above.

In a twelfth aspect of the present disclosure, provided is the compound D3, D5, D6, D8, D9, D10, D13, D14, D15, D17 or D18 or a pharmaceutically acceptable salt thereof described above:

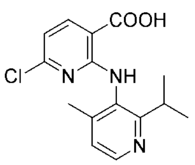
D3 ,

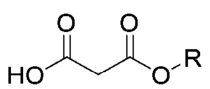
D5 ,

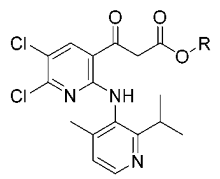
D6 ,

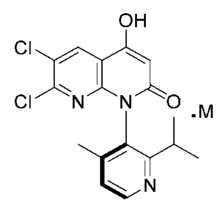
D8 ,

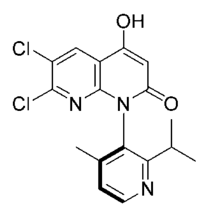
D9 ,

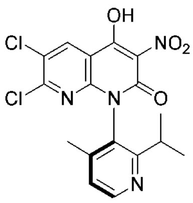
D10,
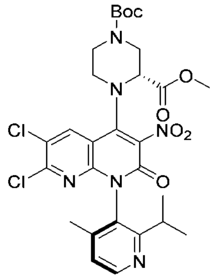
D13,
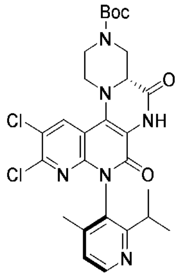
D14,
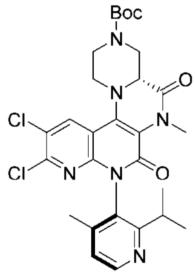
D15,
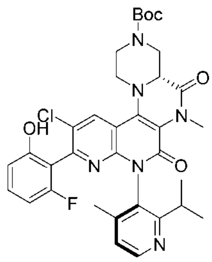
D17,

and

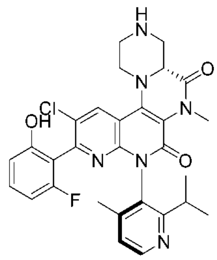
D18.

wherein R is selected from

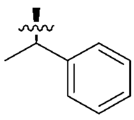, 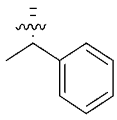, 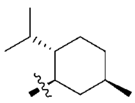, 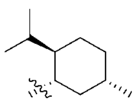, 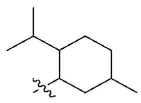, 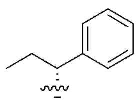,

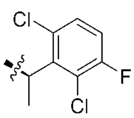, 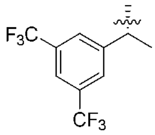, 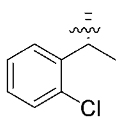, 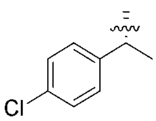, 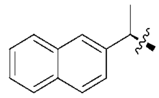, 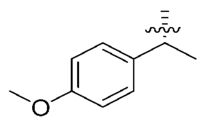, and 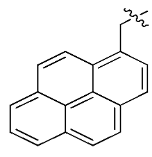;

M is selected from D-(+)-di-p-toluoyl tartaric acid, N-acetyl-L-phenylalanine, L-(-)-dibenzoyl tartaric acid, riboflavin, L-(-)-camphorsulfonic acid, glutamic acid, L-tartaric acid, D-(+)-di-p-anisoyl tartaric acid, D-glycine, D-mandelic acid, R-methoxy-trifluoromethylphenylacetic acid, L-aspartic acid, D-(+)-dibenzoyl tartaric acid, L-pyroglutamic acid, and (R)-(-)-1,1'-binaphthyl-2,2'-diyl hydrogenphosphate.

In a thirteenth aspect of the present disclosure, provided is use of the compound D3, D5, D6, D8, D9, D10, D13, D14, D15, D17 or D18 or the pharmaceutically acceptable salt thereof described above in the manufacture of a KRAS inhibitor.

In a fourteenth aspect of the present disclosure, provided is use of the compound D3, D5, D6, D8, D9, D10, D13, D14, D15, D17 or D18 or the pharmaceutically acceptable salt thereof described above in the manufacture of a medicament for treating cancer.

Preferably, the cancer is a cancer associated with a KRAS gene mutation.

In one embodiment, the cancer is pancreatic cancer, colorectal cancer, or lung cancer.

In one embodiment, the cancer is non-small cell lung cancer.

In a fifteenth aspect of the present disclosure, provided is use of the compound D3, D5, D6, D8, D9, D10, D13, D14, D15, D17, or D18 described above in the preparation of a compound of formula I.

Compared with the prior art, the present disclosure has the following advantages:

1. The column chromatography purification operation is avoided for the process product, and the post-treatment process is simple; by introducing the post-crystallization treatment operation, the purity of the product is improved, and the separation of the product is facilitated.
2. Introducing asymmetric synthesis into the process product increases the proportion of the target configuration; and

bringing the separation, purification and refining of isomers from the final drug substance step to an earlier intermediate stage can effectively reduce the process volume and the generation of three wastes.

3. The significantly reduced cost of starting materials, coupled with improved yield, has lowered the overall route cost; process optimizations have been implemented to meet the demands of industrial scale-up, for example, by utilizing the inexpensive starting material, 2,6-dichloronicotinic acid.

4. Hydroxyl is prepared into an activated sulfonate compound, which is then reacted with the piperazine compound. Compared with the original route, the reaction has high selectivity, is easier to perform, and has fewer side reactions; the problem that impurities are easily generated in the demethylation step of boron tribromide in the process of Comparative Example 1 can be effectively avoided, such that the quality of the drug substance in the production process can be better controlled.

5. The reaction is stable and the overall reaction process is controllable.

## DETAILED DESCRIPTION

The present disclosure will be further illustrated with reference to the following specific examples. It should be understood that these examples are merely intended to illustrate the present disclosure rather than limit the scope of the present disclosure. Procedures without specified conditions in the following examples are generally conducted in conventional conditions, or conditions recommended by the manufacturers.

As used herein and unless otherwise specified, the term "about" or "approximately" means within plus or minus 10% of a given value or range. Where an integer is required, the term refers to rounding up or down to the nearest integer within plus or minus 10% of a given value or range.

In the description herein, reference to "one embodiment" or "some embodiments" describes a subset of all possible embodiments, but it can be understood that "one embodiment" may be the same subset or different subsets of all possible embodiments, and can be combined with one another without conflict.

As used herein, unless otherwise stated, the terms "comprise", "include", "have", and "contain", including grammatical equivalents thereof, should generally be interpreted as open-ended and non-limiting, for example, without excluding other unrecited elements or procedures.

The "step C", "step D", "step E", etc. described herein are merely numbering effects, and are not intended to limit the strict implementation of the synthetic route according to a conventionally understood numbering order. As used herein, the term "room temperature" includes temperatures of 4 °C to 30 °C, and generally refers to 15 °C to 30 °C, preferably 20 °C to 30 °C.

As used herein, the term "solution" refers to an aqueous solution unless otherwise specified.

As used herein, the term "complete dissolution" means that a product is completely dissolved and becomes clear. As used herein, the term "condensation reaction" refers to a reaction in which two or more organic molecules interact and then bind to a macromolecule via a covalent bond, with the loss of a small molecule (e.g., water, hydrogen chloride, or alcohol,).

As used herein, the term "substitution reaction" is a "one-step or stepwise reaction in which one atom or group is substituted with another atom or group in the entirety of the molecule" as defined according to the Compendium of Chemical Terminology of the International Union of Pure and Applied Chemistry (IUPAC). Meldrum's acid: 2,2-dimethyl-1,3-dioxane-4,6-dione.

The analysis method involved in the present disclosure may adopt the following parameters:

HPLC analysis method 1: chromatographic column: Waters sunfire C18, 4.6 × 150 mm, 3.5 μm; mobile phases: A: 10 mM aqueous ammonium acetate solution; and B: acetonitrile; detection wavelength: 220 nm; flow rate: 1.0 mL/min; column temperature: 30 °C; elution gradient: 0-30 min, A% (20-95), and B% (5-80).

HPLC analysis method 2: chromatographic column: Waters sunfire C18, 4.6 × 150 mm, 3.5 μm; mobile phases: A: 0.05% trifluoroacetic acid/water; and B: 0.05% trifluoroacetic acid/acetonitrile; detection wavelength: 220 nm; flow rate: 1.0 mL/min; column temperature: 65 °C; elution gradient: 0-32 min, A% (20-90), and B% (10-80).

Chiral analysis method: chromatographic column: CHIRALPAK IB 4.6 × 250 mm, 3 μm; mobile phase: 0.01% aqueous trifluoroacetic acid solution: methanol:ethanol (3:4:3); detection wavelength: 329 nm; flow rate: 0.35 mL/min; column temperature: 25 °C; run time: 40 min.

The structures of the compounds were determined by nuclear magnetic resonance (NMR) spectroscopy or/and mass spectrometry (MS). The NMR analysis was performed using a Bruker AVANCE-400 nuclear magnetic resonance instrument, with deuterated dimethyl sulfoxide (DMSO-$d_6$) as a solvent, and tetramethylsilane (TMS) as an internal standard.

The yield is calculated according to the following formula:

$$\text{yield} = (\text{production amount of target product/theoretical production amount of target product}) \times 100\%;$$

wherein the theoretical production amount = the number of moles of the starting material administered multiplied by the relative molecular mass of the target product.

That is, the $\text{yield} = \frac{\text{Product quality} \times \text{Molecular mass of starting material}}{\text{Molecular mass of product} \times \text{Mass of starting material}}$.

As used herein, the term "complete reaction" means that the degree of consumption of the reactant is greater than about 90%, preferably greater than 95%. In one embodiment, whether the reaction is completed or not is monitored using conventional methods in the prior art, such as thin-layer chromatography (TLC), high-performance liquid chromatography (HPLC), and gas chromatography (GC).

As used herein, the term "chiral resolving reagent" refers to a compound that is used to resolve a racemate (a mixture comprising two enantiomers) into its single enantiomers. These reagents generally have chiral centers and are capable of forming diastereoisomeric mixtures with racemates. The differences in solubility and crystallization rates of these mixtures are utilized for separation by crystallization, thereby achieving chiral resolution. The chiral resolving reagent may be a chiral acid or a chiral base, and the resolution is achieved by forming a salt bond with the racemate to give a diastereomeric salt.

As used herein, the term "post-treatment" means a process of extracting and/or separating a reaction product from a mixture of reactants, products, and solvents, etc. For example, the post-treatment may be crystallization, filtration, extraction, washing, concentration, chromatographic separation, optical purity detection, neutralization, adsorbent addition, drying, or the like. By appropriate selection of the solvents and the crystallization conditions, the separation of the products can be achieved. Differences in the crystallization rates and solubilities of products of different enantiomers may be caused, thereby achieving the separation of chiral products; the product (preferably chiral product) in the aqueous phase is extracted with an organic solvent to improve the purity and yield of the product; after filtration or extraction, the product may need to be washed with water or other suitable solvents to remove the attached impurities, and the washing solvent may also be other solvents (preferably solvents used during the reaction to avoid the introduction of more impurities) that can dissolve the impurities or the product; the product is concentrated by evaporating the solvent to prepare for further purification or analysis; the optical purity of the chiral product is determined using instruments such as a nuclear magnetic resonance spectrometer and high-performance liquid chromatograph, and the product needs to be derivatized (e.g., pre-column derivatization before chromatographic detection, optionally aniline derivatization) and modified if necessary; if the product exists in the form of a salt, it may need to be neutralized with a suitable base to give the chiral product in the form of a free base; impurities, catalysts, reaction by-products, and the like are removed by adding specific adsorbents, for example, an unreacted or completely reacted palladium catalyst may be removed by modifying resins, and the by-products may be adsorbed on activated carbon; and finally, the product needs to be dried to remove the residual solvent or moisture.

It should be understood that in the reaction routes provided in the present disclosure, the intermediates can be separated by preparative chromatography and then used in the next step, or can be directly dissolved in a solvent (preferably a solvent to be used in the next step) and directly used in the next step. It should be understood that if the solvent used for the solvent product is not a good solvent for the next step, it needs to be replaced with the good solvent before the reaction.

In general, the reactants, catalysts, solvents, reagents that provide a basic or acidic environment, and the like can be added into the reaction system at one time or in portions, as long as the addition is performed before the next step of the reaction steps described herein.

The compounds described in the present disclosure may exist in the form of stereoisomers and thus encompass all possible stereoisomeric forms, including but not limited to cis-trans isomers, tautomers, enantiomers, diastereomers, atropisomers (also referred to as rotamers), and the like. The compounds described in the present disclosure may also exist in the form of any combination or any mixture of the aforementioned stereoisomers, such as equimolar mixtures of mesomers, racemates, and atropisomers. For example, a single enantiomer, a single diastereomer, or a mixture thereof, or a single atropisomer or a mixture thereof. When the compounds described in the present disclosure contain an olefinic double bond, they include cis isomers and trans isomers, as well as any combination thereof, unless otherwise specified. The atropisomers of the present disclosure are stereoisomers with axial or planar chirality that are produced on the basis of restricted rotation within the molecule. Atropisomers of the compounds of the present disclosure may be indicated in bold (e.g.,

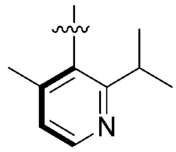
)

or labeled in other ways commonly used and well known in the art.

As used herein,

“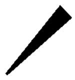” and “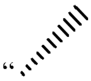”

refer to the absolute configuration of a stereogenic center. Unless otherwise specified,

“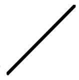”

includes the possible

“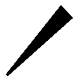” or “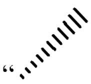”

configuration, as long as the structure allows.

Unless otherwise specified, the reagents and starting materials used in the present disclosure are commercially available.

**Example 1: Preparation of Compound D3**

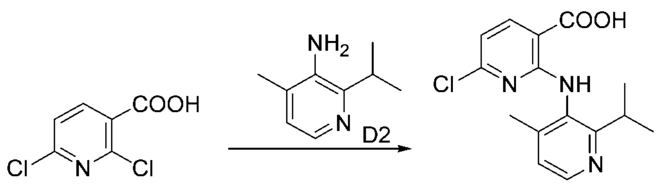


D1 D3

To a reaction flask A were added a compound D1 (20.5 g, 1.0 eq) and tetrahydrofuran (100 mL, 5 V). The mixture was cooled to 0-5 °C, and lithium bis(trimethylsilyl)amide (a 1 M solution in tetrahydrofuran, 110 mL, 1 eq) was added dropwise with the temperature controlled below 10 °C. After the dropwise addition was completed, the mixture was stirred with the temperature maintained to give a stock solution 1. To a reaction flask B were added a compound D2 (16.5 g, 1.0 eq) and tetrahydrofuran (100 mL, 5 V). The mixture was cooled to 0-5 °C, and lithium bis(trimethylsilyl)amide (a 1 M solution in tetrahydrofuran, 210 mL, 2.0 eq) was added dropwise. After the dropwise addition was completed, the mixture was stirred with the temperature maintained at 0-5 °C for 30 min to give a stock solution 2. The stock solution 2 was added dropwise to the stock solution 1. After the dropwise addition was completed, the mixture was stirred at 0-5 °C until the reaction was completed. With the temperature controlled at < 20 °C, the reaction solution was poured into 150 mL of water to quench the reaction. The solvent was removed by rotary evaporation to give a concentrated solution. The pH of the reaction solution was adjusted with a 4 N hydrochloric acid solution until a large amount of solid was precipitated. The mixture was filtered, and the filter cake was dried to give a compound D3 (28.3 g, purity: 100%, yield: 86.68%). ES-API:$[M+H]^+$ = 306.8. $^1H$ NMR (400 MHz, DMSO-$d_6$) δ 8.27 (d, *J* = 4.8 Hz, 1H), 8.08 (d, *J* = 7.7 Hz, 1H), 7.12 (d, *J* = 4.8 Hz, 1H), 6.58 (d, *J* = 7.7 Hz, 1H), 3.21 (p, *J* = 6.7 Hz, 2H), 2.12 (s, 3H), 1.12 (d, *J* = 6.7 Hz, 6H).

**Example 2: Preparation of Compound D3**

The feeding molar ratio of a compound D1 to the base in a stock solution 1 was compound D1:lithium bis(trimethylsilyl)amide = 1:2, the feeding molar ratio of a compound D2 to the base in a stock solution 2 was compound D2:lithium bis(trimethylsilyl)amide = 1:1, and the feeding molar ratio of the compound D1 to the compound D2 was compound D1:compound D2 = 1:1. The tetrahydrofuran used to dissolve the compound D1 and the compound D2 was used in an amount of 15 times by volume relative to the weight of the compound D1 and the compound D2, the reaction temperature was 0-10 °C, and the other operations were performed as those in Example 1 to give a compound D3 (yield: 86.7%).

**Example 3: Preparation of Compound D3**

The feeding molar ratio of a compound D1 to the base in a stock solution 1 was compound D1:sodium bis(trimethylsilyl)amide = 1:2, the feeding molar ratio of a compound D2 to the base in a stock solution 2 was compound D2:lithium bis(trimethylsilyl)amide = 1:1, and the feeding molar ratio of the compound D1 to the compound D2 was compound D1:compound D2 = 1:1. The tetrahydrofuran used to dissolve the compound D1 and the compound D2 was used in an amount of 15 times by volume relative to the weight of the compound D1 and the compound D2, the reaction temperature was 0-10 °C, and the other operations were performed as those in Example 1 to give a compound D3 (yield: 77.91%).

**Example 4: Preparation of Compound D3**

To a reaction flask were added a compound D1 and tetrahydrofuran. After the mixture was stirred until complete dissolution was achieved, the reaction solution was cooled to 10-20 °C, and lithium bis(trimethylsilyl)amide was then added. After the addition was completed, the solution of a compound D2 in tetrahydrofuran was added. The mixture was stirred at 10-20 °C for 1-2 h. After the reaction was completed, as detected by sampling, water was added dropwise to the reaction system to quench the reaction. Water was added, and then the reaction solution was concentrated under reduced pressure. The pH was adjusted to 2-3 with a 4 M hydrochloric acid solution, and a solid was precipitated. The solid was removed by filtration, and the filter cake was washed with water and then dried to give a compound D3 (yield: 94%) as a white solid.

The feeding molar ratio of the compound D1 to the compound D2 to lithium bis(trimethylsilyl)amide was 1:1:3.42.

**Example 5: Preparation of Compound D3**

Referring to the method in Example 1, to a reaction flask 1 were added tetrahydrofuran (4435 g) and a compound D2 (782.4 g, 5.20 mol), and the mixture was cooled to 0-5 °C. A 1 M solution of lithium bis(trimethylsilyl)amide in tetrahydrofuran (6719 g, 7.81 mol) was added dropwise to the reaction flask 1, and the reaction solution in the reaction flask 1 was stirred at 0-5 °C for 30-60 min. To a reaction flask 2 were added tetrahydrofuran (8870 g) and a compound D1 (1000 g, 5.20 mol), and the mixture was cooled to 10-20 °C. A 1 M solution of lithium bis(trimethylsilyl)amide in tetrahydrofuran (6719 g, 7.81 mol) was added dropwise to the reaction flask 2, and the reaction solution in the reaction flask 2 was stirred at 10-20 °C for 30-60 min. With the temperature controlled at 10-20 °C, the reaction solution in the reaction flask 1 was added to the reaction flask 2, and then the mixture was heated to 25-30 °C and stirred for 30-60 min. To the reaction flask 2 were added water (20000 g) and activated carbon (400 g), and the mixture was stirred for 0.5-1.5 h and then filtered. The filter cake was rinsed with water. A 4 M aqueous hydrochloric acid solution was added dropwise to the filtrate to adjust the pH to 2-3. The mixture was filtered, and the filter cake was rinsed with water and dried to give a compound D3 (140 g, yield: 88%) as a yellow solid.

**Example 6: Preparation of Compound D4**

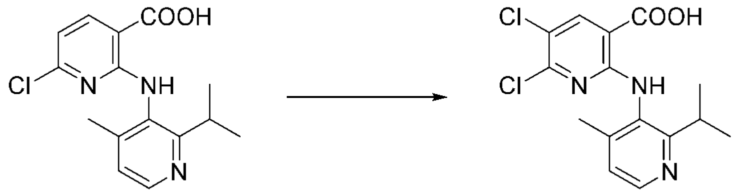

D3 D4

To a reaction flask were added a compound D3 (28.14 g, 1.0 eq), acetonitrile (200 mL, 7 V), and methanesulfonic acid (36.20 g, 4.0 eq) at room temperature. The mixture was cooled to 0-10 °C and kept warm for later use. 1,3-dichloro-5,5-dimethylhydantoin (9.07 g, 0.5 eq) was added to acetonitrile (100 mL, 3 V) and completely dissolved, and the resulting solution was added dropwise to the above reaction solution with the temperature controlled at < 25 °C during the dropwise addition. After the dropwise addition was completed, the reaction solution was transferred to the room temperature condition. After the reaction was completed, the reaction solution was concentrated to dryness, slurried with water (100 mL), and filtered. The solid was collected and dried to give a compound D4 (31.20 g, purity: 100%, yield: 77.96%). ES-API:$[M+H]^+$ = 340.2. $^1H$ NMR (500 MHz, DMSO-*d6*) δ 9.74 (s, 1H), 8.35 (d, *J* = 4.8 Hz, 1H), 8.28 (s, 1H), 7.18 (d, *J* = 4.9 Hz, 1H), 2.13 (s, 3H), 1.12 (d, *J* = 6.8 Hz, 6H).

**Example 7: Preparation of Compound D4**

Referring to the preparation method of Example 4, a compound D3 (1.0 eq) and acetonitrile (8 V) were added and stirred at 20-25 °C, and then concentrated hydrochloric acid (3.2 L, 4.0 eq) was added to the resulting solution, during which the system gradually turned clear. After the addition was completed, the system was kept warm at 20-25 °C for later use. A solution of 1,3-dichloro-5,5-dimethylhydantoin in acetonitrile was added dropwise to the reaction solution prepared above. The temperature was controlled at 20-30 °C during the dropwise addition. After the dropwise addition was completed, the mixture was sampled for detection until D3 disappeared. The pH of the resulting solution was adjusted to 2-2.5 with 2 M sodium hydroxide, and the mixture was stirred for 1 h with the temperature maintained at 20-25 °C. The mixture was filtered, and the solid was collected and dried to give a compound D4 (purity: 97.42%, yield: 91.3%).

**Example 8: Preparation of Compound D4**

To a reaction flask were added a compound D3 (9.52 g, 0.031 mol), acetonitrile (63.2 g), water (30 g), and concentrated hydrochloric acid (6.5 g). A solution of 1,3-dichloro-5,5-dimethylhydantoin (3.68 g, 0.019 mol) in acetonitrile (15.8 g) was added dropwise and slowly at 10-30 °C. After the dropwise addition was completed, the mixture was stirred at 20-30 °C for 2-6 h. After the reaction was completed as detected, the pH of the reaction solution was adjusted to 2-3 with a 2 M aqueous sodium hydroxide solution. The mixture was stirred for 2-6 h and then filtered, and the filter cake was rinsed with water (20 g). The residue was dried to give a compound D4 (10.03 g, yield: 90%) as a white solid.

**Example 9: Preparation of Compound D4**

Referring to the preparation method of Example 7, to a reaction flask were added a compound D3, acetonitrile, and concentrated hydrochloric acid. After the addition was completed, the mixture was cooled to -5 °C to 5 °C, and the solution of 1,3-dichloro-5,5-dimethylhydantoin in acetonitrile, the solution of 1,3-dichloro-5,5-dimethylhydantoin in acetonitrile and water, and the solution of 1,3-dichloro-5,5-dimethylhydantoin in acetonitrile were added to the reaction system in three steps. The feeding molar ratio of the compound D3 to 1,3-dichloro-5,5-dimethylhydantoin was 1.68:1; the molar ratio of 1,3-dichloro-5,5-dimethylhydantoin added in three steps was 1:1:1; the molar ratio of concentrated hydrochloric acid to the compound D3 was 9:1. The other procedures were referred to those in Example 7 to give a compound D4 as a yellow solid (yield: 93%).

**Example 10: Preparation of Compound D5**

(1-1) Preparation of compound D5-1

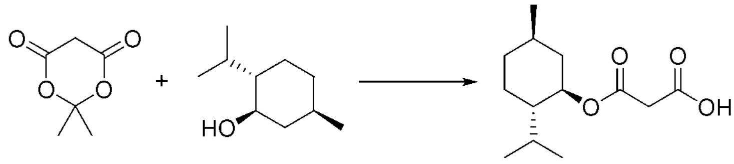

D5-1

To a reaction flask were added Meldrum's acid (50 g), L-menthol (50 g) and toluene (250 mL). The mixture was stirred at 100 °C for 1-2 h. The reaction solution was cooled to room temperature, and water (500 mL), methyl tert-butyl ether (500 mL), and potassium carbonate (66.3 g) were added to the reaction solution. The mixture was stirred for 0.5 h, followed by liquid separation. Methyl tert-butyl ether (300 mL) was added to the aqueous phase. 6 M hydrochloric acid was added dropwise and slowly to the above system to adjust the pH to 3-5. The mixture was stirred for 0.5 h and left to stand for layer separation. The organic phase was dried over anhydrous sodium sulfate and filtered, and the filtrate was concentrated to dryness to give a compound D5-1 (79 g, purity: 95.28%, yield: 97.1%) as an oily product.

(1-2) Preparation of compound D5-1

To a reaction flask were added L-menthol (10 g, 64 mmol), Meldrum's acid (10 g, 69.1 mmol), and toluene (43.6 g), and the mixture was stirred for 6-10 h with the temperature maintained at 90-100 °C. After the reaction was completed, as detected by sampling, water (100 g), potassium carbonate (11.5 g), and methyl tert-butyl ether (37 g) were added to the reaction system. The mixture was stirred for layer separation. The organic phase was discarded. The aqueous phase was washed once with methyl tert-butyl ether (37 g), and then the organic phase was discarded. Methyl tert-butyl ether (44.4 g) was added to the aqueous phase, and a 6 M aqueous hydrochloric acid solution was added dropwise and slowly with stirring to adjust the pH of the reaction solution to 3-4. The mixture was stirred for layer separation, and the organic phase was collected. Then the mixture was concentrated under reduced pressure to 2-3 V, n-heptane (35 g) was added, and the resulting mixture was concentrated under reduced pressure again to 2-3 V. To the system was added n-heptane (14 g), then a seed crystal of compound D5-1 (0.1 g) was added at 25-35 °C, and the resulting mixture was slowly cooled to -15-0 °C, and stirred for 2-6 h. The reaction solution was filtered, and the filter cake was rinsed with n-heptane (10 g) and dried to give a compound D5-1 (12.4 g, yield: 80%) as a white solid.

(2) Preparation of compound D5-2

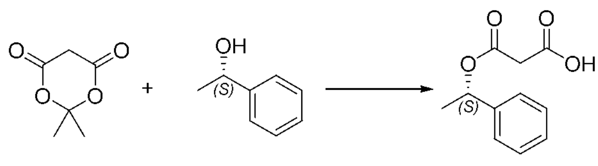

D5-2

L-menthol was replaced with S-phenylethanol, and the other operations were performed as those in Example 10 (1-1) to give a compound D5-2.

(3) Preparation of compound D5-3

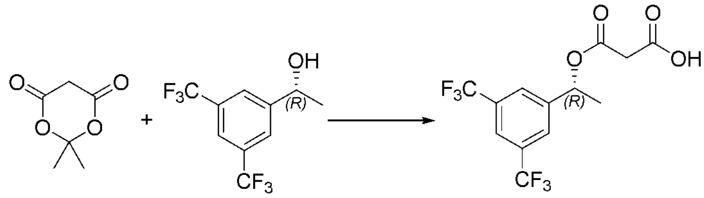

D5-3

L-menthol was replaced with (R)-1-[3,5-bis(trifluoromethyl)phenyl]ethanol, and the other operations were

performed as those in Example 10 (1-1) to give a compound D5-3.

**Example 11: Preparation of Compound D6-1**

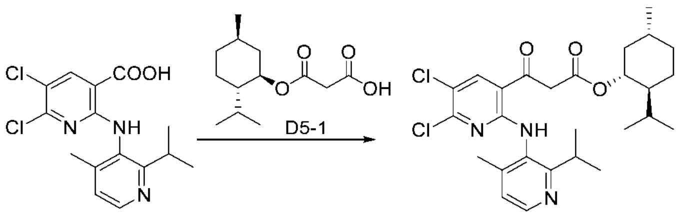


D4 D6-1

To a reaction flask A were added a compound D4 (50 g, 1.0 eq) and dichloroethane (250 mL). Thionyl chloride (50 mL, 4.7 eq) was added dropwise, and the mixture was reacted at 80 °C until the reaction was completed.

After the reaction solution was concentrated to dryness, tetrahydrofuran (500 mL, 10 V) was added, and the resulting solution was taken as a reaction solution A.

To a reaction flask B was added acetonitrile (500 mL) under an ice-water bath, then a compound D5-1 (71 g, 1.0 eq) was added, and magnesium chloride (42 g, 3.0 eq) and triethylamine (89 g, 3.0 eq) were added sequentially. After the addition was completed, the mixture was stirred at room temperature to give a reaction solution B.

The reaction solution A was added to the reaction solution B in an ice-water bath. After the reaction was completed, water (500 mL) and ethyl acetate (500 mL) were added to the reaction solution. The pH of the aqueous phase was adjusted to 5-6, followed by liquid separation. The organic phase was washed with a saturated sodium bicarbonate solution (500 mL) and then with a 10% sodium chloride solution (500 mL). The mixture was concentrated to give a crude compound D6-1 (71 g, purity: 97.07%, yield: 94.8%).

**Example 12: Preparation of Compound D6-1**

To a reaction flask were added a compound D4 (200 g, 588 mmol), toluene (1740 g), and DMF (0.2 g), and then thionyl chloride (140 g, 2.00 eq, 0.70 w/w) was added at 20-30 °C. The mixture was stirred for 2-6 h, then heated to 50-60 °C, and stirred until the reaction was completed. The reaction solution was cooled to 20-30 °C, and filtered, and the filter cake was rinsed with toluene to give an acyl chloride intermediate for later use.

To a reaction flask were sequentially added D5-1 (285 g, 2.00 eq), acetonitrile (1572 g), and dichloromethane (2253 g), and then the mixture was stirred. Magnesium chloride (168 g, 3.00 eq) was added at 20-30 °C, and triethylamine (357 g, 1.79 w/w) was then added dropwise. After the dropwise addition was completed, the mixture was stirred for 2-4 h. The acyl chloride intermediate described above was added to the system, and the mixture was reacted at 20-30 °C until the reaction was completed. The reaction mixture was concentrated under reduced pressure to 11-13 V, and water (2000 g) was added dropwise and slowly to quench the reaction system. Isopropyl acetate (1744 g) was added, and the pH of the reaction solution was adjusted to 3-4 with 4 M hydrochloric acid. The mixture was stirred for layer separation, and the organic phase was collected and sequentially washed once with water (2000 g), once with a 5% sodium bicarbonate solution (2000 g), and once with water (2000 g). The organic phase was concentrated under reduced pressure to 5-7 V, and the resulting solution of a product D6-1 in isopropyl acetate was collected and directly used in the next step.

**Example 13: Preparation of Compound D6-1**

To a reaction flask A were added dichloroethane (313 g), a compound D4 (50 g, 147 mmol), and thionyl chloride (52.5 g, 441 mmol). The mixture was stirred for 2-3 h with the temperature maintained at 45-55 °C and then concentrated under reduced pressure to 2-3 V. Dichloroethane (500 g) was added, and the mixture was concentrated under reduced pressure again to 2-3 V. Tetrahydrofuran (444 g) was added to the reaction solution for later use.

To a reaction flask B were added a solution of a compound D5-1 (53.4 g, 220 mmol) in acetonitrile (150 g), and acetonitrile (275 g), and the mixture was cooled to 0-10 °C. Magnesium chloride (42.0 g, 441 mmol) and triethylamine (89.3 g, 882 mmol) were added in portions, and the mixture was stirred at 0-10 °C for 30-60 min. Then, with the temperature controlled at 0-10 °C, the reaction solution in the reaction flask A was added dropwise to the reaction flask B. The mixture was heated to 30-40 °C and stirred for 0.5-2 h with the temperature maintained. The reaction solution was cooled to 20-30 °C, and water (500 g) and ethyl acetate (451 g) were added. A 4 M aqueous hydrochloric acid solution was added dropwise to adjust the pH of the reaction solution to 3-5, followed by liquid separation. The organic phase was washed three times

with a 10% sodium bicarbonate solution (500 g) and then with a 10% sodium chloride solution (500 g), and concentrated under reduced pressure to 1-2 V. Methanol (120 g) was added, the mixture was concentrated under reduced pressure to 1-2 V, and then methanol (605 g) was added to give a brownish-red solution of D6 in methanol (yield: 95%).

**Example 14: Preparation of Compound D8**

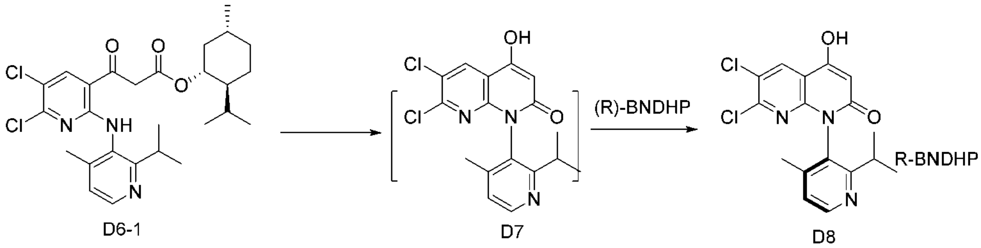


Methanol (200 mL, 10 V) and sodium carbonate (8.15 g, 2.0 eq) were added to 20 g of a crude compound D6-1. The mixture was stirred at 25-30 °C until the reaction was completed, and then filtered. The filter cake was rinsed with methanol. The combined filtrates were concentrated to dryness, and methanol (6 V) and (R)-(-)-1,1'-binaphthyl-2,2'-diyl hydrogenphosphate (R-BNDHP, 13.38 g, 1.0 eq) were added. The mixture was cooled to 0-5 °C, and isopropyl acetate (12 V) was added dropwise with the temperature maintained. The reaction solution was filtered, and the solid was collected and dried to give a compound D8 (17.8 g, three-step yield: 52.80%, R/S = 99.14:0.86). $^1$H NMR (400 MHz, DMSO-$d_6$) δ 12.58 (s, 1H), 8.56 (d, *J* = 5.1 Hz, 1H), 8.47 (s, 1H), 8.15 (d, *J* = 8.8 Hz, 2H), 8.08 (dd, *J* = 8.4, 1.3 Hz, 2H), 7.60 - 7.46 (m, 4H), 7.47 - 7.30 (m, 3H), 7.24 (d, *J* = 8.5 Hz, 2H), 6.06 (s, 1H), 4.86 (m, *J* = 6.3 Hz, 1H), 1.96 (s, 3H), 1.12 - 0.96 (m, 6H).

**Example 15: Preparation of Compound D8**

Acetone and water (9.5:1, V/V) were added to the concentrated solution described above instead of methanol. The reaction temperature was room temperature. The other operations were the same as those in Example 14 to give a compound D8 (purity: 97.33%, yield: 43.55%, R/S = 89.33/10.67).

**Example 16: Preparation of Compound D8**

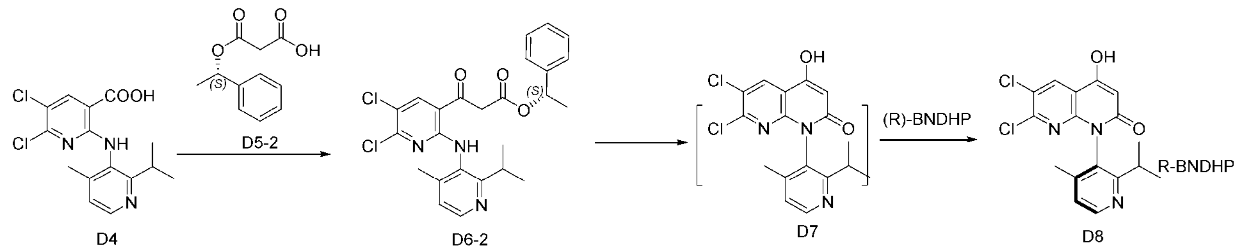


A compound D5-1 was replaced with a compound D5-2, and the other operations were performed as those in Example 11 and Example 14 to give a compound D8 (R/S = 65.44:34.56).

**Example 17: Preparation of Compound D8**

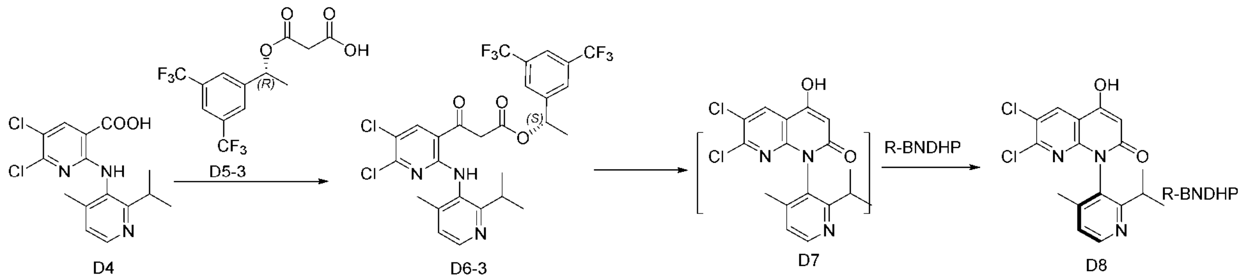

D4 D6-3 D7 D8

A compound D5-1 was replaced with a compound D5-3, and the other operations were performed as those in Example 11 and Example 14 to give a compound D8 (R/S = 54.02:45.98).

### Example 18: Preparation of Compound D8

To a reaction flask was added a solution of a compound D6-1 (10 g, 1.0 eq) in isopropyl acetate, and the mixture was concentrated under reduced pressure to 1-2 V. Methanol (40.0 g) was added, and the mixture was concentrated under reduced pressure to 1-2 V. Methanol (80.0 g) was added, and the mixture was stirred until complete dissolution was achieved. With the temperature controlled at 25±5 °C, 300-mesh sodium carbonate (6.1 g, 3.0 eq) was added. The mixture was stirred until the reaction was completed, and then filtered, and concentrated hydrochloric acid (36%) was added dropwise to the filtrate to adjust the pH to 3-5. The mixture was concentrated under reduced pressure to 2-2.5 V, and methanol (16.0 g, 1.6 w/w) was added. 20% sodium hydroxide was added dropwise to the system to adjust the pH of the system to 4.5-5.0. With the temperature controlled at 0-5 °C, (R)-(-)-1,1'-binaphthyl-2,2'-diyl hydrogenphosphate (6.7 g, 0.67 w/w) and isopropyl acetate (52.3 g, 5.23 w/w) were sequentially added to the system. The mixture was stirred for 24 h. Sampling, filtration, and detection: The impurity content of the S-configuration isomer in the filter cake was < 5.0%. With the temperature controlled at -15±5 °C, the mixture was stirred for 2-4 h. The mixture was filtered, and the filter cake was rinsed with isopropyl acetate (17.5 g, 1.75 w/w) and water (30 g, 3.0 w/w). The residue was dried to give a compound D8 (7.47 g, three-step yield: 55.1%).

### Example 19: Preparation of Compound D8

To a reaction flask were added a solution of a compound D6-1 (100 g, 192 mmol) in methanol and sodium carbonate (61.0 g, 576 mmol). The mixture was stirred for 48-72 h with the temperature maintained at 20-30 °C, and then filtered, and the filter cake was rinsed with methanol (79 g). Concentrated hydrochloric acid (60 g) was added dropwise to the filtrate to adjust the pH to 3-5. Then the mixture was concentrated under reduced pressure to 2-3 V, methanol (158 g) was added, and a 20% sodium hydroxide solution (10.0 g) was added dropwise to adjust the pH of the reaction solution to 4.5-5. The mixture was cooled to -5 °C to 5 °C, and (R)-(-)-1,1'-binaphthyl-2,2'-diyl hydrogenphosphate (66.9 g, 192 mmol) and isopropyl acetate (524 g) were added. The mixture was stirred at 20-30 °C for 24-48 h with the temperature maintained, and then cooled to -25 °C to -15 °C and stirred for 2-4 h with the temperature maintained. The mixture was filtered, and the filter cake was rinsed with isopropyl acetate (175 g) and water (300 g). The filter cake and water (1000 g) were stirred for 4-6 h with the temperature maintained at 20-30 °C. The mixture was filtered and dried to give a compound D8 (71.2 g, yield: 52%) as a white solid.

### Example 20: Preparation of Compound D9

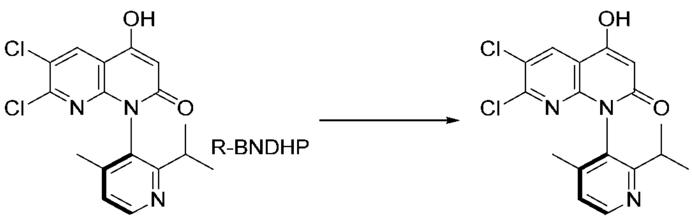

D8 D9

To a reaction kettle were added a compound D8 (1.60 kg, 1.0 eq) and acetonitrile (8 L, 5 V) at room temperature, and the mixture was stirred. Methanesulfonic acid (2.11 kg, 5.0 eq) was added dropwise to the system, and the mixture was

stirred with the temperature maintained at 20-25 °C until the reaction was completed. The mixture was filtered, and the filter cake was rinsed once with acetonitrile (1 V). Water (5 V) was added to the filtrate, and the mixture was then concentrated under reduced pressure to remove acetonitrile. The pH of the aqueous phase was adjusted to 5-6 with a 6 M sodium hydroxide solution, and the reaction solution was stirred at room temperature for 1 h. The mixture was filtered, and the filter cake was rinsed once with water (2 V) and dried to give a compound D9 (810 g, purity: 76.6%, yield: 90.39%). $^1H$ NMR (400 MHz, DMSO-$d_6$) δ 8.50 (d, *J* = 4.9 Hz, 1H), 8.46 (s, 1H), 7.27 (d, *J* = 4.8 Hz, 1H), 6.02 (s, 1H), 4.04 (q, *J* = 7.1 Hz, 1H), 1.90 (s, 3H), 1.01 (dd, *J* = 24.7, 6.7 Hz, 6H).

**Example 21: Preparation of Compound D9**

To a reaction flask were added acetonitrile (159.89 g, 9.40 w/w) and methanesulfonic acid (35.26 g, 2.08 w/w) at room temperature. With the temperature controlled at 20-30 °C, D8 (17 g in total, 42.5% content) was added in portions, and the mixture was stirred until the reaction was completed. The reaction solution was filtered, and the filtrate was concentrated under reduced pressure to 6-7 V. With the temperature controlled at 20-30 °C, water (10 V) was added, and the pH of the reaction solution was adjusted to 4.5-5.2 with a 10% sodium hydroxide solution. 2-Methyltetrahydrofuran (10 V) was added to the system described above, followed by liquid separation. The organic phase was washed with a 5% sodium chloride solution (4 V). The organic phase was concentrated under reduced pressure, and then isopropyl acetate (8 V) was added. The mixture was concentrated under reduced pressure again to 5-6 V. With the temperature controlled at 50-60 °C, n-heptane (8 V) was added. The mixture was stirred, and filtered, and the filter cake was dried to give a compound D9 (15.3 g, yield: 90%).

**Example 22: Preparation of Compound D10**

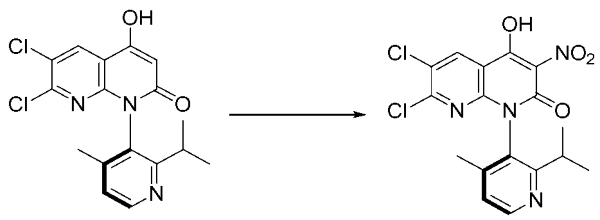
D9 D10

To a reaction flask was added acetic acid (1200 mL, 3 V), and the reaction solution was cooled to 20-25 °C. 95% fuming nitric acid (146 g, 1.0 eq mmol) was added dropwise and slowly, with the temperature controlled at 20-30 °C. After the dropwise addition was completed, a compound D9 (400 g, 1.0 eq) was added in portions with the internal temperature controlled at 17-28 °C, and the mixture was stirred until the reaction was completed. Water (34 V, 13.6 L) was added to the system, and then the system was stirred for 2 h, cooled to 10-15 °C, and filtered. The filter cake was rinsed with water (800 mL, 2 V), dried, and slurried with methanol (2000 mL, 5 V) for 1 h, and the mixture was filtered and dried to give a compound D10 (367 g, purity: 98.57%, yield: 100%). $^1H$ NMR (400 MHz, DMSO-$d_6$) δ 8.74 (d, *J* = 5.7 Hz, 1H), 8.45 (s, 1H), 7.83 (s, 1H), 2.94 (p, *J* = 6.9 Hz, 1H), 2.17 (s, 3H), 1.17 (dd, *J* = 29.4, 6.9 Hz, 6H).

**Example 23: Preparation of Compound D10**

To a reaction flask A was added acetic acid (54 g), and with the temperature controlled at 20-30 °C, fuming nitric acid (7 g) was added dropwise and slowly. To a reaction flask B were added a compound D9 (20 g) and acetic acid (64 g), and the mixture was stirred until complete dissolution was achieved. With the temperature controlled at 20-30 °C, the solution in the reaction flask B was added dropwise and slowly to the reaction flask A. After the dropwise addition was completed, the mixture was stirred at 20-30 °C until the reaction was completed. Water (400 g) was then added dropwise, and the mixture was stirred at 20-30 °C for 1-2 h and then filtered. The filter cake was slurried with water (200 g) and acetonitrile (80 g) with stirring, and the slurry was filtered. The filter cake was rinsed with a mixed solvent of acetonitrile (16 g) and water (40 g), and dried to give a compound D10 (21.30 g, yield: 94.6%, purity: 99.5%) as a yellow powdery solid.

**Example 24: Preparation of Compound D10**

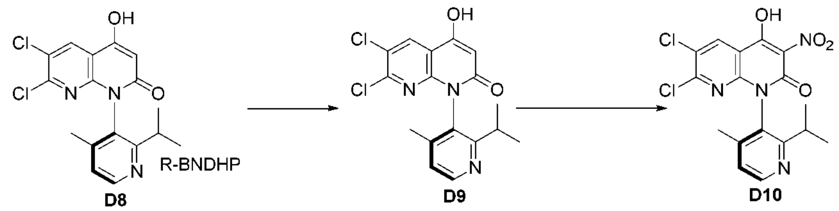

D8 D9 D10

To a reaction flask A were added D8 (225 g) containing compound D9 (100 g, 275 mmol) and acetonitrile (1179 g), and methanesulfonic acid (212 g, 2196 mmol) was added to the reaction flask A with stirring. The mixture was stirred for 4-8 h with the temperature maintained at 20-30 °C, and then filtered. The filter cake was rinsed with acetonitrile (236 g). The filtrate was transferred to a reaction flask B and concentrated under reduced pressure until no distillate was obtained. Acetic acid (158 g) was added to the reaction flask B and concentrated under reduced pressure until no distillate was obtained to give a light yellow solution D9. To a reaction flask C were added acetic acid (149 g) and 95% fuming nitric acid (34.8 g, 552 mmol), and the mixture was mixed well. The materials in the reaction flask B and the materials in the reaction flask C were subjected to a nitration reaction in a fluid reactor. After the nitration reaction was completed, with the temperature controlled at 20-30 °C, the reaction solution was added dropwise to water (3760 g) with stirring. Then, a 30% sodium hydroxide solution was added dropwise to adjust the pH of the reaction solution to 3-4, and the mixture was stirred for 2-3 h with the temperature maintained. The mixture was filtered, and the filter cake was rinsed with water (200 g). The dried crude product was slurried with methanol (1120 g) at 20-30 °C for 8-12 h, and then filtered. The filter cake was rinsed with methanol (149 g) and dried to give a compound D10 (98.9 g, yield: 88%) as a yellow solid.

**Example 25: Preparation of Compound D13**

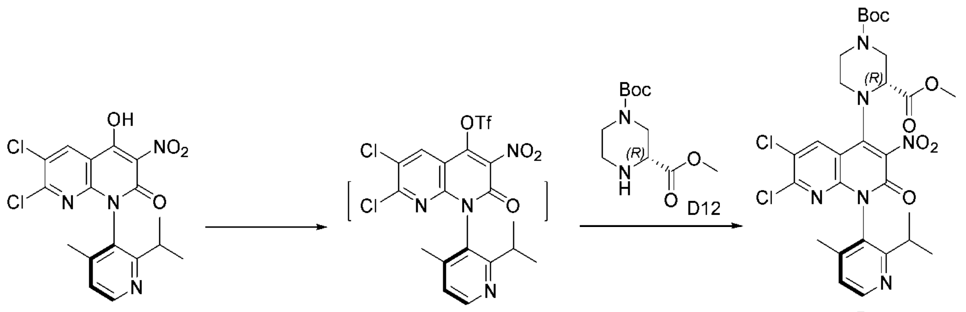

D10 D11 D13

To a reaction kettle were added a compound D10 (330 g, 1 eq) and dichloromethane (3.3 L, 10 V), and the mixture was stirred. N-methylmorpholine (245 g, 3 eq) was added, and the system turned clear. The mixture was cooled to an internal temperature of -20 °C to -30 °C, and trifluoromethanesulfonic anhydride (375 g, 1.65 eq) was added dropwise. With the temperature controlled at -20 °C to -30 °C, the mixture was stirred with the temperature maintained until the reaction was completed.

With the temperature controlled at -20 °C to -30 °C, a solution of a compound D12 (256 g, 1.3 eq) in dichloromethane (2 V) was added dropwise. After the dropwise addition was completed, the mixture was stirred until the reaction was completed. Water (5 V) was added, and the mixture was stirred for layer separation. The organic phase was washed once with a saturated aqueous sodium bicarbonate solution (5 V) and once with an aqueous sodium chloride solution (5 V). The organic phase was collected and concentrated to dryness by rotary evaporation, then ethyl acetate (2 V) was added, and n-heptane (20 V) was added with stirring. The mixture was stirred at 20-30 °C for 2-3 h. The mixture was filtered, and the filter cake was rinsed with n-heptane (1 V) and dried to give a compound D13 (501.2 g, purity: 94.88%, yield: 95.18%). $^1$H NMR (400 MHz, DMSO-$d_6$) $\delta$ 8.56 (d, $J$ = 4.8 Hz, 2H), 7.37 - 7.28 (m, 1H), 4.52 (s, 1H), 4.31 (d, $J$ = 13.5 Hz, 1H), 4.12 - 3.85 (m, 3H), 3.67 (s, 3H), 3.52 (s, 2H), 3.12 (s, 1H), 1.98 (s, 3H), 1.43 (s, 9H), 1.03 (dd, $J$ = 13.4, 6.7 Hz, 6H).

**Example 26: Preparation of Compound D13**

To a reaction flask were added a compound D10 (100 g) and dichloromethane (1320 g), and N-methylmorpholine (98.87 g) was added with stirring. With the temperature controlled at -55 °C to -45 °C, trifluoromethanesulfonic anhydride (103.42 g) was added dropwise. After the dropwise addition was completed, the mixture was stirred with the temperature

maintained until the reaction was completed. A solution of a compound D12 (80 g) in dichloromethane (300 g) was added dropwise with the internal temperature controlled at -55 °C to -45 °C. After the dropwise addition was completed, the mixture was stirred with the temperature maintained until the reaction was completed, and then heated to 0±5 °C. Water (500 g) was added, and hydrochloric acid was added dropwise to adjust the pH of the reaction solution to 2-3. The mixture was stirred for 1-2 h, followed by liquid separation. The organic phase was washed twice with an aqueous hydrochloric acid solution (2 g of concentrated hydrochloric acid, 498 g of water), then washed twice with a 5% sodium bicarbonate solution (500 g), washed once with water (500 g), and then filtered. The filter cake was rinsed with dichloromethane (300 g) to give a solution of a compound D13 in dichloromethane, which was directly used in the next step.

**Example 27: Preparation of Compound D13**

To a reaction flask were added D10 (100 g, 244 mmol), dichloromethane (1125 g), and N-methylmorpholine (74.2 g, 733 mmol). The mixture was cooled to -45 °C to -35 °C, and trifluoromethanesulfonic anhydride (96.5 g, 342 mmol) was added dropwise. After the dropwise addition was completed, the mixture was stirred for 1-1.5 h with the temperature maintained. Then a solution of a compound D12 (77.6 g, 318 mmol) in dichloromethane (265 g) was added dropwise to the reaction flask. After the dropwise addition was completed, the mixture was stirred for 1-1.5 h with the temperature maintained at -45 °C to -35 °C. The mixture was heated to 15-25 °C, water (500 g) was then added, and the mixture was stirred for layer separation. The organic phase was sequentially washed twice with a 0.1 N hydrochloric acid solution (500 g), with a 9% aqueous sodium bicarbonate solution (500 g), and with a 26% aqueous sodium chloride solution. The organic phase was concentrated under reduced pressure to 3.0-4.0 V, ethyl acetate (450 g) was then added, and n-Heptane (1360 g) was added dropwise at 40-50 °C. The mixture was stirred for 3.0-4.0 h with the temperature maintained at 20-30 °C. The reaction solution was filtered, and the filter cake was rinsed with n-heptane (136 g) and dried to give a compound D13 (139.8 g, yield: 90%) as a brown solid.

**Example 28: Preparation of Compound D14**

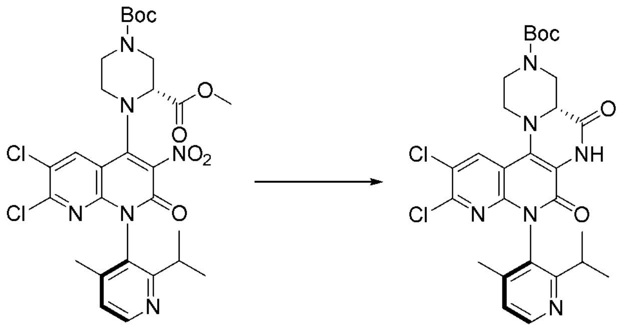

D13 D14

To a reaction kettle were added a compound D13 (500 g, 1 eq) and 1,4-dioxane (5 L, 10 V), and the mixture was stirred until complete dissolution was achieved. Sodium hydrosulfite (465 g, 3.4 eq) was dissolved in water (3 V), and the mixture was stirred until it turned clear. With the temperature controlled at 20-30 °C, an aqueous sodium hydrosulfite solution was added dropwise to the reaction kettle. After the dropwise addition was completed, the system was purged with argon. The mixture was heated to 75-85 °C and reacted until the reaction was completed. The reaction solution was cooled to 20-30 °C, and water (10 V) and ethyl acetate (15 V) were added, followed by liquid separation. The organic phase was concentrated under reduced pressure, and then isopropyl acetate (5 V) was added. Concentration was performed, and isopropyl acetate (2 V) was added to the concentrated crude product. The mixture was stirred for 2 h with the temperature maintained at 0-10 °C. The mixture was filtered, and the filter cake was rinsed with isopropyl acetate (0.5 V). The residue was dried to give a compound D14 (400.97 g, purity: 97.37%, yield: 89.33%). $^1$H NMR (400 MHz, DMSO-$d_6$) δ 10.50 (s, 1H), 8.56 (d, $J$ = 4.9 Hz, 1H), 8.45 (s, 1H), 7.32 (dd, $J$ = 4.8, 0.9 Hz, 1H), 4.94 - 4.37 (m, 2H), 4.04 (q, $J$ = 7.1 Hz, 1H), 3.87 (d, $J$ = 29.7 Hz, 2H), 2.76 (s, 1H), 2.60 (m, $J$ = 6.6 Hz, 2H), 1.85 (s, 3H), 1.45 (s, 9H), 1.05 (dd, $J$ = 27.7, 6.6 Hz, 6H).

**Example 29: Preparation of Compound D14**

To a hydrogenation flask were added a solution of a compound D13 in dichloromethane (actual amount of D13: 155 g), 1% platinum on activated carbon (10.85 g), and isopropyl acetate (527 g). The reaction solution was sequentially

purged with nitrogen and hydrogen, and adjusted to a pressure of 0.1-0.3 MPa. With the temperature controlled at 20-30 °C, the reaction solution was stirred until the reaction was completed. The mixture was filtered, and the filtrate was transferred to a reaction flask. Acetic acid (31 g) was added, and the mixture was heated to 20-30 °C and stirred until the reaction was completed. A 5% aqueous sodium bicarbonate solution (775 g) was then added dropwise. After the dropwise addition was completed, the mixture was stirred for 1-2 h and left to stand for liquid separation. The organic phase was washed once with water (500 g), and then concentrated under reduced pressure to 3-4 V, then isopropyl acetate (697.5 g) was added, and the mixture was concentrated with solvent entrainment for 2 times to 3-4 V, and then stirred for 1-2 h. n-Heptane (418.5 g) was added dropwise and slowly. After the dropwise addition was completed, the mixture was stirred for 1-2 h, with the temperature controlled at 80±5 °C. The mixture was slowly cooled to 0-10 °C and stirred for 5 h. The mixture was filtered, and the filter cake was rinsed with a mixed solution of n-heptane (155 g) and isopropyl acetate (155 g). The residue was dried to give a compound D14 (106.08 g, purity: 99.9%, yield: 80.0%) as a yellow solid.

**Example 30: Preparation of Compound D15**

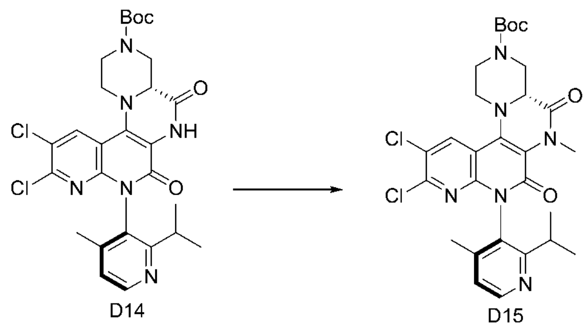

D14 D15

To a reaction kettle were added a compound D14 (400 g, 1.0 eq) and N,N-dimethylformamide (4 L, 10 V). Potassium carbonate (193.0 g, 2.0 eq) was added to the reaction kettle with stirring, and the resulting mixture was stirred homogeneously. The mixture was stirred for 2 h with the temperature maintained at 20-30 °C. Dimethyl sulfate (132 g, 1.5 eq) was added dropwise and slowly to the reaction kettle with the temperature controlled at 20-30 °C. After the addition was completed, the mixture was stirred with the temperature maintained until the reaction was completed. Water (20 V) was added dropwise to the reaction kettle with the temperature controlled at 20-30 °C. After the dropwise addition was completed, the mixture was stirred with the temperature maintained for 2 h. The mixture was filtered, and the filter cake was rinsed with water (2 V). The filter cake was dissolved in dichloromethane (10 V) and extracted once with water (10 V). After liquid separation, the aqueous layer was washed once with dichloromethane (10 V). The organic phases were combined and concentrated to give a compound D15 (415 g, purity: 97.36%, yield: 92.09%). $^1$H NMR (400 MHz, DMSO-$d_6$) δ 8.53 (d, *J* = 4.9 Hz, 1H), 8.50 (s, 1H), 7.30 (dd, *J* = 4.9, 0.9 Hz, 1H), 4.65 (d, *J* = 13.3 Hz, 1H), 3.85 (d, *J* = 40.6 Hz, 2H), 3.30 (s, 3H), 3.44 - 3.34 (dd, 2H), 3.13 (s, 1H), 2.71 (dt, *J* = 14.7, 7.3 Hz, 2H), 1.80 (s, 3H), 1.44 (s, 9H), 1.06 (dd, *J* = 19.8, 6.7 Hz, 6H).

**Example 31: Preparation of Compound D15**

To a reaction flask were sequentially added a compound D14 (60 g), potassium carbonate (42 g), NMP (255 g), and DMSO (270 g), and the mixture was stirred with the temperature controlled at -10-0 °C. Dimethyl sulfate (19.8 g) was added dropwise with the temperature controlled at -10-0 °C. After the dropwise addition was completed, the mixture was stirred until the reaction was completed. The reaction system was added dropwise and slowly to water (810 g). The mixture was stirred for 1-3 h with the temperature controlled at 20-30 °C. The mixture was filtered, and the filter cake was rinsed with water (300 g). The filter cake was transferred to the reaction flask, and dichloromethane (780 g), water (570 g), and sodium chloride (30 g) were added. The mixture was stirred until complete dissolution was achieved, and then left to stand for 2-5 min for liquid separation. The organic phase was collected. The aqueous phase was extracted once with dichloromethane (180 g). The organic phases were combined, washed once with water (300 g), and then concentrated under reduced pressure to 2-3 V. Dichloromethane (420 g) was added, and the mixture was concentrated under reduced pressure to 2-3 V. Methyl tert-butyl ether (300 g) was added, and the mixture was concentrated under reduced pressure to 2-3 V. n-Heptane (120 g) was added. The mixture was heated to 50-60 °C and stirred for 3-5 h. The reaction solution was slowly cooled to 20±5 °C and filtered. The filter cake was dried to give a compound D15 (54.1 g, purity: 99.7%, yield: 95.8%) as a yellow solid.

**Example 32: Preparation of Compound D15**

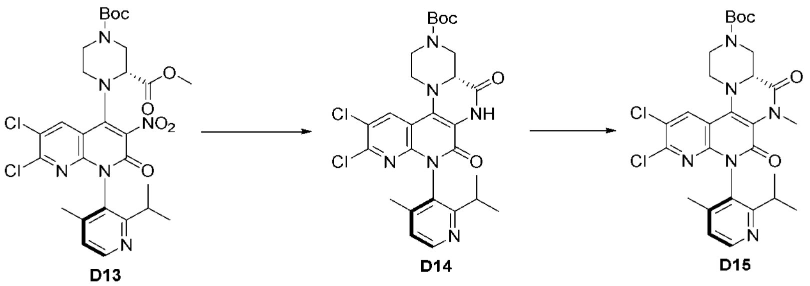

To a reaction flask A were added a compound D13 (50 g, 79 mmol) and 1,4-dioxane (515 g), and the mixture was stirred until complete dissolution was achieved. To a reaction flask B were added water (150 g) and sodium dithionite (46.6 g, 267 mmol), and the mixture was stirred until complete dissolution was achieved. The materials in the reaction flask B were added to the reaction flask A. The mixture was stirred for 16-20 h with the temperature maintained at 75-85 °C. The mixture was cooled to 20-30 °C, and water (500 g) and ethyl acetate (675 g) were added. The mixture was stirred, followed by layer separation. The organic phase was concentrated under reduced pressure to 1.5-2.5 V. Ethyl acetate (90 g) was added, and the mixture was concentrated with solvent entrainment for 3 times to 1.5-2.5 V, then N,N-dimethylformamide (428.6 g) was added, and the mixture was concentrated under reduced pressure to 9.5-11.0 V. Potassium carbonate (29 g, 210 mmol) was added, and dimethyl sulfate (19.85 g, 160 mmol) was added dropwise. The mixture was stirred for 16-20 h with the temperature maintained at 20-30 °C. Potassium carbonate (7.18 g, 52 mmol) was then added, and dimethyl sulfate (4.96 g, 39 mmol) was added dropwise. The mixture was stirred for 5-7 h with the temperature maintained at 20-30 °C. Water (902 g) was added to the reaction flask. The mixture was stirred for 2-3 h with the temperature maintained at 20-30 °C, and then filtered. The filter cake was rinsed with water (90 g). Dichloromethane (600 g) and water (451 g) were added to the filter cake, and the mixture was stirred until complete dissolution was achieved, followed by liquid separation. The organic phase was separated, and the aqueous phase was washed twice with dichloromethane (600 g). The organic phases were combined and concentrated to 1.5-2.2 V, then n-Heptane (614 g) was added dropwise at 25-35 °C, and the mixture was stirred for 1-2 h with the temperature maintained. The mixture was slowly cooled to 0-5 °C, stirred for 1.5-2.5 h with the temperature maintained, and then filtered. The filter cake was rinsed with n-heptane (61.4 g), and dried to give a compound D15 (37 g, two-step yield: 80%) as a brown solid.

**Example 33: Preparation of Compound D17**

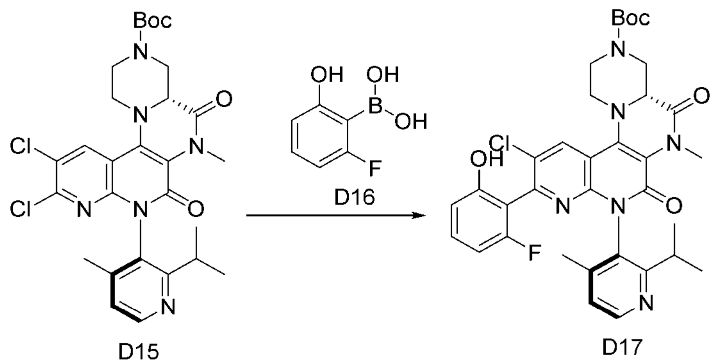

To a reaction kettle were added a compound D15 (415 g, 1.0 eq), 1,4-dioxane (6.56 L, 16 V), and water (1.6 L, 4 V). The reaction system was purged with argon. Potassium carbonate (117.0, 1.2 eq) and tetrakis(triphenylphosphine) palladium (20.0 g, 0.025 eq) were added. The reaction system was purged with argon. After the addition was completed, the mixture was heated to 75-85 °C, and a solution of a compound D16 in aqueous dioxane (D16 (165.0 g, 1.5 eq) dissolved in 1,4-dioxane (1.64 L, 4 V) and water (0.4 L, 1 V)) was added dropwise. After the dropwise addition was completed, the mixture was reacted at 75-85 °C until the reaction was completed. The reaction solution was cooled to 20-30 °C and concentrated under reduced pressure, and 2-methyltetrahydrofuran (10 V) and water (10 V) were added, followed by liquid

separation. The organic phase was retained. The aqueous phase was washed once with 2-methyltetrahydrofuran (5 V). The organic phases were combined and concentrated under reduced pressure. Ethanol (4 V) was added to the concentrated material, and the mixture was heated to 40 °C, followed by addition of water (4 V). After the addition was completed, the mixture was stirred for 1 h with the temperature maintained at 40-45 °C. The mixture was naturally cooled to 25 °C, then continuously cooled to 0-10 °C, and stirred for 1 h with the temperature maintained. The mixture was filtered and dried to give a compound D17 (366.9 g, purity: 97.63%, yield: 85.88%). $^1$H NMR (400 MHz, DMSO-$d_6$) δ 10.02 (d, *J* = 6.0 Hz, 1H), 8.44 (d, *J* = 5.1 Hz, 2H), 7.23 (q, *J* = 8.3 Hz, 2H), 6.70 (d, *J* = 8.3 Hz, 1H), 6.68 - 6.61 (m, 1H), 4.69 (d, *J* = 13.3 Hz, 1H), 4.04 (q, *J* = 7.1 Hz, 1H), 3.90 (d, *J* = 31.2 Hz, 2H), 3.49 (d, *J* = 12.0 Hz, 2H), 3.35 (s, 3H), 3.30 (s, 2H), 1.80 (d, *J* = 10.1 Hz, 3H), 1.47 (s, 9H), 1.04 (dd, *J* = 55.3, 6.2 Hz, 6H).

**Example 34: Preparation of Compound D17**

To a reaction flask A were added a compound D15 (40 g), dipotassium hydrogen phosphate (28.4 g), 1,4-dioxane (660 g), and water (160 g), and the mixture was stirred, with the internal temperature controlled at 25±5 °C. The reaction solution was purged with nitrogen and heated to 90±5 °C, and tetrakis(triphenylphosphine)palladium (1.18 g) was added.

To a reaction flask B were added D16 (16 g), 1.4-dioxane (164 g), and water (40 g), with the internal temperature controlled at 25±5 °C, and the reaction system was stirred and purged with nitrogen.

The temperature of the reaction flask was controlled at 90±5 °C. The solution in the reaction flask B was added dropwise to the reaction flask A. After the dropwise addition was completed, the mixture was stirred until the reaction was completed, then cooled to below 50 °C, and left to stand for layer separation. The organic phase was concentrated under reduced pressure to 6-7 V and then cooled to 25±5 °C, and a mixed solution of ethanol (120 g) and water (304 g) was added dropwise and slowly to the concentrated system. After the dropwise addition was completed, the mixture was stirred for another 2-4 h and filtered. The filter cake was washed with water (200 g). Dichloromethane (240 g) and water (200 g) were added to the filter cake. The mixture was stirred until complete dissolution was achieved, and then left to stand for layer separation. The organic phase was collected, and the aqueous phase was extracted once with dichloromethane (120 g). The organic phases were combined and washed once with water (200 g). The modified resin (2 g) was added to the organic phase. The mixture was stirred at 20-30 °C for 4-6 h and then filtered. Dichloromethane (40 g) was added for rinsing, and the organic phase was concentrated under reduced pressure to 3-4 V. Dichloromethane (240 g) was added again, and the mixture was concentrated under reduced pressure to 3-4 V. Methyl tert-butyl ether (40 g) was added dropwise to the concentrated system, and the mixture was stirred for 2 h with the internal temperature controlled at 30-36 °C. Methyl tert-butyl ether (240 g) was then added dropwise for 3-4 h. After the dropwise addition was completed, n-heptane (240 g) was added dropwise for 3-4 h. The mixture was slowly cooled to 20-30 °C and stirred for more than 9 h. The mixture was filtered, and the filter cake was rinsed with n-heptane (120 g) and dried to give a compound D17 (41.50 g, purity: 99.2%, yield: 91.9%) as an off-white powdery solid.

**Example 35: Preparation of Compound D17**

To a reaction flask A were added a compound D15 (500 g, 851 mmol), 1,4-dioxane (8272 g), and water (2000 g). The reaction system was purged with nitrogen and heated to 75-85 °C. Dipotassium hydrogen phosphate (355.8 g, 2042 mmol) and tetrakis(triphenylphosphine)palladium (19.7 g, 17 mmol) were added. To a reaction flask B were added 1,4-dioxane (2060 g), water (500 g), and compound D16 (185.8 g, 1192 mmol). The reaction system was purged with nitrogen and stirred until complete dissolution was achieved. With the temperature of the reaction flask A controlled at 75-85 °C, the solution in the reaction flask B was added dropwise to the reaction flask A in three portions. After each dropwise addition was completed, the mixture was stirred with the temperature maintained for 30-60 min. After the dropwise addition was completed, the mixture was stirred for 8-16 h with the temperature maintained at 75-85 °C, and then cooled to 35-45 °C. The pH of the reaction solution was adjusted to 5.5-6.5 with acetic acid, and then the mixture was concentrated under reduced pressure to 4.5-5.5 V. 2-Methyltetrahydrofuran (4315 g) and a 20% sodium chloride solution (3750 g) were added, and the mixture was stirred. The organic phase was separated, and the aqueous phase was washed with 2-methyltetrahydrofuran (2157 g). The organic phases were combined, activated carbon (50 g) was added, and the mixture was stirred for 30-60 min with the temperature maintained at 45-55 °C, and then filtered. The filter cake was rinsed with 2-methyltetrahydrofuran (863 g). The filtrates were combined, and then modified resin (50 g) was added. The mixture was stirred at 45-55 °C for 2.5-3.5 h, and then filtered. The filter cake was rinsed with 2-methyltetrahydrofuran. The filtrates were concentrated under reduced pressure to 1.5-2.0 V, then ethanol (1578 g) was added, and the mixture was concentrated for 2 times with solvent entrainment to 1.5-2.0 V. Ethanol (1184 g) was added, and the mixture was heated to 35-45 °C. Water (2000 g) was added dropwise, and the mixture was stirred at 35-45 °C for 1-2 h, cooled to 0-10 °C, stirred with the temperature maintained for 1-2 h, and then filtered. The filter cake was rinsed with ethanol (200 g) and dried to give a compound D17 (508 g, yield: 90%) as a brownish-yellow solid.

Example 36: Preparation of Compound D18

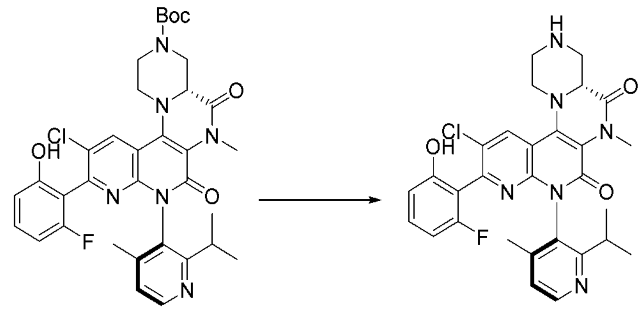

D17 D18

To a reaction kettle were added a compound D17 (360.0 g, 1.0 eq) and methanol (2.8 kg, 8 W). A 4 M solution of hydrogen chloride in dioxane (1.4 L, 10.0 eq) was added dropwise with stirring until complete dissolution was achieved. After the reaction was completed, the mixture was concentrated under reduced pressure. Water (10 W) and dichloromethane (13 W) were added to the concentrated material, followed by liquid separation. The aqueous layer was washed once with dichloromethane (6.5 W). The aqueous layer was collected and filtered through celite, and N,N-dimethylformamide (1.1 W) and methyl tert-butyl ether (1.5 W) were added to the aqueous layer. With the temperature controlled at 10-20 °C, a 10% aqueous sodium carbonate solution was added dropwise to the system for 3-4 h to adjust the pH to 7-8. After the dropwise addition was completed, n-heptane (2.0 W) was added dropwise and slowly with the temperature controlled at 10-20 °C. After the dropwise addition was completed, the mixture was stirred for 16-20 h with the temperature maintained. The mixture was filtered, and the filter cake was rinsed once with water (2 W). The filter cake was dried to give a compound D18 (309.05 g, purity: 98.93%, yield: 100%). $^1$H NMR (400 MHz, DMSO-$d_6$) δ 10.02 (s, 1H), 8.44 (d, *J* = 4.9 Hz, 1H), 8.41 (s, 1H), 7.31 - 7.14 (m, 2H), 6.70 (d, *J* = 8.3 Hz, 1H), 6.66 (t, *J* = 8.8 Hz, 1H), 3.61 (dd, *J* = 8.2, 4.8 Hz, 2H), 3.37 (s, 3H), 3.02 (dd, *J* = 13.5, 4.4 Hz, 2H), 2.91 (d, *J* = 13.5 Hz, 1H), 2.75 (q, *J* = 6.8 Hz, 1H), 2.63 (dd, *J* = 12.5, 9.6 Hz, 2H), 1.80 (d, *J* = 9.8 Hz, 3H), 1.21 - 0.84 (m, 6H).

**Example 37: Preparation of Compound D18**

To a reaction flask A were added D17 (25 g), modified resin (1.25 g), and water (125 g). The mixture was stirred and heated to 50-60 °C. Concentrated hydrochloric acid (11.5 g) was added dropwise with the temperature controlled at 50-60 °C. The mixture was stirred until the reaction was completed. The mixture was cooled to 20-30 °C and filtered. The filter cake was washed with water (25 g), and the filtrate was transferred to the reaction flask A. 2-methyltetrahydrofuran (100 g) was added, and the mixture was stirred and left to stand for layer separation. The organic phase was discarded, and the aqueous phase was washed once with methyl tert-butyl ether (100 g) and then transferred to the reaction flask A.

To a reaction flask B were added water (100 g), potassium bicarbonate (15 g), DMF (35 g), and MTBE (37.5 g). With the temperature controlled at 10-20 °C, the organic phase in the reaction flask A was added dropwise and slowly to the system in the reaction flask B for 2-4 h. After the dropwise addition was completed, n-heptane (50 g) was added dropwise to the reaction flask B. The mixture was cooled to 10-20 °C and filtered, and the filter cake was rinsed with water (75 g). The filter cake was dried to give a compound D18 (26.10 g, purity: 99.3%, yield: 100%), which was used directly in the next step.

**Example 38: Preparation of Compound D18**

To a reaction flask A were added a compound D17 (500 g, 754 mmol) and methanol (3955 g). A 4 M hydrogen chloride/1,4-dioxane solution (1949 g, 7540 mmol) was added dropwise with stirring, and the mixture was stirred for 1-2 h with the temperature maintained at 20-30 °C and then concentrated under reduced pressure to 3 V. Water (2500 g) was added to the reaction flask A, and the mixture was then concentrated under reduced pressure to 6 V. Water (2500 g) and dichloromethane (6625 g) were added to the reaction flask A, and the mixture was stirred, followed by layer separation. The organic phase was discarded, and the aqueous phase was washed three times with dichloromethane (3250 g) and filtered through celite. The filter cake was washed with water (1500 g), and the filtrate was transferred to a reaction flask B and left to stand. The organic phase was discarded, and N,N-dimethylformamide (550 g), methyl tert-butyl ether (750 g), and n-heptane (1000 g) were added to the reaction flask B. A 10% aqueous sodium carbonate solution (2500 g) was added dropwise to the reaction flask B to adjust the pH of the reaction solution to 7-8. The mixture was stirred for 16-20 h with the temperature maintained at 10-20 °C, and then filtered. The filter cake was rinsed with water (1000 g), then rinsed with n-heptane (700 g), and dried to give a compound D18 (403.3 g, yield: 95%) as a yellow solid.

**Example 39: Preparation of Compound of Formula I**

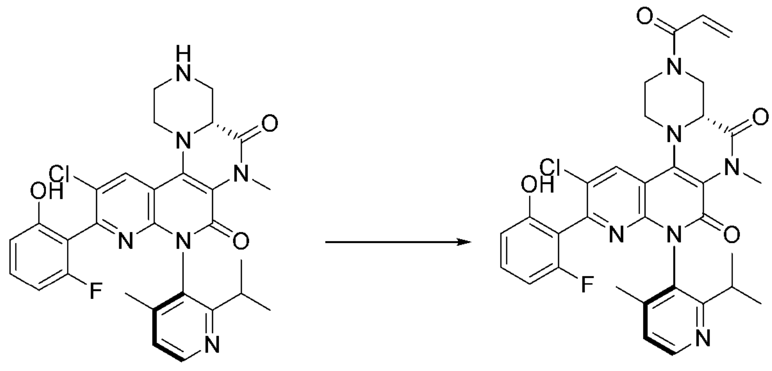

D18

I

To a reaction kettle were added a compound D18 (300.0 g, 1.0 eq), tetrahydrofuran (1200 g, 4 W), and water (675 g, 2.25 W). Acryloyl chloride (60.0 g, 0.2 W) was added dropwise to the reaction kettle with stirring with the temperature controlled at 0-10 °C until the reaction was completed. Water (6 W) was added to the reaction solution, and the mixture was extracted twice with dichloromethane (15 W). The combined organic phases were washed once with a 5% aqueous sodium bicarbonate solution (6 W) and extracted once with water (6 W). The organic phase was concentrated under reduced pressure, and butanone was added to the concentrated crude product. The mixture was concentrated with solvent entrainment until the contents of the residues of tetrahydrofuran and dichloromethane were qualified. After the reaction was completed as detected, butanone (2.0 W) was kept in the system, and the system was heated to 60-70 °C and stirred for 0.5-1 h with the temperature maintained. The mixture was cooled to 35-40 °C and stirred for 1-2 h with the temperature maintained. With the temperature controlled at 35-40 °C, methyl tert-butyl ether (0.8 W) was added, and the mixture was stirred at 35-40 °C for 2 h with the temperature maintained. Methyl tert-butyl ether (4 W) was then added dropwise, and after the dropwise addition was completed, the mixture was stirred at 35-40 °C for 2 h with the temperature maintained. The mixture was cooled to 15-20 °C and stirred for 16-20 h with the temperature maintained. The mixture was filtered, and the filter cake was rinsed with methyl tert-butyl ether. The filter cake was dried to give a compound of formula I (275.18 g, purity: 99.54%, yield: 84.96%).

To a reaction kettle were added the compound of formula I (270.0 g, 1.0 eq) obtained in the previous step and dichloromethane (1430.0 g, 5.3 W). After the addition was completed, the mixture was stirred for 6-10 h with the temperature controlled at 35-45 °C. The reaction system was cooled to 0-8 °C, and stirred for 16-20 h with the temperature controlled at 0-8 °C. The mixture was filtered, and the filter cake was rinsed once with dichloromethane (1.3 W) at 0-8 °C. The filter cake was dried to give the compound of formula I (248.46 g, purity: 99.65%, yield: 94.74%).

**Example 40: Preparation of Compound of Formula I**

To a reaction flask were added D18 (20 g), THF (80 g), and water (38.9 g), and the mixture was stirred and cooled to 5±5 °C. Acryloyl chloride (4 g) was added dropwise and slowly. After the dropwise addition was completed, the reaction system was reacted at 5±5 °C until the reaction was completed. Water (120 g) was added to the reaction solution and stirred for 2 h. Dichloromethane (300 g) was added, and the mixture was stirred and left to stand for layer separation. The organic phase was separated, and the aqueous phase was extracted once with dichloromethane (150 g). The organic phases were combined and washed once successively with diluted hydrochloric acid (200 g of water + 3.4 g of concentrated hydrochloric acid), a sodium bicarbonate solution (114 g of water + 6 g of sodium bicarbonate), and water (120 g). Butanone (88 g) was added to the organic phase for concentration with solvent entrainment for 2 times to 3.5-4.5 V. The mixture was heated to 60-70 °C and stirred for 1-2 h. The mixture was cooled to 30-38 °C, and a seed crystal (0.02 g) of a compound of formula I was added. The mixture was stirred for 1-2 h. With the temperature controlled at 30-38 °C, methyl tert-butyl ether (16 g) was added dropwise for 5-6 h. After the dropwise addition was completed, the mixture was stirred at 30-38 °C for 2-4 h. The mixture was cooled to 15-25 °C, stirred at 5-25 °C for 3-10 h, and then filtered. The filter cake was washed with methyl tert-butyl ether, and filtered and dried to give a crude product of the compound of formula I (18.24 g, yield: 83.2%).

To a reaction flask were added the crude product of the compound of formula I (50 g), dichloromethane (2500 g),

and activated carbon (0.5 g), and the mixture was stirred at 35-45 °C for 2-4 h. The reaction solution was cooled to 20-30 °C and filtered. The filter cake was rinsed with dichloromethane. The filtrate was concentrated under reduced pressure to 4-5 V. After the concentration was completed, the mixture was heated to 35-45 °C and stirred for 6-10 h with the temperature maintained at 35-45 °C. The mixture was cooled to 2-10 °C and stirred for 6-10 h with the temperature maintained at 2-10 °C. The mixture was filtered, and the filter cake was washed with pre-cooled (0-8 °C) dichloromethane (66.5 g). The wet product was dried to give 45 g of the compound of formula I (yield: 90%).

**Comparative Example 1: Preparation of Compound of Formula I**

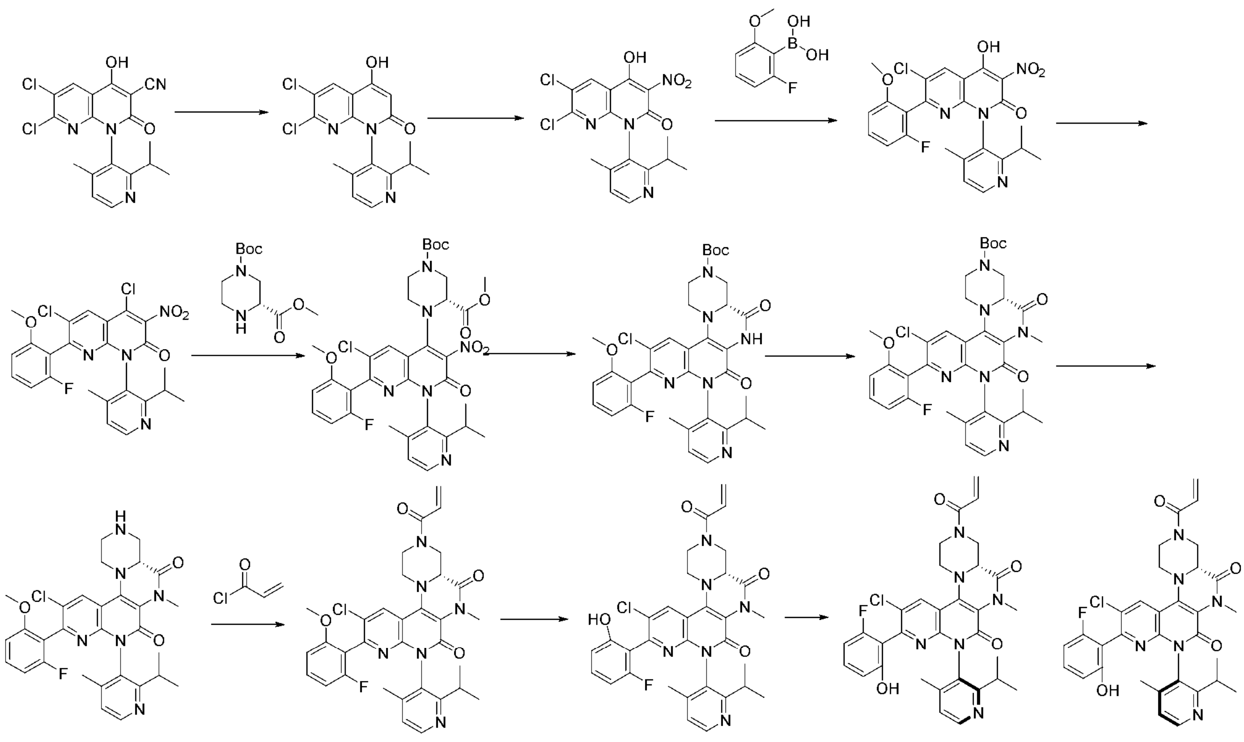

I

Step 1: 6,7-Dichloro-4-hydroxy-1-(2-isopropyl-4-methylpyridin-3-yl)-2-oxo-1,2-dihydro-1,8-naphthyridine-3-carbonitrile (30.0 g, 77.319 mmol) was suspended in a mixed solution of 1,4-dioxane (120 mL) and water (120 mL), and then concentrated sulfuric acid (120 mL) was slowly added. The reaction system was stirred at 120 °C for 36 h. The reaction solution was cooled, poured into 200 mL of ice water, adjusted to pH 2-3 with sodium carbonate, and extracted with ethyl acetate (1000 mL × 2). The ethyl acetate phases were combined, dried over anhydrous sodium sulfate, and filtered. The filtrate was dried in vacuum to give a product 6,7-dichloro-4-hydroxy-1-(2-isopropyl-4-methylpyridin-3-yl)-1,8-naphthyridin-2(1H)-one (24 g, yield: 85.7%) as a pale brown solid. ES-API: $[M+H]^+$ = 364.1.

Step 2: 6,7-Dichloro-4-hydroxy-1-(2-isopropyl-4-methylpyridin-3-yl)-1,8-naphthyridin-2(1H)-one (3.16 g, 8.705 mmol) was dissolved in acetic acid (15 mL), and then sodium nitrite (100 mg, 1.58 mmol) and concentrated nitric acid (5.0 mL, 74.52 mmol) were sequentially added. The reaction system was stirred at room temperature for 30 min. The reaction solution was slowly poured into 100 mL of ice water. The precipitated solid was filtered, and the filter cake was washed with 20 mL of ice water and dried in vacuum to give a product 6,7-dichloro-4-hydroxy-1-(2-isopropyl-4-methylpyridin-3-yl)-3-nitro-1,8-naphthyridin-2(1H)-one (3.5 g, yield: 92%) as a yellow solid. ES-API: $[M+H]^+$ = 409.1.

Step 3: To a 100-mL three-necked round-bottom flask were added 6,7-dichloro-4-hydroxy-1-(2-isopropyl-4-methylpyridin-3-yl)-3-nitro-1,8-naphthyridin-2(1H)-one (3.5 g, 8.570 mmol), (2-fluoro-6-methoxyphenyl)boronic acid (5.8 g, 34.10 mmol), tetrakis(triphenylphosphine)palladium (1.15 g, 0.9956 mmol), sodium carbonate (3.5 g, 33.02 mmol), 10 mL of water, and 40 mL of dioxane. The reaction system was stirred at 100 °C for 2-3 h under nitrogen atmosphere. After the reaction was completed, the reaction solution was cooled to room temperature, then 80 mL of water and 100 mL of methyl tert-butyl ether were added, and the mixture was extracted once. The aqueous phase was adjusted to pH 3-5 by adding a 1 M hydrochloric acid solution and extracted with ethyl acetate (200 mL × 2). The ethyl acetate phases were combined, dried over anhydrous sodium sulfate, and filtered. The filtrate was dried in vacuum to give a product 6-chloro-7-(2-fluoro-6-methoxyphenyl)-4-hydroxy-1-(2-isopropyl-4-methylpyridin-3-yl)-3-nitro-1,8-naphthyridin-2(1H)-one (4.5 g, crude) as a pale yellow solid. ES-API: $[M+H]^+$ = 499.1.

Step 4: 6-Chloro-7-(2-fluoro-6-methoxyphenyl)-4-hydroxy-1-(2-isopropyl-4-methylpyridin-3-yl)-3-nitro-1,8-naphthyridin-2(1H)-one (4.6 g, 8.57 mmol) was dissolved in acetonitrile (30 mL), and then phosphorus oxychloride (7.5 g, 48.92 mmol) and N,N-diisopropylethylamine (10.5 g, 81.24 mmol) were sequentially added. The reaction system was heated to 80 °C and stirred for 30 min. The reaction solution was concentrated, and 30 mL of cold acetonitrile was added. In an ice-water bath, the mixture was added dropwise to 150 mL of saturated sodium bicarbonate solution, and extracted with ethyl acetate (200 mL $\times$ 2). The ethyl acetate phases were combined and washed once with 200 mL of saturated brine. The mixture was dried over anhydrous sodium sulfate, and filtered. The organic phase was dried and concentrated, and the crude product was purified on a flash silica gel column (EtOAc/PE: 0-50%) to give 4,6-dichloro-7-(2-fluoro-6-methoxyphenyl)-1-(2-isopropyl-4-methylpyridin-3-yl)-3-nitro-1,8-naphthyridin-2(1H)-one (3.05 g, yield: 76%) as a yellow solid. ES-API: $[M+H]^+$ = 517.2.

Step 5: 4,6-Dichloro-7-(2-fluoro-6-methoxyphenyl)-1-(2-isopropyl-4-methylpyridin-3-yl)-3-nitro-1,8-naphthyridin-2(1H)-one (2.5 g, 4.843 mmol) was dissolved in N,N-dimethylacetamide (25 mL), and then 1-(tert-butyl) 3-methyl(R)-piperazine-1,3-dicarboxylate (3.5 g, 14.34 mmol) and N,N-diisopropylethylamine (2.0 g, 15.47 mmol) were sequentially added. The mixture was stirred at 120 °C for 2 h. 80 mL of ethyl acetate was added to the reaction solution, and the mixture was washed three times with 80 mL of saturated brine. The ethyl acetate phase was dried and concentrated, and the crude product was purified on a flash silica gel column (EtOAc/PE: 0-80%) to give a product 1-(tert-butyl) 3-methyl(3R)-4-(6-chloro-7-(2-fluoro-6-methoxyphenyl)-1-(2-isopropyl-4-methylpyridin-3-yl)-3-nitro-2-oxo-1,2-dihydro-1,8-naphthyridin-4-yl)piperazine-1,3-dicarboxylate (2.7 g, yield: 77%) as a yellow solid. ES-API: $[M+H]^+$ = 725.2.

Step 6: 1-(tert-Butyl) 3-methyl(3R)-4-(6-chloro-7-(2-fluoro-6-methoxyphenyl)-1-(2-isopropyl-4-methylpyridin-3-yl)-3-nitro-2-oxo-1,2-dihydro-1,8-naphthyridin-4-yl)piperazine-1,3-dicarboxylate (2.7 g, 3.728 mmol) was dissolved in acetic acid (30 mL), and then iron powder (835 mg, 14.91 mmol) was added. The mixture was stirred at 80 °C for 30 min. The reaction solution was concentrated, and 200 mL of ethyl acetate and 100 mL of saturated sodium bicarbonate were sequentially added. The suspension was filtered through celite, and the filter cake was washed with ethyl acetate. The organic phase was discarded, sequentially washed with 100 mL of saturated sodium bicarbonate and 150 mL of saturated brine, dried, and concentrated to give a product tert-butyl (4aR)-11-chloro-10-(2-fluoro-6-methoxyphenyl)-8-(2-isopropyl-4-methylpyridin-3-yl)-5,7-dioxo-1,2,4,4a,5,6,7,8-octahydro-3H-pyrazino[1',2':4,5]pyrazino[2,3-*c*][1,8]naphthyridine-3-carboxylate (2.70 g, crude) as a yellow solid. ES-API: $[M+H]^+$=663.2.

Step 7: To a 150-mL sealed tube were sequentially added tert-butyl (4aR)-11-chloro-10-(2-fluoro-6-methoxyphenyl)-8-(2-isopropyl-4-methylpyridin-3-yl)-5,7-dioxo-1,2,4,4a,5,6,7,8-octahydro-3H-pyrazino[1',2':4,5]pyrazino [2,3-*c*][1,8]naphthyridine-3-carboxylate (2.7 g, 3.728 mmol), 30 mL of acetone, anhydrous potassium carbonate (2.2 g, 15.94 mmol), and iodomethane (5.4 g, 38.03 mmol). After the sealed tube was sealed, the mixture was stirred at 55 °C for 18 h. 150 mL of ethyl acetate was added to the reaction solution, and the mixture was washed three times with 100 mL of saturated brine, dried, and concentrated. The crude product was purified on a flash silica gel column (EtOAc/PE: 0-80%) to give a product tert-butyl (4aR)-11-chloro-10-(2-fluoro-6-methoxyphenyl)-8-(2-isopropyl-4-methylpyridin-3-yl)-6-methyl-5,7-dioxo-1,2,4,4a,5,6,7,8-octahydro-3H-pyrazino[1',2':4,5]pyrazino[2,3-*c*][1,8]naphthyridine-3-carboxylate (2.2 g, yield: 87%) as a yellow solid. ES-API: $[M+H]^+$ = 677.2.

Step 8: tert-Butyl (4aR)-11-chloro-10-(2-fluoro-6-methoxyphenyl)-8-(2-isopropyl-4-methylpyridin-3-yl)-6-methyl-5,7-dioxo-1,2,4,4a,5,6,7,8-octahydro-3H-pyrazino[1',2':4,5]pyrazino[2,3-*c*][1,8]naphthyridine-3-carboxylate (517 mg,0.7549 mmol) was dissolved in dichloromethane (8 mL), and then trifluoroacetic acid (2 mL) was added. The reaction solution was stirred at room temperature for 2 h, and concentrated to give a product (4aR)-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5] pyrazino[2,3-c][1,8]naphthyridine-5,7-dione (530 mg, crude), which was directly used in the next reaction. ES-API: $[M+H]^+$ = 577.2.

Step 9: (4aR)-11-Chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-*c*][1,8]naphthyridine-5,7-dione (530 mg, 0.7549 mmol) was dissolved in dichloromethane (15 mL), and then triethylamine (3.0 mL, 21.62 mmol) was added. The reaction solution was cooled to 0 °C, and then acryloyl chloride (100 mg, 1.1048 mmol) was added dropwise. The mixture was stirred at 0 °C for 15 min. 80 mL of dichloromethane was added to the reaction solution. The mixture was washed with 100 mL of saturated aqueous $NaHCO_3$ solution and 80 mL of saturated brine, dried, and concentrated. The crude product was purified on a flash silica gel column (EtOAc/PE: 0-60%) to give a product (4aR)-3-acryloyl-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino [2,3-c][1,8]naphthyridine-5,7-dione (280 mg, yield: 59%) as a yellow solid. ES-API: $[M+H]^+$ = 631.2.

Step 10: In an ice-water bath, (4aR)-3-acryloyl-11-chloro-10-(2-fluoro-6-methoxyphenyl)-8-(2-isopropyl-4-methylpyridin-3-yl)-6-methyl-2,3,4,4a,6,8-hexahydro-1H-pyrazino[1',2':4,5]pyrazino[2,3-c][1,8]naphthyridine-5,7-dione (280 mg, 0.444 mmol) was added to anhydrous dichloromethane (6.0 mL), and then boron tribromide (5.0 mL, 5.0 mmol) was added. The mixture was heated to room temperature and reacted overnight. In an ice-water bath, the reaction solution

described above was added dropwise to a saturated sodium bicarbonate solution. The mixture was extracted twice with dichloromethane (80 mL), dried, and concentrated. The crude product was purified on a flash silica gel column (EtOAc/PE: 0-60%) to give a product (4aR)-3-acryloyl-11-chloro-10-(2-fluoro-6-hydroxyphenyl)-8-(2-isopropyl-4-methylpyridin-3-yl)-6-methyl-2,3,4,4a,6,8-hexahydro-1H-pyrazino[1',2':4,5]pyrazino[2,3-*c*][1,8]naphthyridine-5,7-dione (233 mg, yield: 85%).

Step 11: Compound (4aR)-3-acryloyl-11-chloro-10-(2-fluoro-6-hydroxyphenyl)-8-(2-isopropyl-4-methylpyridin-3-yl)-6-methyl-2,3,4,4a,6,8-hexahydro-1H-pyrazino[1',2':4,5]pyrazino[2,3-*c*][1,8]naphthyridine-5,7-dione was resolved by preparative chiral HPLC (column: IA: 10 μm, 30 × 250 mm, mobile phase: hexane:EtOH = 60:40, flow rate: 25 mL/min, column temperature: room temperature) to give: an atropisomer compound of formula I (76.8 mg, peak 1, retention time: 2.531 min, yield: 34%). $^1$H NMR (500 MHz, DMSO-$d_6$) δ 10.03 (d, *J* = 18.4 Hz, 1H), 8.52 (d, *J* = 7.3 Hz, 1H), 8.43 (d, *J* = 4.7 Hz, 1H), 7.23 (d, *J* = 9.6 Hz, 2H), 7.08 (dd, *J* = 16.6, 10.5 Hz, 1H), 6.74 - 6.62 (m, 2H), 6.15 (d, *J* = 16.8 Hz, 1H), 5.75 (d, *J* = 10.7 Hz, 1H), 4.73 (d, *J* = 14.2 Hz, 1H), 4.46 (d, *J* = 12.9 Hz, 1H), 4.00 (s, 1H), 3.61 (d, *J* = 10.0 Hz, 1H), 3.51 (s, 1H), 3.34 (s, 3H), 3.22 (s, 1H), 2.64 (t, *J* = 11.5 Hz, 1H), 2.48 - 2.42 (m, 1H), 1.98 (d, *J* = 5.1 Hz, 3H), 1.03 (t, *J* = 6.9 Hz, 3H), 0.86 (t, *J* = 7.9 Hz, 3H).ES-API: [M+H]$^+$=617.2; and another atropisomer compound (70 mg, peak 2, retention time: 3.683 min, yield: 31%). $^1$H NMR (500 MHz, $CDCl_3$) δ 8.64 - 8.59 (m, 1H), 8.35 (s, 1H), 8.07 (s, 1H), 7.27 - 7.20 (m, 2H), 7.14 - 7.02 (m, 1H), 6.75 - 6.63 (m, 2H), 6.39 (dd, *J* = 17.0, 2.0 Hz, 1H), 5.88 - 5.77 (m, 1H), 4.91 (d, J = 14.0 Hz, 1H), 4.83 (d, J = 13.0 Hz, 1H), 3.72 - 3.58 (m, 2H), 3.50 (s, 3H), 3.43 (d, *J* = 12.0 Hz, 1H), 3.16 (t, *J* = 13.0 Hz, 1H), 2.91 (t, *J* = 12.0 Hz, 1H), 2.82-2.73 (m, 1H), 1.93 (s, 3H), 1.24 (d, *J* = 7.0 Hz, 3H), 1.12 (d, *J* = 7.0 Hz, 3H). ES-API: [M+H]$^+$= 617.2. The isomeric compound was detected by analytical chiral HPLC method (column: IA: 5 μm, 4.6 × 150 mm, mobile phase: hexane:EtOH = 60:40, flow rate: 1 mL/min, column temperature = 30 °C).

In the preparation method, 6,7-dichloro-4-hydroxy-1-(2-isopropyl-4-methylpyridin-3-yl)-2-oxo-1,2-dihydro-1,8-naphthyridine-3-carbonitrile is used as a starting material, and the starting material is difficult to purchase on a large scale. The products in steps 4, 5, 7, 9 and 10 need to be purified by silica gel column chromatography. Moreover, the final product has chirality and needs to be resolved by chirality, resulting in high material consumption. The preparation method is for small-scale preparation in laboratories and is not suitable for large-scale industrial production.

In addition, it should be understood that various modifications or changes may be made by those skilled in the art after reading the above teachings of the present disclosure, and these equivalent forms also fall within the scope defined by the appended claims of the present application.

## Claims

**1.** A method for preparing a compound D9, comprising the following steps:

step D: subjecting a compound D6 to a ring-closing reaction in the presence of a base and then to a resolution reaction with a chiral resolving reagent to give a compound D8;

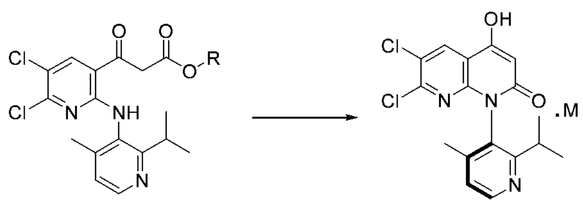

D6 D8 ;

and

step E: removing the chiral resolving reagent from the compound D8 to give the compound D9;

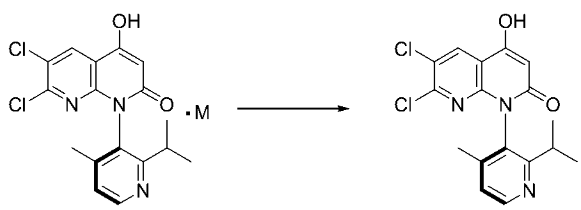

D8 D9 ;

wherein M is the chiral resolving reagent;
R is a chiral auxiliary group.

**2.** The preparation method according to claim 1, wherein R is a chiral auxiliary group, and is preferably selected from

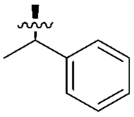, 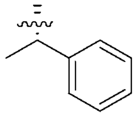, 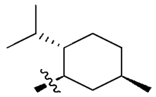, 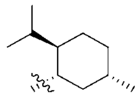, 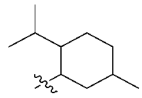, 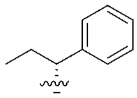, 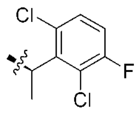,

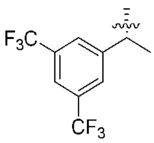, 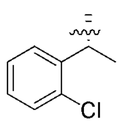, 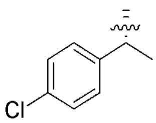, 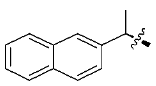, 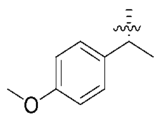, and 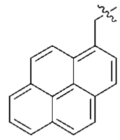;

and/or the chiral resolving reagent M is selected from one or more of D-(+)-di-p-toluoyl tartaric acid, N-acetyl-L-phenylalanine, L-(-)-dibenzoyl tartaric acid, riboflavin, L-(-)-camphorsulfonic acid, glutamic acid, L-tartaric acid, D-(+)-di-p-anisoyl tartaric acid, D-glycine, D-mandelic acid, R-methoxy-trifluoromethylphenylacetic acid, L-aspartic acid, D-(+)-dibenzoyl tartaric acid, L-pyroglutamic acid, and (R)-(-)-1,1'-binaphthyl-2,2'-diyl hydrogenphosphate;

preferably, the chiral resolving reagent M is selected from one or more of D-(+)-di-p-toluoyl tartaric acid, L-(-)-camphorsulfonic acid, L-tartaric acid, (R)-(-)-1,1'-binaphthyl-2,2'-diyl hydrogenphosphate, and D-(+)-dibenzoyl tartaric acid;

more preferably, the chiral resolving reagent M is (R)-(-)-1,1'-binaphthyl-2,2'-diyl hydrogenphosphate.

**3.** The preparation method according to claim 1 or 2, wherein the compound D9 is used for preparing a compound of formula I:

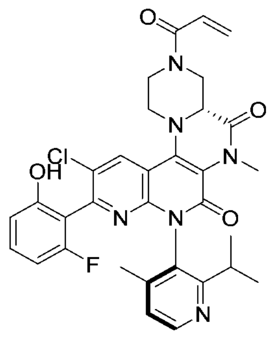

I ;

or for preparing any one or a combination of compounds selected from a compound D10, a compound D13, a compound D14, a compound D15, a compound D17, and a compound D18;

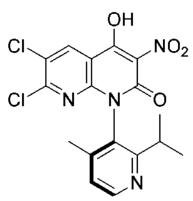
D10,

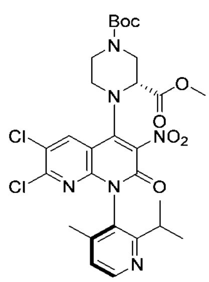
D13,

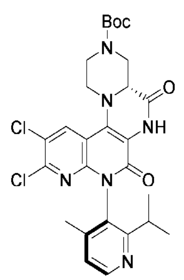
D14,

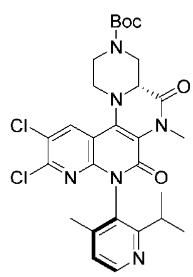
D15,

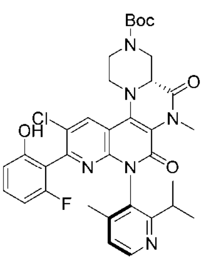
D17,

and

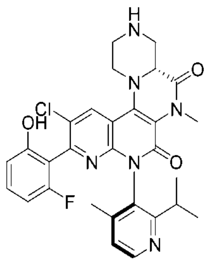

D18 ;

preferably, the compound D10, the compound D13, the compound D14, the compound D15, the compound D17, and the compound D18, alone or in any combination thereof, are used for preparing the compound of formula I.

**4.** The preparation method according to claim 3, wherein a method for preparing the compound D10 comprises the following step:

step F: subjecting the compound D9 to a nitration reaction to give the compound D10;

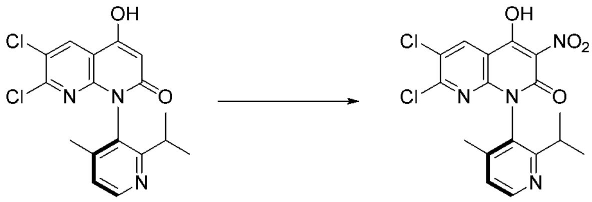

D9 D10 ;

and/or a method for preparing the compound D13 comprises the following step:

step G: substituting the hydroxy group in the compound D10 to give the compound D13;

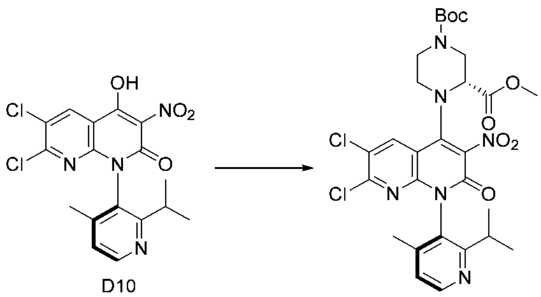

D10 D13 ;

preferably, a method for substituting the hydroxy group in the compound D10 comprises: subjecting the compound D10 to an esterification reaction with a sulfonic anhydride or a sulfonyl halide, and then subjecting to a substitution reaction with a compound D12 to give the compound D13;

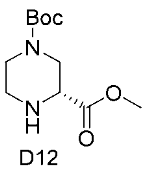

D12 ;

and/or a method for preparing the compound D14 comprises the following step:

step H: subjecting the compound D13 to a reaction in the presence of a reductant to give the compound D14;

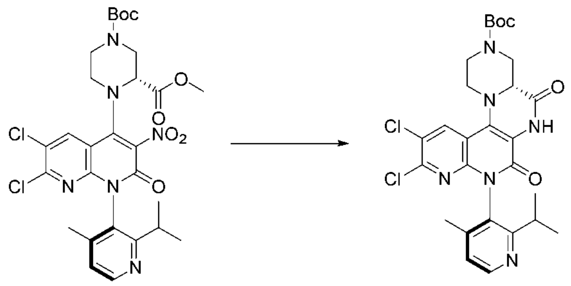

D13 D14

preferably, the reductant is sodium dithionite;
preferably, in the presence of the reductant, the compound D13 is subjected to reduction and cyclization reactions to give the compound D14;
and/or a method for preparing the compound D15 comprises the following step:

step I: subjecting the compound D14 to a reaction with a methylating reagent to give the compound D15;

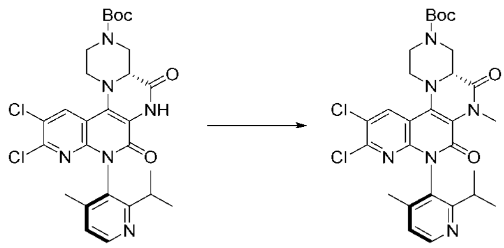

D14 D15 ;

and/or a method for preparing the compound D17 comprises the following step:

step J: subjecting the compound D15 to a coupling reaction with a compound D16 to give the compound D17;

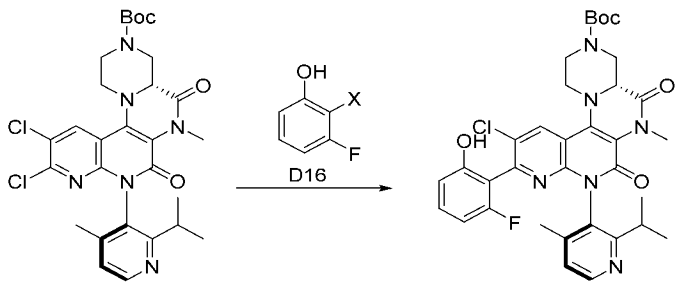

D15 D16 D17 ;

wherein X is a group capable of being subjected to a coupling reaction with a chlorine substituent at an ortho position to the N atom on the naphthyridine ring;
and/or a method for preparing the compound D18 comprises the following step:

step K: removing the Boc group from the compound D17 to give the compound D18;

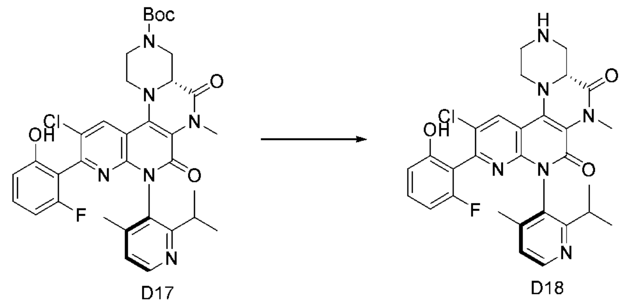

D17 D18

;

and/or a method for preparing the compound of formula I comprises the following step:

step L: subjecting the compound D18 to a reaction with an acylating agent to give the compound of formula I;

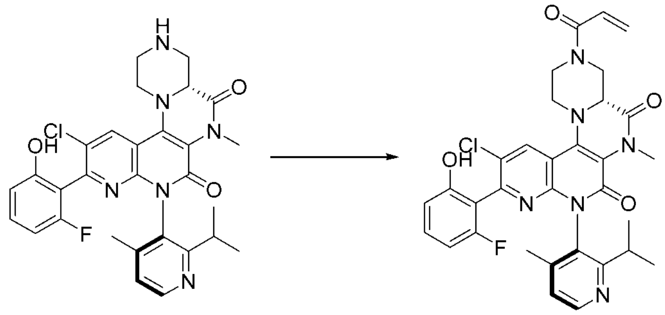

D18 I

.

**5.** The preparation method according to any one of claims 1 to 4, wherein a method for preparing the compound D6 comprises the following step:

step C: subjecting a compound D4 to a condensation reaction with a compound D5 to give the compound D6;

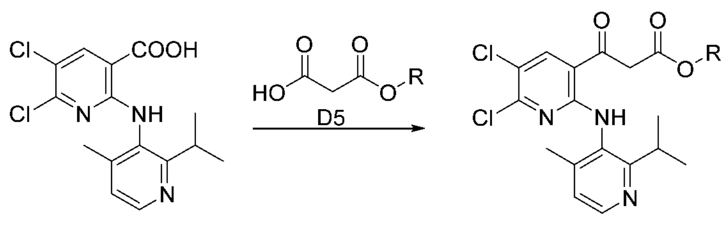


D4 D6

;

wherein R is a chiral auxiliary group, and is preferably selected from

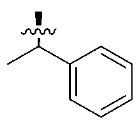
,
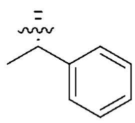
,
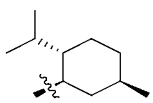
,

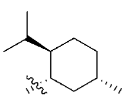
,
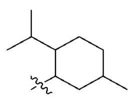
,
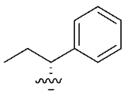
,
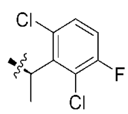
,
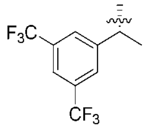
,
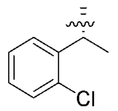
,
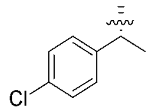
,

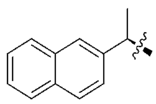, 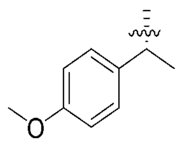, and 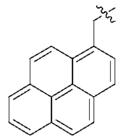.

6. The preparation method according to claim 5, wherein a method for preparing the compound D4 comprises the following step:

step B: subjecting a compound D3 to a reaction with a chlorinating reagent to give the compound D4;

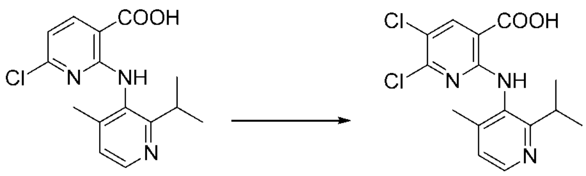

D3 D4 ;

preferably, the chlorinating reagent is selected from one or more of N-chlorosuccinimide, 1,3-dichloro-5,5-dimethylhydantoin, and chlorine gas;
preferably, a feeding molar ratio of the chlorinating reagent to the compound D3 is (0.5-2):1.

7. The preparation method according to claim 6, wherein a method for preparing the compound D3 comprises the following step:
step A: subjecting a compound D1 to a substitution reaction with a compound D2 to give the compound D3;

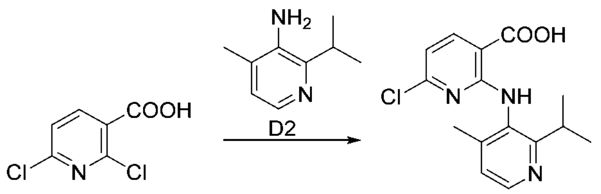


D1 D3 .

8. The preparation method according to claim 7, wherein a feeding equivalent ratio of the compound D1 to the compound D2 is (0.5-2):1;

preferably, the substitution reaction comprises the following steps: in a solvent, mixing the compound D1 with a base to give a stock solution 1, and mixing the compound D2 with a base to give a stock solution 2; and contacting the stock solution 1 with the stock solution 2 for a reaction to give the compound D3;
or mixing the compound D1 with a base in a solvent, and then adding the compound D2 for a reaction to give the compound D3;
preferably, the base is selected from one or more of lithium bis(trimethylsilyl)amide, sodium bis(trimethylsilyl)amide, potassium bis(trimethylsilyl)amide, lithium diisopropylamide, isopropylmagnesium chloride, n-butyllithium, and tert-butyllithium;
preferably, the solvent is an ether solvent;
preferably, a feeding equivalent ratio of the base used and the compound D1 and the compound D2 is (1.5-4):1:1.

9. The preparation method according to claim 5, wherein a method for preparing the compound D5 comprises the following step:

subjecting a compound SM1 to a transesterification reaction with a chiral alcohol to give the compound D5;

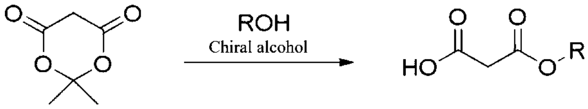


SM1 D5 ;

wherein R is a chiral auxiliary group, and is preferably selected from

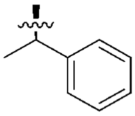 , 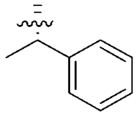 , 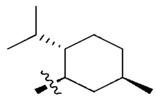 ,

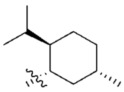 , 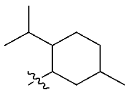 , 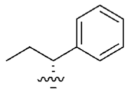 , 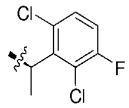 , 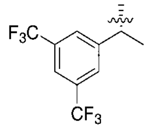 , 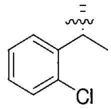 , 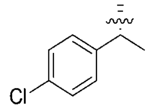 ,

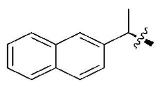 , 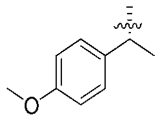 , and 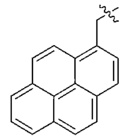 ;

preferably, a feeding equivalent ratio of the chiral alcohol to the compound SM1 is (1-3): 1.

**10.** The preparation method according to claim 3, wherein a method for preparing the compound of formula I comprises the following steps:

step A: subjecting a compound D1 to a substitution reaction with a compound D2 to give a compound D3;

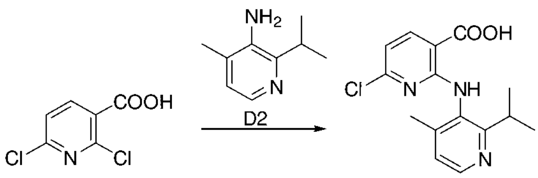
D2

D1 D3 ;

further, step B: subjecting the compound D3 to a reaction with a chlorinating reagent to give a compound D4;

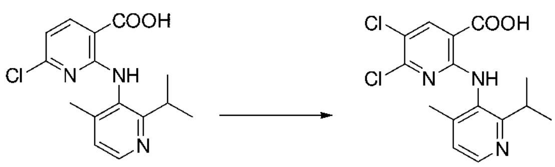

D3 D4 ;

further, step C: subjecting the compound D4 to a condensation reaction with a compound D5 to give the compound D6;

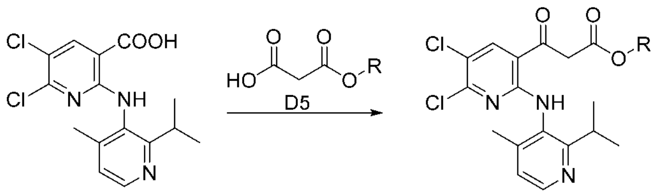


D4 D6 ;

wherein R is a chiral auxiliary group, and is preferably selected from

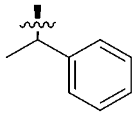, 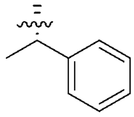, 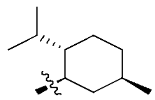,

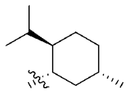, 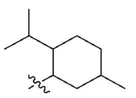, 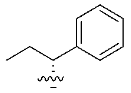, 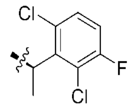, 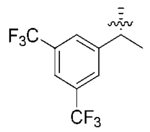, 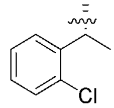, 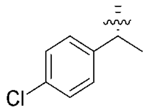,

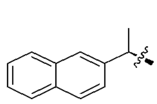, 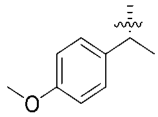, and 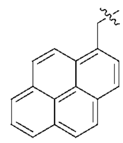;

further, step D: subjecting the compound D6 to a ring-closing reaction in the presence of a base and then to a resolution reaction with the chiral resolving reagent M to give the compound D8;

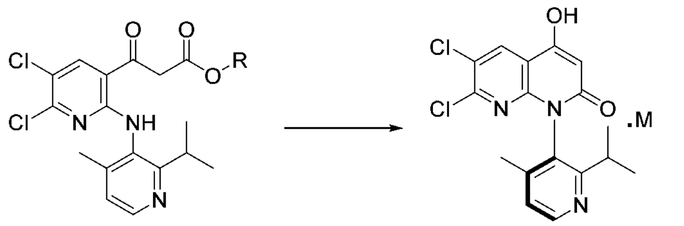

D6 D8 ;

further, step E: removing the chiral resolving reagent M from the compound D8 to give the compound D9;

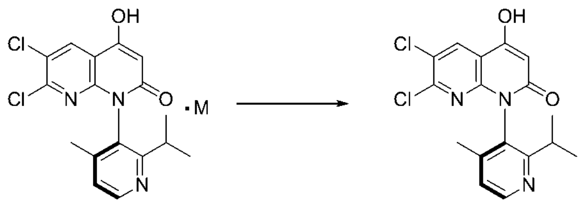

D8 D9 ;

further, step F: subjecting the compound D9 to a nitration reaction to give the compound D10;

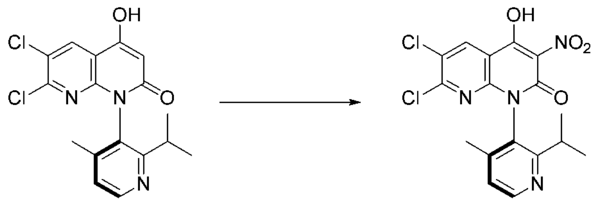

D9 D10 ;

further, step G: substituting the hydroxy group in the compound D10 to give the compound D13;

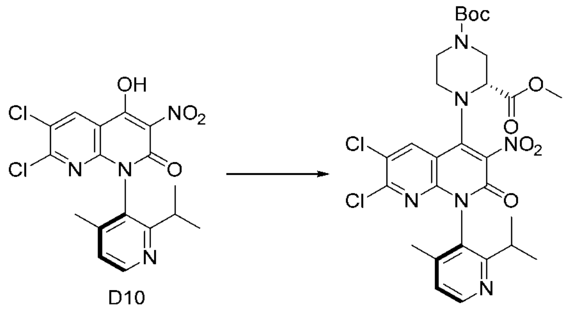

D10 D13 ;

further, step H: subjecting the compound D13 to a reaction in the presence of a reductant to give the compound D14;

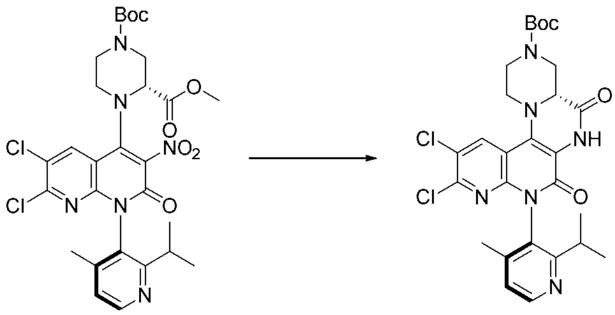

D13 D14 ;

further, step I: subjecting the compound D14 to a reaction with a methylating reagent to give the compound D15;

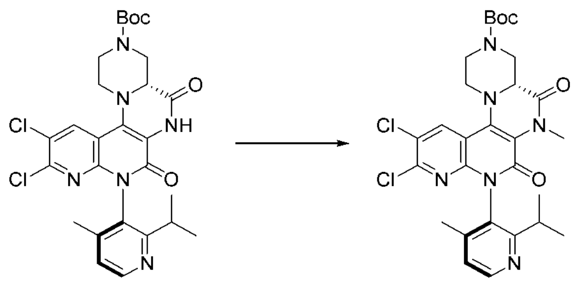

D14 D15 ;

further, step J: subjecting the compound D15 to a coupling reaction with a compound D16 to give the compound D17;

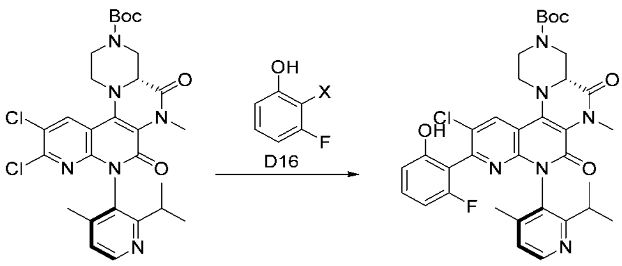


D15 D17 ;

wherein X is a group capable of being subjected to a coupling reaction with a chlorine substituent at an ortho position to the N atom on the naphthyridine ring;

further, step K: removing the Boc group from the compound D17 to give the compound D18;

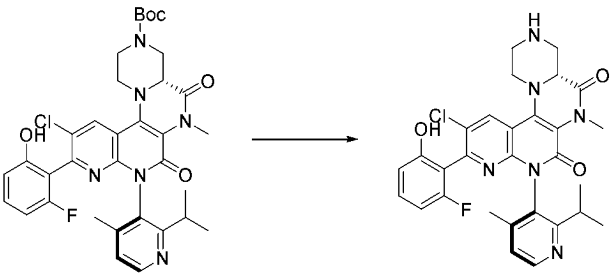

D17 D18 ;

and

further, step L: subjecting the compound D18 to a reaction with an acylating agent to give the compound of formula I;

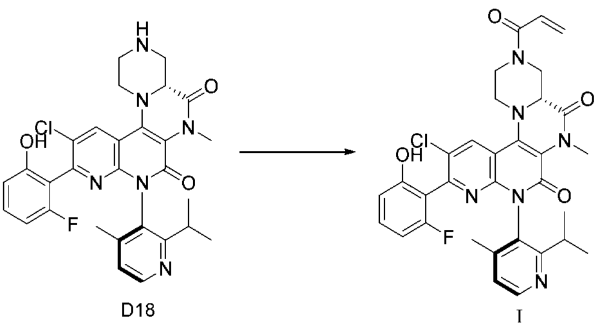

D18 I ;

wherein the acylating agent is selected from acrylic anhydride and acryloyl chloride.

**11.** The following compound or a pharmaceutically acceptable salt thereof:

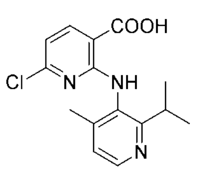
D3 ,
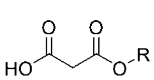
D5 ,
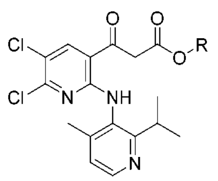
D6 ,
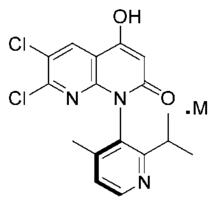
D8 ,
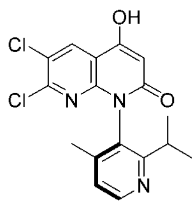
D9 ,

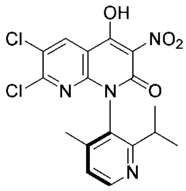
D10 ,
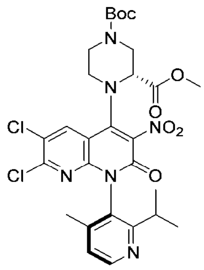
D13 ,
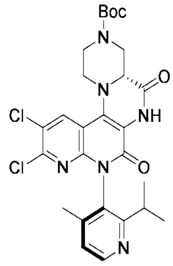
D14 ,
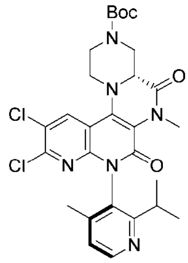
D15 ,
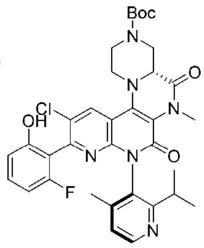
D17 ,

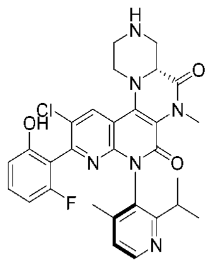
D18 ;

wherein:

R is a chiral auxiliary group, and is preferably selected from

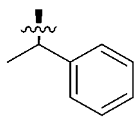 , 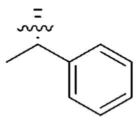 , 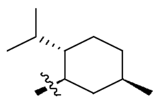 ,

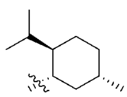 , 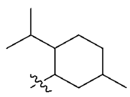 , 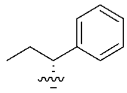 , 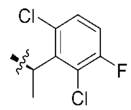 , 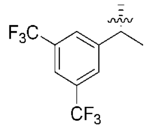 , 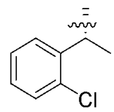 , 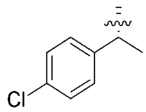 ,

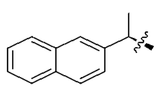 , 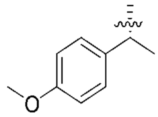 , and 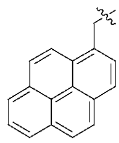 ;

M is selected from D-(+)-di-p-toluoyl tartaric acid, N-acetyl-L-phenylalanine, L-(-)-dibenzoyl tartaric acid, riboflavin, L-(-)-camphorsulfonic acid, glutamic acid, L-tartaric acid, D-(+)-di-p-anisoyl tartaric acid, D-glycine, D-mandelic acid, R-methoxy-trifluoromethylphenylacetic acid, L-aspartic acid, D-(+)-dibenzoyl tartaric acid, L-pyroglutamic acid, and (R)-(-)-1,1'-binaphthyl-2,2'-diyl hydrogenphosphate.

**12.** Use of the compound or the pharmaceutically acceptable salt thereof according to claim 11 in the manufacture of a KRAS inhibitor.

**13.** Use of the compound or the pharmaceutically acceptable salt thereof according to claim 11 in the manufacture of a medicament for treating a cancer,
preferably, the cancer is a cancer associated with a KRAS gene mutation.

**INTERNATIONAL SEARCH REPORT**

International application No.
**PCT/CN2024/112283**

**A. CLASSIFICATION OF SUBJECT MATTER**

C07D471/04(2006.01)i; C07D401/04(2006.01)i; C07D403/04(2006.01)i; A61K31/495(2006.01)i; A61P35/00(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS, CNTXT, ENTXT, ISI web of knowledge, STN: 劲方医药科技（上海）有限公司, 吡嗪, KRAS抑制剂, 癌症, ENFLEET THERAPEUTICS (SHANGHAI) INC., PYRAZINE, KRAS REGULATOR, CANCER, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| A | CN 113015724 A (AMGEN INC.) 22 June 2021 (2021-06-22)<br>claims 1-30, and description, paragraphs 317-407 | 1-13 |
| A | CN 115304602 A (ZHEJIANG HISUN PHARMACEUTICAL CO., LTD. et al.) 08 November 2022 (2022-11-08)<br>claims 1-12, and embodiments 1-6 | 1-13 |
| A | WO 2021083167 A1 (GENFLEET THERAPEUTICS (SHANGHAI) INC. et al.) 06 May 2021 (2021-05-06)<br>claims 1-27, and embodiments 1-342 | 1-13 |
| A | WO 2021052499 A1 (SHANGHAI JEMINCARE PHARMACEUTICALS CO., LTD. et al.) 25 March 2021 (2021-03-25)<br>claims 1-27, and embodiments 1-57 | 1-13 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **28 October 2024** | **07 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/112283**

| Patent document cited in search report | Publication date (day/month/year) | Patent family member(s) | Publication date (day/month/year) |
|---|---|---|---|
| CN 113015724 A | 22 June 2021 | DK 3880670 T3 | 21 August 2023 |
| | | MX 2023007748 A | 06 July 2023 |
| | | SG 11202104927 QA | 29 June 2021 |
| | | US 2022220112 A1 | 14 July 2022 |
| | | TW 202035354 A | 01 October 2020 |
| | | PL 3880670 T3 | 02 October 2023 |
| | | AU 2019381817 A1 | 20 May 2021 |
| | | MA 54233 A | 23 February 2022 |
| | | SI 3880670 T1 | 30 October 2023 |
| | | IL 282682 A | 30 June 2021 |
| | | CL 2021001266 A1 | 26 November 2021 |
| | | EP 4234546 A2 | 30 August 2023 |
| | | EP 4234546 A3 | 22 November 2023 |
| | | EA 202191381 A1 | 04 August 2021 |
| | | PT 3880670 T | 23 August 2023 |
| | | FI 3880670 T3 | 17 August 2023 |
| | | JP 2020090482 A | 11 June 2020 |
| | | JP 7516029 B2 | 16 July 2024 |
| | | HUE 062656 T2 | 28 November 2023 |
| | | CA 3117221 A1 | 22 May 2020 |
| | | AR 117101 A1 | 14 July 2021 |
| | | US 2020216446 A1 | 09 July 2020 |
| | | US 11299491 B2 | 12 April 2022 |
| | | ES 2953752 T3 | 15 November 2023 |
| | | MX 2021005598 A | 30 June 2021 |
| | | LT 3880670 T | 11 September 2023 |
| | | KR 20210093276 A | 27 July 2021 |
| | | EP 3880670 A1 | 22 September 2021 |
| | | EP 3880670 B1 | 07 June 2023 |
| | | BR 112021009384 A2 | 10 August 2021 |
| | | JP 2024127907 A | 20 September 2024 |
| | | WO 2020102730 A1 | 22 May 2020 |
| CN 115304602 A | 08 November 2022 | None | |
| WO 2021083167 A1 | 06 May 2021 | BR 112022008375 A2 | 12 July 2022 |
| | | JP 2023175993 A | 12 December 2023 |
| | | US 2023084095 A1 | 16 March 2023 |
| | | US 12054497 B2 | 06 August 2024 |
| | | JP 2023502891 A | 26 January 2023 |
| | | AU 2020374844 A1 | 16 June 2022 |
| | | AU 2020374844 B2 | 25 January 2024 |
| | | AU 2020374844 C1 | 25 July 2024 |
| | | EP 4328229 A2 | 28 February 2024 |
| | | EP 4328229 A3 | 02 October 2024 |
| | | US 2024158417 A1 | 16 May 2024 |
| | | ZA 202205758 B | 22 February 2023 |
| | | EP 4053118 A1 | 07 September 2022 |
| | | EP 4053118 A4 | 04 January 2023 |
| | | EP 4053118 B1 | 09 October 2024 |
| | | CA 3156777 A1 | 06 May 2021 |
| | | KR 20220106765 A | 29 July 2022 |

Form PCT/ISA/210 (patent family annex) (July 2022)

**INTERNATIONAL SEARCH REPORT**
**Information on patent family members**

International application No.
**PCT/CN2024/112283**

| Patent document cited in search report | | | Publication date (day/month/year) | Patent family member(s) | | | Publication date (day/month/year) |
|---|---|---|---|---|---|---|---|
| | | | | TW | 202128701 | A | 01 August 2021 |
| | | | | TWI | 754438 | B | 01 February 2022 |
| WO | 2021052499 | A1 | 25 March 2021 | JP | 2022549171 | A | 24 November 2022 |
| | | | | IL | 291467 | A | 01 May 2022 |
| | | | | CL | 2022000666 | A1 | 14 October 2022 |
| | | | | CA | 3155066 | A1 | 25 March 2021 |
| | | | | TW | 202126654 | A | 16 July 2021 |
| | | | | TWI | 761961 | B | 21 April 2022 |
| | | | | MX | 2022003401 | A | 13 July 2022 |
| | | | | KR | 20220086573 | A | 23 June 2022 |
| | | | | AU | 2020350745 | A1 | 07 April 2022 |
| | | | | EP | 4043464 | A1 | 17 August 2022 |
| | | | | EP | 4043464 | A4 | 04 October 2023 |
| | | | | BR | 112022005193 | A2 | 16 August 2022 |
| | | | | CO | 2022004686 | A2 | 08 July 2022 |
| | | | | US | 2022389029 | A1 | 08 December 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2023110285571 A
- WO 2021083167 A1